# EUROPEAN PATENT APPLICATION

(11) **EP 4 141 004 A1**
(43) Date of publication of application: **01.03.2023**
(21) Application number: 21804353.7
(22) Date of filing: 30.04.2021
(51) Int. Cl.: C07D 471/04, C07D 487/02, A61K 31/4545, A61K 31/4439, A61K 31/40, A61K 31/437, A61P 35/00

(54) **POLYCYCLIC AMIDE DERIVATIVE AS CDK9 INHIBITOR, PREPARATION METHOD THEREFOR AND USE THEREOF**

(30) Priority: 12.05.2020 CN 202010399116
(71) Applicant: Suzhou Alphama Biotechnology Co., Ltd., Jiangsu 215000 (CN)
(72) Inventor: ZHENG, Suxin, Suzhou Jiangsu 215000 (CN); XIE, Chengying, Shanghai 201203 (CN); ZHENG, Mingyue, Shanghai 201203 (CN); LU, Xiaojie, Suzhou Jiangsu 215000 (CN); QIAO, Gang, Suzhou Jiangsu 215000 (CN)
(74) Representative: Ipside
(86) International application number: PCT/CN2021/091688
(87) International publication number: WO 2021/227904

(57) **Abstract**

A polycyclic amide derivative as a CDK9 inhibitor, and a preparation method therefor and use thereof. The polycyclic amide derivative exhibits an excellent CDK9 enzyme inhibitory activity, and can be used for preparing a medicine for treating cancers, wherein the cancers are especially hematological cancers, including acute myeloid leukemia, multiple myeloma, chronic lymphocytic leukemia, follicular lymphoma and solid tumors including breast cancer, prostate cancer, ovarian cancer, hepatocellular cancer, pancreatic cancer, renal cancer, gastric cancer, colorectal cancer and lung cancer.

## Description

### TECHNICAL FIELD

The present disclosure belongs to the technical field of polycyclic amide derivatives, and particularly relates to a polycyclic amide derivative as a CDK9 inhibitor, a preparation method therefor and an application thereof.

### BACKGROUND OF THE INVENTION

Cyclin dependent protein kinases (CDKs) are a type of serine/threonine kinase, and play a key role in cell cycle regulation and cell transcription process, which have gained more and more attention as important targets for treating cancers and other diseases. The CDKs family has 13 different subtypes with similar structures, and can be activated by about 10 cyclins, exerting different biological functions. According to different effecting mechanisms, they are generally classified into cell-cycle CDK (CDK1-6) and transcriptional CDK (CDK7-9). CDK9 mainly participates in the transcriptional regulation process, and the heterodimer composed of CDK9 and cyclin (T1, T2a, T2b, K) participates in forming a positive transcription elongation factor (P-TEFb), playing a key role in the transcriptional regulation process. CDK9 has a classical protein kinase fold, consisting of C-terminal and N-terminal kinase domains and a small C-terminal extension. CDK9 participates in the elongation process of transcription as a subunit of P-TEFb, and plays an important role in the transcription process. CDK9 regulates RNA transcription of short-life anti-apoptotic proteins, and CDK9 regulates the expression of anti-apoptotic proteins by phosphorylating RNApol II. The transcription catalyzed by RNApol II is a multi-step process. The phosphorylation of Ser and Thr on the landem heptapeptide repeat sequence contained in the C-terminal domain of RNApol II large subunit plays an important role in the transcription process. While CDK9 in the P-TEFb complex phosphorylates RNApol II CTD Ser2, it also phosphorylates the sensitivity inducing factor DSIF and the negative elongation factor NELF. Phosphorylation makes NELF leave while DSIF converted to a positive transcription factor, followed by RNApol II entering the transcription elongation mode and the transcription elongation process starting¹.

Inhibition of CDK9 leads to down-regulation of anti-apoptotic proteins Mcl-1, XIAP and the like, so that these anti-apoptotic proteins lose the ability to maintain the stability of tumor cells, thereby inducing the apoptosis of tumor cells. CDK9 also participates in the regulation of many cell functions. The selective inhibition of CDK9 can also act as a potential therapeutic strategy for tumor invasion and metastasis. Recently, inhibitors of CDK9 have been reported to down-regulate MYC proteins by inhibiting transcription and post-transcriptional modification, and thus have an inhibitory effect on MYC-driven tumors².

In addition, inhibition of CDK9 can increase the number of CD45⁺ cells in the tumor environment, increase the proportion of CD3⁺T cells, and activate dendritic cells, and therefore combining with CDK9 inhibitors can enhance the immune response of the tumor immune checkpoint blockage³. CDKs are important targets for the treatment of tumors and other diseases, in the recent 20 years many CDK inhibitors have entered the clinic, and there have been selective CDK4/6 inhibitors on the market, which brings confidence and hope to develop selective CDK inhibitors. CDK9 as a novel anti-tumor drug target with great potential has gained more and more attention, there have been a plurality of selective CDK9 inhibitors entering the clinic (Fadraciclib, Zotitraciclib, KB-130742, AZD-4573, etc.), and CDK9 as an anti-tumor drug target has a broad application prospect.

At present, CDK9 inhibitors are generally classified into flavones, pyrimidines, pyridines, phenyltriazines and other classes according to their parent nucleus structure. Among them some CDK9 inhibitors have higher selectivity to CDK9 (WO2017001354, WO2018192273, WO2019154177, etc.).

However, the compounds and test drugs disclosed in the prior art still have uncertainty in terms of efficacy, safety, selectivity, etc. Therefore, it is necessary to study and develop new selective CDK9 inhibitors.

Reference:
1. Boffo et al. CDK9 inhibitors in acute myeloid leukemia. J. Eep. Clin. Cancer Res. 37, 36 (2018).
2. Blake et al. Application of a MYC degradation screen identifies sensitivity to CDK9 inhibitors in KRAS-mutant pancreatic cancer. Sci. Signal. 12, eeav7259 (2019).
3. Zhang et al. Targeting CDK9 Reactivates Epigenetically Silenced Genes in Cancer. Cell 175, 1244-1258 (2018).

### SUMMARY OF THE INVENTION

In order to solve the above problems in the prior art, an object of the present disclosure is to provide a polycyclic amide derivative, a pharmaceutically acceptable salt thereof, a tautomer thereof, or a stereoisomer thereof, so as to select a compound used as a CDK9 inhibitor with excellent properties in terms of efficacy, safety, selectivity and the like.

Another object of the present disclosure is to provide a method for preparing the derivative, the pharmaceutically acceptable salt thereof, the tautomer thereof, or the stereoisomer thereof.

In order to achieve the objects of the present disclosure, the present disclosure adopts the following technical solutions:
In a first aspect, the present disclosure provides a polycyclic amide derivative, a pharmaceutically acceptable salt thereof, a tautomer thereof, or a stereoisomer thereof, and the structure of the polycyclic amide derivative is represented by formula (I): 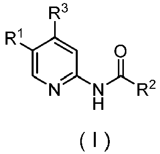
wherein: R¹ is selected from hydrogen, halogen, cyano, substituted or unsubstituted C₁-C₃ alkyl, or substituted or unsubstituted C₁-C₃ alkoxy; preferably, "substituted" refers to optionally substituted with 1-3 halogens;
R² is selected from 5-7 membered cycloalkyl, 5-7 membered cycloalkenyl, 7-10 membered fused cycloalkyl, 7-10 membered bridged cycloalkyl, 7-10 membered spirocycloalkyl, 6-7 membered heterocyclyl, 6-7 membered heterocyclenyl, 7-10 membered fused heterocyclyl, 7-10 membered bridged heterocyclyl, and 7-10 membered spiroheterocyclyl; wherein 6-7 membered cycloalkyl, 6-7 membered cycloalkenyl, 7-10 membered fused cycloalkyl, 7-10 membered bridged cycloalkyl, 7-10 membered spirocycloalkyl, 6-7 membered heterocyclyl, 6-7 membered heterocyclenyl, 7-10 membered fused heterocyclyl, 7-10 membered bridged heterocyclyl, and 7-10 membered spiroheterocyclyl are optionally further substituted with 1-3 R^{a};
R^{a} is selected from C₁-C₃ alkyl, hydroxyl, halogen, cyano, C₁-C₃ alkoxy, 3-7 membered cycloalkyl, 3-7 membered heterocyclyl, phenyl, 5-6 membered heteroaryl, 8-10 membered fused aryl, 8-10 membered fused heteroaryl, =O, NH₂, NHR_{b}, NR^{b}₂, S(O)R^{b}, S(O)₂R^{b}, S(O)NH₂, S(O)NHR^{b}, S(O)N(R^{b})₂, S(O)₂NH₂, S(O)₂NHR^{b}, S(O)₂N(R^{b})₂, NHS(O)R^{b}, NR^{b}S(O)R^{b}, NHS(O)₂R^{b}, NR^{b}S(O)₂R^{b}, C(O)R^{b}, C(O)OR^{b}, OC(O)R^{b}, NHC(O)R^{b}, NR^{b}C(O)R^{b}, NHC(O)OR^{b}, NR^{b}C(O)OR^{b}, C(O)NH₂, C(O)NHR^{b}, and C(O)N(R^{b})₂; wherein alkyl, alkoxy, cycloalkyl, heterocyclyl, phenyl, 5-6 membered heteroaryl, 8-10 membered fused aryl, 8-10 membered fused heteroaryl and amino are optionally further substituted with one or more R^{a1};
R^{b} is independently selected from substituted or unsubstituted C₁-C₃ alkyl, substituted or unsubstituted 3-6 membered cycloalkyl, or substituted or unsubstituted heterocyclyl; wherein "substituted" refers to optionally substituted with 1-3 substituents selected from alkyl, hydroxyl, halogen, cyano, amino, or alkoxy;
R^{a1} is selected from C₁-C₃ alkyl, hydroxyl, halogen, cyano, amino, C₁-C₃ alkoxy, S(O)R^{b}, S(O)₂R^{b}, S(O)NH₂, S(O)NHR^{b}, S(O)N(R^{b})₂, S(O)₂NH₂, S(O)₂NHR^{b}, S(O)₂N(R^{b})₂, NHS(O)R^{b}, NR^{b}S(O)R^{b}, NHS(O)₂R^{b}, NR^{b}S(O)₂R^{b}, C(O)R^{b}, C(O)OR^{b}, OC(O)R^{b}, NHC(O)R^{b}, NR^{b}C(O)R^{b}, NHC(O)OR^{b}, NR^{b}C(O)OR^{b}, C(O)NH₂, C(O)NHR^{b}, C(O)N(R^{b})₂; wherein alkyl and alkoxy are optionally further substituted with 1-3 halogens, hydroxyl groups, cyano groups, amino groups, or alkoxy groups;
R³ is 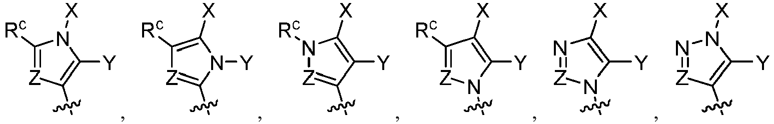 or 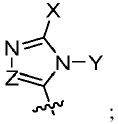
Z is N or CR^{c};
R^{c} is independently selected from H, halogen, CN, C(O)NH₂, C(O)NHR^{b}, C(O)N(R^{b})₂, C(O)R^{b}, substituted or unsubstituted C₁-C₃ alkyl, substituted or unsubstituted 3-6 membered cycloalkyl, or substituted or unsubstituted 4-7 membered heterocyclyl; wherein "substituted" refers to optionally substituted with 1-3 substituents selected from alkyl, hydroxyl, halogen, cyano, amino, or alkoxy; X and Y together with the atoms to which they are bonded form a 5-7 membered heterocyclyl or cycloalkyl, wherein heterocyclyl comprises 1-2 heteroatoms selected from N, O, S; 5-7 membered heterocyclyl or cycloalkyl may be saturated or partially saturated and the ring carbon therein may be optionally further substituted with 1-3 R^{d};
R^{d} is independently selected from halogen, OH, CN, =O, C₁-C₃ alkyl, 3-6 membered cycloalkyl or heterocyclyl, wherein alkyl, cycloalkyl, and heterocyclyl may be optionally further substituted with 1-3 substituents selected from alkyl, hydroxyl, halogen, cyano, amino, or alkoxy;
When R³ is 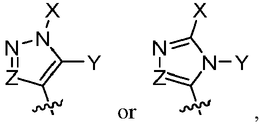 at least one of the following conditions must be satisfied:
(1) R¹ can only be C₁-C₃ alkoxy or C₁-C₃ alkyl substituted with 1-3 halogens;
(2) R² is selected from 6-7 membered heterocyclenyl, 7-10 membered fused cycloalkyl, 7-10 membered bridged cycloalkyl, 7-10 membered spirocycloalkyl, 7-10 membered fused heterocyclyl, 7-10 membered bridged heterocyclyl, 7-10 membered spiroheterocyclyl; wherein 6-7 membered heterocyclenyl, 7-10 membered fused cycloalkyl, 7-10 membered bridged cycloalkyl, 7-10 membered spirocycloalkyl, 7-10 membered fused heterocyclyl, 7-10 membered bridged heterocyclyl, 7-10 membered spiroheterocyclyl must be further substituted with 1-3 R^{e} at the same time;
(3) R² is substituted with phenyl, 5-6 membered heteroaryl, 8-10 membered fused aryl, or 8-10 membered fused heteroaryl, wherein phenyl, 5-6 membered heteroaryl, 8-10 membered fused aryl, or 8-10 membered fused heteroaryl is optionally further substituted with 1-3 R^{a1};

R^{e} is selected from S(O)R^{b}, S(O)₂R^{b}, S(O)NH₂, S(O)NHR^{b}, S(O)N(R^{b})₂, S(O)₂NH₂, S(O)₂NHR^{b}, S(O)₂N(R^{b})₂, NHS(O)₂R^{b}, NR^{b}S(O)₂R^{b}, C(O)R^{b}, C(O)OR^{b}, OC(O)R^{b}, C(O)NH₂, C(O)NHR^{b}, and C(O)N(R^{b})₂.

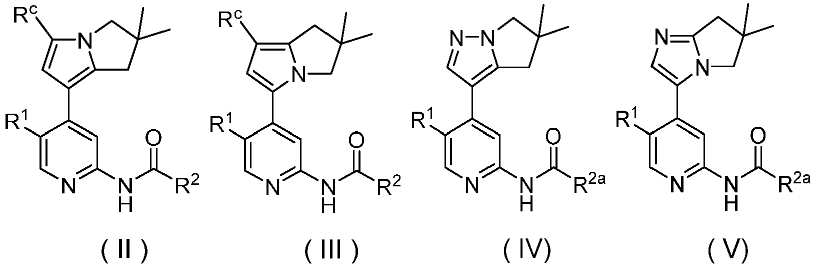

| | |
|---|---|
| 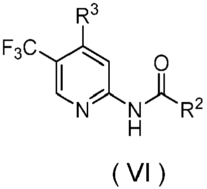 | 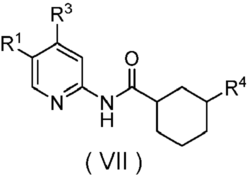 |

Preferably, the structure of the polycyclic amide derivative is represented by formula (II):
wherein R¹, R², and R^{c} have the same defined ranges as in the above general formula (I).

Preferably, the structure of the polycyclic amide derivative is represented by formula (III):
wherein R¹, R², and R^{c} have the same defined ranges as in the above general formula (I).

Preferably, the structure of the polycyclic amide derivative is represented by formula (IV): wherein:
R^{2a} is selected from 6-7 membered heterocyclenyl, 7-10 membered fused cycloalkyl, 7-10 membered bridged cycloalkyl, 7-10 membered spirocycloalkyl, 7-10 membered fused heterocyclyl, 7-10 membered bridged heterocyclyl, and 7-10 membered spiroheterocyclyl; wherein 6-7 membered heterocyclenyl, 7-10 membered fused cycloalkyl, 7-10 membered bridged cycloalkyl, 7-10 membered spirocycloalkyl, 7-10 membered fused heterocyclyl, 7-10 membered bridged heterocyclyl, and 7-10 membered spiroheterocyclyl are optionally further substituted with 1-3 R^{e};
R¹ and R^{e} have the same defined ranges as in the above general formula (I).

Preferably, the structure of the polycyclic amide derivative is represented by formula (V): wherein R¹ has the same defined range as in the above general formula (1), and R^{2a} has the same defined range as in the above general formula (IV).

Preferably, the structure of the polycyclic amide derivative is represented by formula (VI): wherein R² and R³ have the same defined ranges as in the above general formula (I).

Preferably, the structure of the polycyclic amide derivative is represented by formula (VII): wherein R⁴ is phenyl, 5-6 membered heteroaryl, 8-10 membered fused aryl, or 8-10 membered fused heteroaryl, and R⁴ is optionally further substituted with 1-3 R^{a1}; R¹, R³, and R^{a1} have the same defined ranges as in the above general formula (I).

Further preferably, the polycyclic amide derivative is selected from any one of the following structures:

| Compound No. | Structure | Naming |
|---|---|---|
| 1 | 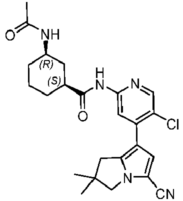 | (1*S*,3*R*)-3-acetylamino-*N*-(5-chloro-4 -(5-cyano-2,2-dimethyl-2,3-dihydro-1H-pyrrolizin-7-yl)pyridin-2-yl)cycl ohexane-1-carboxamide |
| 2 | 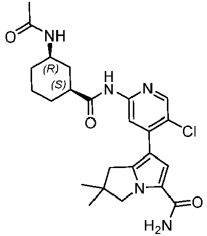 | 7-(2-((1*S*,3*R*)-3-acetylaminocyclohex ane-1-carboxamido)-5-chloropyridin-4-yl)-2,2-dimethyl-2,3-dihydro-1*H*-p yrrolizine-5-carboxamide |
| 3-1 | 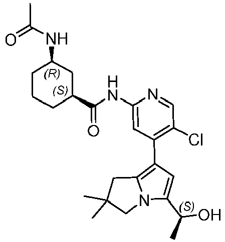 | (1*S*,3*R*)-3-acetylamino-*N*-(5-chloro-4 -(5-((*S*)-1-hydroxyethyl)-2,2-dimethy l-2,3-dihydro-1*H*-pyrrolizin-7-yl)pyri din-2-yl)cyclohexane-1-carboxamide (assumed) |
| 3-2 | 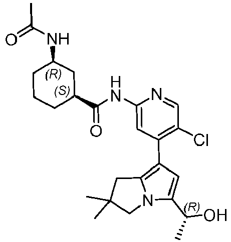 | (1*S*,3*R*)-3-acetylamino-*N*-(5-chloro-4 -(5-((*R*)-1-hydroxyethyl)-2,2-dimeth yl-2,3-dihydro-1*H*-pyrrolizin-7-yl)py ridin-2-yl)cyclohexane-1-carboxamid e (assumed) |
| 4-1 | 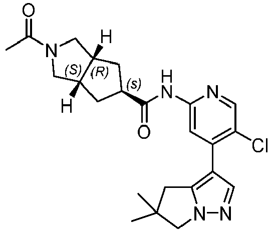 | (3a*R*,5*s*,6a*S*)-2-acetyl-N-(5-chloro-4-(5,5-dimethyl-5,6-dihydro-4*H*-pyrrol o[1,2-*b*]pyrazol-3-yl)pyridin-2-yl)oct ahydrocyclopenta[*c*]pyrrole-5-carbox amide (assumed) |
| 4-2 | 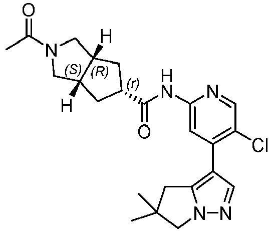 | (3a*R*,5*r*,6a*S*)-2-acetyl-*N*-(5-chloro-4-(5,5-dimethyl-5,6-dihydro-4*H*-pyrrol o[1,2-*b*]pyrazol-3-yl)pyridin-2-yl)oct ahydrocyclopenta[*c*]pyrrole-5-carbox amide (assumed) |
| 5 | 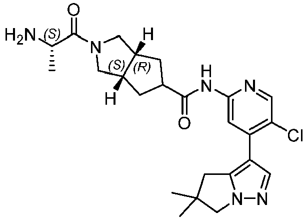 | (3a*R*,6a*S*)-2-(*L*-alanyl)-*N*-(5-chloro-4 -(5,5-dimethyl-5,6-dihydro-4*H*-pyrro lo[1,2-*b*]pyrazol-3-yl)pyridin-2-yl)oc tahydrocyclopenta[*c*]pyrrole-5-carbo xamide |
| 6 | 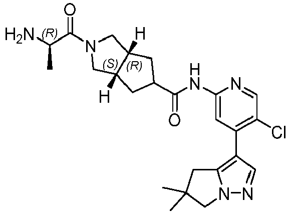 | (3a*R*,6a*S*)-2-(*D*-alanyl)-*N*-(5-chloro-4-(5,5-dimethyl-5,6-dihydro-4*H*-pyrr olo[1,2-*b*]pyrazol-3-yl)pyridin-2-yl)o ctahydrocyclopenta[*c*]pyrrole-5-carb oxamide |
| 7-1 | 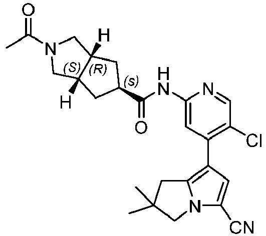 | (3a*R*,5*s*,6a*S*)-2-acetyl-*N*-(5-chloro-4-(5-cyano-2,2-dimethyl-2,3-dihydro-1 *H*-pyrrolizin-7-yl)pyridin-2-yl)octah ydrocyclopenta[*c*]pyrrole-5-carboxa mide (assumed) |
| 7-2 | 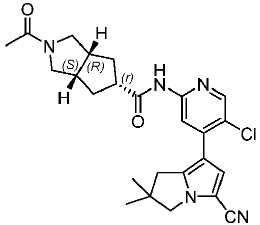 | (3a*R*,5*r*,6a*S*)-2-acetyl-*N*-(5-chloro-4-(5-cyano-2,2-dimethyl-2,3-dihydro-1 *H*-pyrrolizin-7-yl)pyridin-2-yl)octah ydrocyclopenta[*c*]pyrrole-5-carboxa mide (assumed) |
| 8-1 | 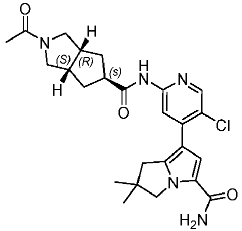 | (3a*R*,5*s*,6a*S*)-2-acetyl-*N*-(4-(5-carba moyl-2,2-dimethyl-2,3-dihydro-1*H*-p yrrolizin-7-yl)-5-chloropyridin-2-yl) octahydrocyclopenta[*c*]pyrrole-5-car boxamide (assumed) |
| 8-2 | 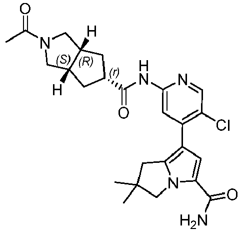 | (3a*R*,5*r*,6a*S*)-2-acetyl-*N*-(4-(5-carba moyl-2,2-dimethyl-2,3-dihydro-1*H*-p yrrolizin-7-yl)-5-chloropyridin-2-yl) octahydrocyclopenta[*c*]pyrrole-5-car boxamide (assumed) |
| 9 | 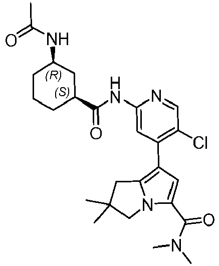 | 7-(2-((1*S*,3*R*)-3-acetylaminocyclohex ane-1-carboxamido)-5-chloropyridin-4-yl)-*N*,*N*,2,2-tetramethyl-2,3-dihydr o-1*H*-pyrrolizine-5-carboxamide |
| 10 | 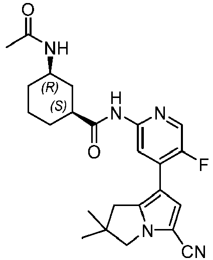 | (1*S*,3*R*)-3-acetylamino-*N*-(4-(5-cyan o-2,2-dimethyl-2,3-dihydro-1*H*-pyrro lizin-7-yl)-5-fluoropyridin-2-yl)cyclo hexane-1-carboxamide |
| 11 | 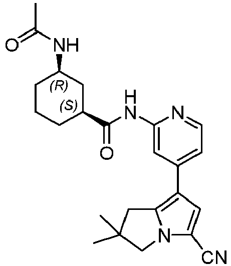 | (1*S*,3*R*)-3-acetylamino-*N*-(4-(5-cyan o-2,2-dimethyl-2,3-dihydro-1*H*-pyrro lizin-7-yl)pyridin-2-yl)cyclohexane-1 -carboxamide |
| 12 | 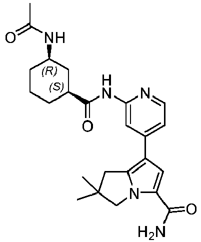 | 7-(2-((1*S*,3*R*)-3-acetylaminocyclohex ane-1-carboxamido)pyridin-4-yl)-2,2 -dimethyl-2,3-dihydro-1*H*-pyrrolizin e-5-carboxamide |
| 13 | 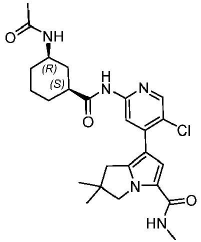 | 7-(2-((1*S*,3*R*)-3-aminocyclohexane-1 -carboxamido)-5-chloropyridin-4-yl) -*N*,2,2-trimethyl-2,3-dihydro-1*H*-pyr rolizine-5-carboxamide |
| 14-1-1 | 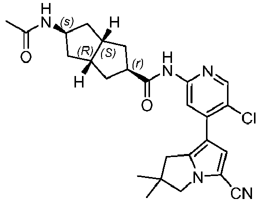 | (2*r*,3a*R*,5*s*,6a*S*)-5-acetylamino-*N*-(5-chloro-4-(5-cyano-2,2-dimethyl-2,3-dihydro-1*H*-pyrrolizin-7-yl)pyridin-2 -yl)octahydropentalene-2-carboxami de (assumed) |
| 14-1-2 | 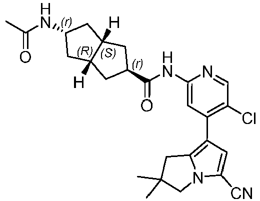 | (2*r*,3a*R*,5*r*,6a*S*)-5-acetylamino-*N*-(5-chloro-4-(5-cyano-2,2-dimethyl-2,3-dihydro-1H-pyrrolizin-7-yl)pyridin-2 -yl)octahydropentalene-2-carboxami de (assumed) |
| 14-2-1 | 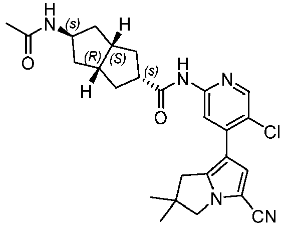 | (2*s*,3a*R*,5*s*,6a*S*)-5-acetylamino-*N*-(5-chloro-4-(5-cyano-2,2-dimethyl-2,3-dihydro-1*H*-pyrrolizin-7-yl)pyridin-2 -yl)octahydropentalene-2-carboxami de (assumed) |
| 14-2-2 | 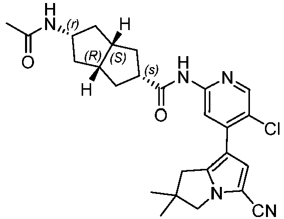 | (2*s*,3a*R*,5*r*,6a*S*)-5-acetylamino-*N*-(5-chloro-4-(5-cyano-2,2-dimethyl-2,3-dihydro-1*H*-pyrrolizin-7-yl)pyridin-2 -yl)octahydropentalene-2-carboxami de (assumed) |
| 15-1 | 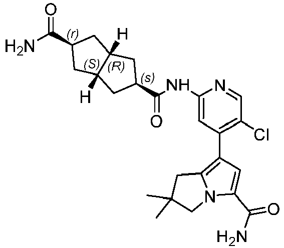 | (2*s*,3a*R*,5*r*,6a*S*)-*N*-(4-(5-carbamoyl-2 ,2-dimethyl-2,3-dihydro-1*H*-pyrrolizi n-7-yl)-5-chloropyridin-2-yl)octahyd ropentalene-2,5-dicarboxamide (assumed) |
| 15-2 | 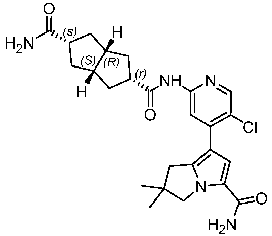 | (2r,3aR,5s,6aS)-N-(4-(5-carbamoyl-2 ,2-dimethyl-2,3-dihydro-1*H*-pyrrolizi n-7-yl)-5-chloropyridin-2-yl)octahyd ropentalene-2,5-dicarboxamide (assumed) |
| 16 | 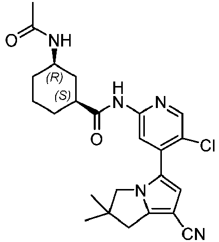 | (1*S*,3*R*)-3-acetylamino-*N*-(5-chloro-4 -(7-cyano-2,2-dimethyl-2,3-dihydro-1*H*-pyrrolizin-5-yl)pyridin-2-yl)cycl ohexane-1-carboxamide |
| 17 | 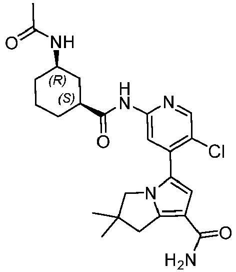 | 5-(2-((1*S*,3*R*)-3-acetylaminocyclohex ane-1-carboxamido)-5-chloropyridin-4-yl)-2,2-dimethyl-2,3-dihydro-1*H*-p yrrolizin-7-carboxamide |
| 18-1 | 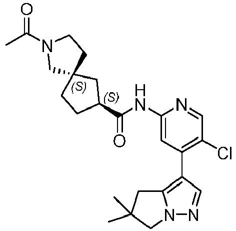 | (5*S*,7*S*)-2-acetyl-*N*-(5-chloro-4-(5,5-dimethyl-5,6-dihydro-4*H*-pyrrolo[1,2 -*b*]pyrazol-3-yl)pyridin-2-yl)-2-diaza spiro[4.4]nonane-7-carboxamide (assumed) |
| 18-2 | 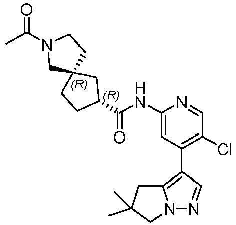 | (5*R*,7*R*)-2-acetyl-*N*-(5-chloro-4-(5,5-dimethyl-5,6-dihydro-4*H*-pyrrolo[1,2 -*b*]pyrazol-3-yl)pyridin-2-yl)-2-diaza spiro[4.4]nonane-7-carboxamide (assumed) |
| 19-1 | 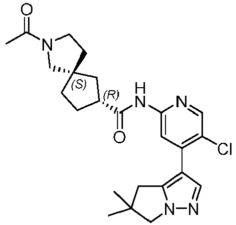 | (5*S*,7*R*)-2-acetyl-*N*-(5-chloro-4-(5,5-dimethyl-5,6-dihydro-4*H*-pyrrolo[1,2 -*b*]pyrazol-3-yl)pyridin-2-yl)-2-diaza spiro[4.4]nonane-7-carboxamide (assumed) |
| 19-2 | 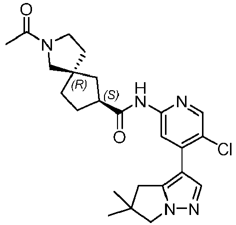 | (5*R*,7*S*)-2-acetyl-*N*-(5-chloro-4-(5,5-dimethyl-5,6-dihydro-4*H*-pyrrolo[1,2 -*b*]pyrazol-3-yl)pyridin-2-yl)-2-diaza spiro[4.4]nonane-7-carboxamide (assumed) |
| 20-1 | 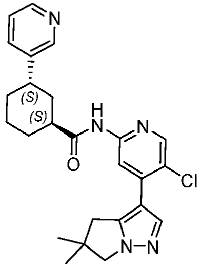 | (1*S*,3*S*)-*N*-(5-chloro-4-(5,5-dimethyl-5,6-dihydro-4*H*-pyrrolo[1,2-*b*]pyrazo 1-3-yl)pyridin-2-yl)-3-(pyridin-3-yl)c yclohexane-1-carboxamide (assumed) |
| 20-2 | 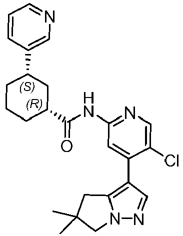 | (1*R*,3*S*)-*N*-(5-chloro-4-(5,5-dimethyl-5,6-dihydro-4*H*-pyrrolo[1,2-b]pyrazo 1-3-yl)pyridin-2-yl)-3-(pyridin-3-yl)c yclohexane-1-carboxamide (assumed) |
| 20-3 | 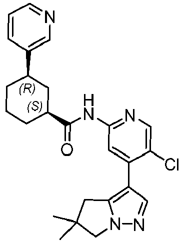 | (1*S*,3*R*)-*N*-(5-chloro-4-(5,5-dimethyl-5,6-dihydro-4*H*-pyrrolo[1,2-*b*]pyrazo 1-3-yl)pyridin-2-yl)-3-(pyridin-3-yl)c yclohexane-1-carboxamide (assumed) |
| 20-4 | 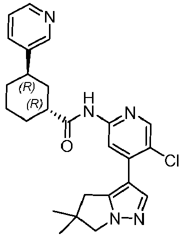 | (1*R*,3*R*)-*N*-(5-chloro-4-(5,5-dimethyl -5,6-dihydro-4*H*-pyrrolo[1,2-*b*]pyraz ol-3-yl)pyridin-2-yl)-3-(pyridin-3-yl) cyclohexane-1-carboxamide (assumed) |
| 21-1 | 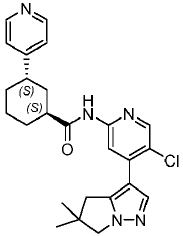 | (1*S*,3*S*)-*N*-(5-chloro-4-(5,5-dimethyl-5,6-dihydro-4*H*-pyrrolo[1,2-*b*]pyrazo l-3-yl)pyridin-2-yl)-3-(pyridin-4-yl)c yclohexane-1-carboxamide (assumed) |
| 21-2 | 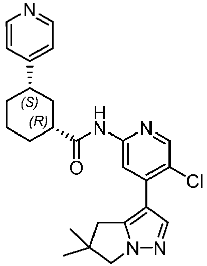 | (1*S*,3*R*)-*N*-(5-chloro-4-(5,5-dimethyl-5,6-dihydro-4*H*-pyrrolo[1,2-b]pyrazo l-3-yl)pyridin-2-yl)-3-(pyridin-4-yl)c yclohexane-1-carboxamide (assumed) |
| 21-3 | 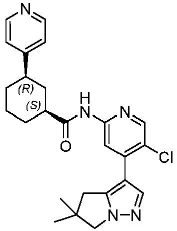 | (1*R*,3*S*)-*N*-(5-chloro-4-(5,5-dimethyl-5,6-dihydro-4*H*-pyrrolo[1,2-*b*]pyrazo l-3-yl)pyridin-2-yl)-3-(pyridin-4-yl)c yclohexane-1-carboxamide (assumed) |
| 21-4 | 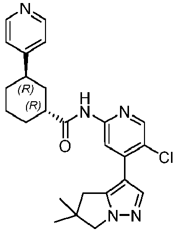 | (1*R*,3*R*)-*N*-(5-chloro-4-(5,5-dimethyl -5,6-dihydro-4*H*-pyrrolo[1,2-*b*]pyraz ol-3-yl)pyridin-2-yl)-3-(pyridin-4-yl) cyclohexane-1-carboxamide (assumed) |
| 22-1-1 | 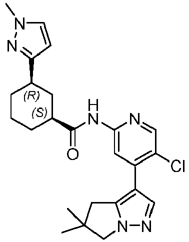 | (1*S*,3*R*)-*N*-(5-chloro-4-(5,5-dimethyl-5,6-dihydro-4*H*-pyrrolo[1,2-*b*]pyrazo 1-3-yl)pyridin-2-yl)-3-(1-methyl-1*H-*pyrazol-3-yl)cyclohexane-1-carboxa mide (assumed) |
| 22-1-2 | 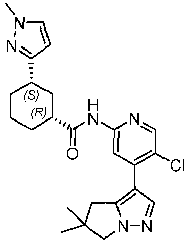 | (1*R*,3*S*)-*N*-(5-chloro-4-(5,5-dimethyl-5,6-dihydro-4*H*-pyrrolo[1,2-*b*]pyrazo 1-3-yl)pyridin-2-yl)-3-(1-methyl-1*H-*pyrazol-3-yl)cyclohexane-1 -carboxa mide (assumed) |
| 22-2-1 | 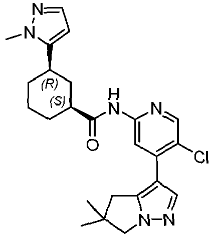 | (1*S*,3*R*)-*N*-(5-chloro-4-(5,5-dimethyl-5,6-dihydro-4*H*-pyrrolo[1,2-b]pyrazo 1-3-yl)pyridin-2-yl)-3-(1-methyl-1*H-*pyrazol-5-yl)cyclohexane-1-carboxa mide (assumed) |
| 22-2-2 | 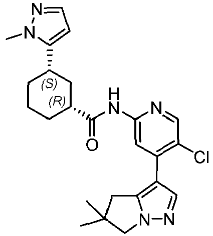 | (1*R*,3*S*)-*N*-(5-chloro-4-(5,5-dimethyl-5,6-dihydro-4*H*-pyrrolo[1,2-b]pyrazo l-3-yl)pyridin-2-yl)-3-(1-methyl-1*H-*pyrazol-5-yl)cyclohexane-1-carboxa mide (assumed) |
| 23-1 | 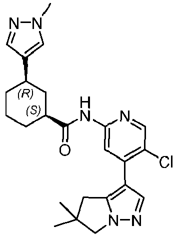 | (1*S*,3*R*)-*N*-(5-chloro-4-(5,5-dimethyl-5,6-dihydro-4*H*-pyrrolo[1,2-*b*]pyrazo l-3-yl)pyridin-2-yl)-3-(1-methyl-1*H-*pyrazol-4-yl)cyclohexane-1-carboxa mide (assumed) |
| 23-2 | 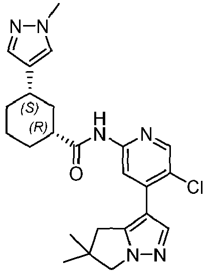 | (1*R*,3*S*)-*N*-(5-chloro-4-(5,5-dimethyl-5,6-dihydro-4*H*-pyrrolo[1,2-*b*]pyrazo 1-3-yl)pyridin-2-yl)-3-(1-methyl-1*H-*pyrazol-4-yl)cyclohexane-1-carboxa mide (assumed) |
| 24-1 | 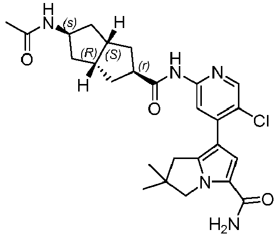 | 7-(2-((2*r*,3a*R*,5*s*,6a*S*)-5-(acetylamino )octahydropentalene-2-carboxamido) -5-chloropyridin-4-yl)-2,2-dimethyl-2,3-dihydro-1*H*-pyrrolizine-5-carbox amide (assumed) |
| 24-2 | 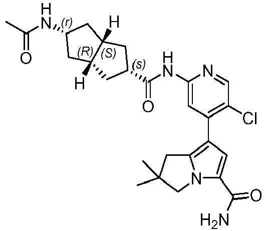 | 7-(2-((2*s*,3a*R*,5*r*,6a*S*)-5-(acetylamino )octahydropentalene-2-carboxamido) -5-chloropyridin-4-yl)-2,2-dimethyl-2,3-dihydro-1*H*-pyrrolizine-5-carbox amide (assumed) |
| 25-1 | 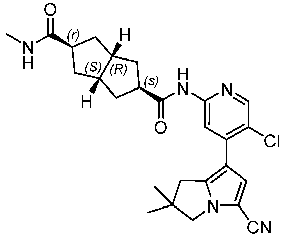 | (2*s*,3a*R*,5*r*,6a*S*)-*N*²-(5-chloro-4-(5-cy ano-2,2-dimethyl-2,3-dihydro-1*H*-py rrolizin-7-yl)pyridin-2-yl)-*N*⁵-methyl octahydropentalene-2,5-dicarboxami de (assumed) |
| 25-2 | 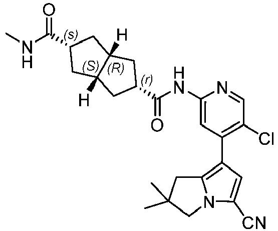 | (2*r*,3a*R*,5*s*,6a*S*)-*N*²-(5-chloro-4-(5-cy ano-2,2-dimethyl-2,3-dihydro-1*H*-py rrolizin-7-yl)pyridin-2-yl)*-N*⁵-methyl octahydropentalene-2,5-dicarboxami de (assumed) |
| 26-1 | 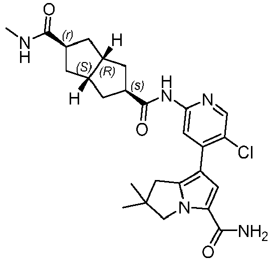 | (2*s*,3a*R*,5*r*,6a*S*)-*N*²-(4-(5-carbamoyl-2,2-dimethyl-2,3-dihydro-1*H*-pyrroli zin-7-yl)-5-chloropyridin-2-yl)-*N*⁵-m ethyloctahydropentalene-2,5-dicarbo xamide (assumed) |
| 26-2 | 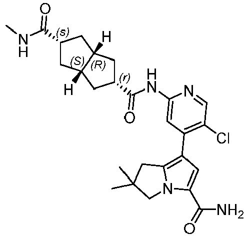 | (2*r*,3a*R*,5*s*,6a*S*)-*N*²-(4-(5-carbamoyl-2,2-dimethyl-2,3-dihydro-1*H*-pyrroli zin-7-yl)-5-chloropyridin-2-yl)-*N*⁵-m ethyloctahydropentalene-2,5-dicarbo xamide (assumed) |
| 27 | 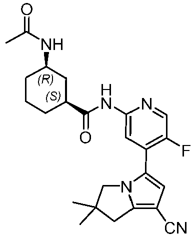 | (1*S*,3*R*)-3-acetylamino-*N*-(4-(7-cyan o-2,2-dimethyl-2,3-dihydro-1*H*-pyrro lizin-5-yl)-5-fluoropyridin-2-yl)cyclo hexane-1-carboxamide |
| 28 | 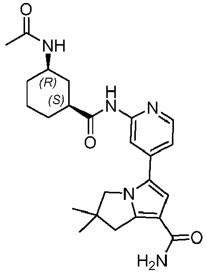 | 5-(2-((1*S*,3*R*)-3-acetylaminocyclohex ane-1-carboxamido)pyridin-4-yl)-2,2 -dimethyl-2,3-dihydro-1*H*-pyrrolizin e-7-carboxamide |
| 29 | 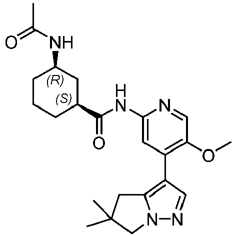 | (1*S*,3*R*)-3-acetylamino-*N*-(4-(5,5-dim ethyl-5,6-dihydro-4*H*-pyrrolo[1,2-*b*] pyrazol-3-yl)-5-methoxypyridin-2-yl )cyclohexane-1-carboxamide |
| 30 | 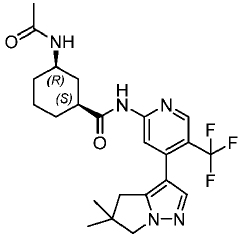 | (1*S*,3*R*)-3-acetylamino-*N*-(4-(5,5-dim ethyl-5,6-dihydro-4*H*-pyrrolo[1,2-*b*] pyrazol-3-yl)-5-(trifluoromethyl)pyri din-2-yl)cyclohexane-1-carboxamide |
| 31 | 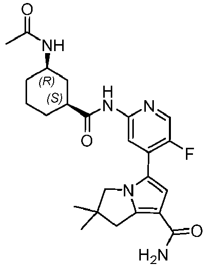 | 5-(2-((1*S*,3*R*)-3-acetylaminocyclohex ane-1-carboxamido)-5-fluoropyridin-4-yl)-2,2-dimethyl-2,3-dihydro-1*H*-p yrrolizin-7-carboxamide |
| 32-1 | 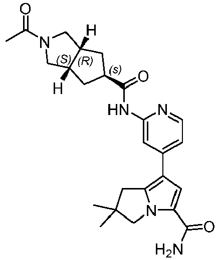 | (3a*R*,5*s*,6a*S*)-2-acetyl-*N*-(4-(5-carba moyl-2,2-dimethyl-2,3-dihydro-1*H*-p yrrolizin-7-yl)pyridin-2-yl)octahydro cyclopenta[*c*]pyrrole-5-carboxamide (assumed) |
| 32-2 | 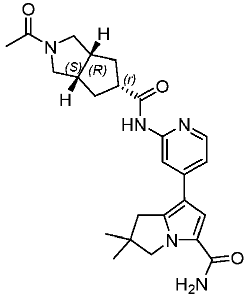 | (3a*R*,5*r*,6a*S*)-2-acetyl-*N*-(4-(5-carba moyl-2,2-dimethyl-2,3-dihydro-1*H*-p yrrolizin-7-yl)pyridin-2-yl)octahydro cyclopenta[*c*]pyrrole-5-carboxamide (assumed) |
| 33 | 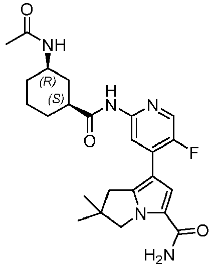 | 7-(2-((1*S*,3*R*)-3-acetylaminocyclohex ane-1-carboxamido)-5-fluoropyridin-4-yl)-2,2-dimethyl-2,3-dihydro-1*H*-p yrrolizine-5-carboxamide |
| 34-1 | 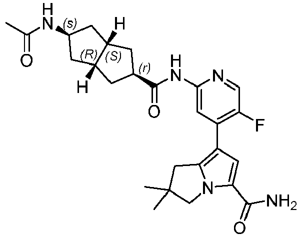 | 7-(2-((2*r*,3a*R*,5*s*,6a*S*)-5-acetylamino octahydropentalene-2-carboxamido)-5-fluoropyridin-4-yl)-2,2-dimethyl-2, 3-dihydro-1*H*-pyrrolizine-5-carboxa mide (assumed) |
| 34-2 | 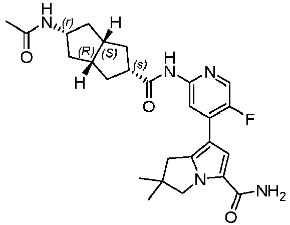 | 7-(2-((2*s*,3a*R*,5*r*,6a*S*)-5-acetylamino octahydropentalene-2-carboxamido)-5-fluoropyridin-4-yl)-2,2-dimethyl-2, 3-dihydro-1*H*-pyrrolizine-5-carboxa mide (assumed) |

In a second aspect, the present disclosure provides a preparation method for the polycyclic amide derivative, the pharmaceutically acceptable salt thereof, the tautomer thereof, or the stereoisomer thereof as described in the first aspect, selected from one of the following three solutions:
Solution 1 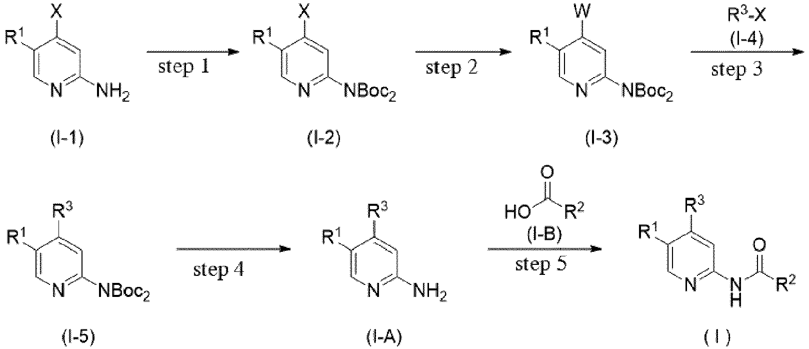
   In step 1, the amine of the compound of general formula (1-1) is protected with Boc under an alkaline condition to obtain a compound of general formula (1-2);
   In step 2, the compound of general formula (1-2) is in the presence of a metal catalyst under an alkaline condition to obtain a compound of general formula (1-3);
   In step 3, the compound of general formula (1-3) and the compound of general formula (1-4) undergo Suzuki reaction in the presence of a metal catalyst and a ligand under an alkaline condition to obtain a compound of general formula (1-5);
   In step 4, the Boc protecting group of the compound of general formula (1-5) is removed under an acidic condition to obtain a compound of general formula (I-A);
   In step 5, the compound of general formula (I-A) and the compound of general formula (I-B) undergo a condensation reaction under an alkaline condition to obtain a compound of general formula (I).
Solution 2 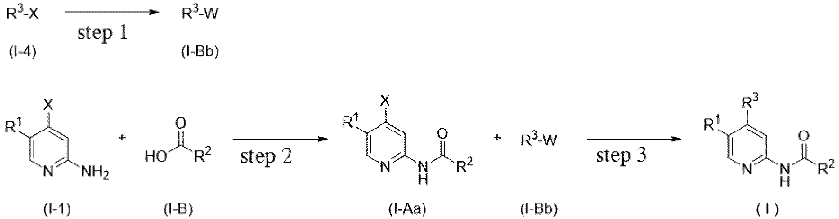
   In step 1, the compound of general formula (1-4) is in the presence of a metal catalyst under an alkaline condition to obtain a compound of general formula (I-Bb);
   In step 2, the compound of general formula (1-1) and the compound of general formula (I-B) undergo a condensation reaction under an alkaline condition to obtain a compound of general formula (I-Aa);
   In step 3, the compound of general formula (I-Aa) and the compound of general formula (I-Bb) undergo a Suzuki reaction in the presence of a metal catalyst and a ligand under an alkaline condition to obtain a compound of general formula (I).
Solution 3 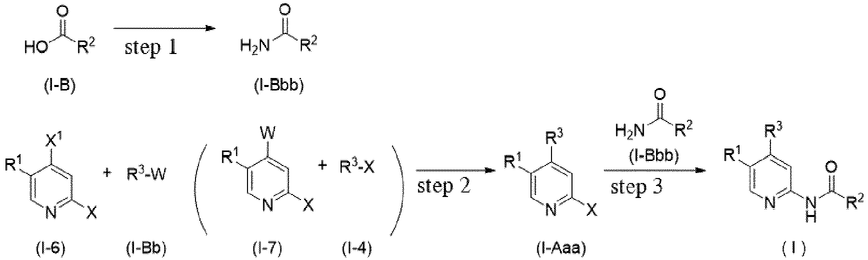
   In step 1, the compound of general formula (I-B) and ammonium chloride undergo a condensation reaction under an alkaline condition to obtain a compound of general formula (I-Bbb);
   In step 2, the compound of general formula (1-6) and the compound of general formula (I-Bb) undergo a Suzuki reaction in the presence of a catalyst under an alkaline condition to obtain a compound of general formula (I-Aaa); or the compound of general formula (1-7) and the compound of general formula (1-4) undergo a Suzuki reaction in the presence of a catalyst under an alkaline condition to obtain a compound of general formula (I-Aaa);
   In step 3, the compound of general formula (I-Aaa) and the compound of general formula (I-Bbb) undergo a condensation reaction under an alkaline condition to obtain a compound of general formula (I).

Wherein W is 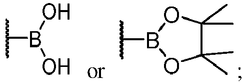 X is halogen; R¹, R², and R³ have the same defined ranges as in the above general formula (I).

In the above preparation method, the reagent providing the alkaline condition is selected from an organic base or an inorganic base, the organic base is one or more of triethylamine, *N*,*N*-diisopropylethylamine, n-butyllithium, lithium diisopropylamide, lithium bis(trimethylsilyl)amide, potassium acetate, sodium acetate, sodium tert-butoxide, sodium methoxide and potassium tert-butoxide, and the inorganic base is one or more of sodium hydride, potassium phosphate, sodium carbonate, potassium carbonate, potassium acetate, cesium carbonate, sodium hydroxide, potassium hydroxide, sodium bicarbonate and lithium hydroxide; The reagent providing the acidic condition is one or more of hydrogen chloride, 1,4-dioxane solution of hydrogen chloride, trifluoroacetic acid, formic acid, acetic acid, hydrochloric acid, sulfuric acid, methanesulfonic acid, nitric acid, and phosphoric acid;

The metal catalyst is one or more of palladium/carbon, Raney nickel, tetrakis(triphenylphosphine)palladium, palladium dichloride, palladium acetate, [1,1'-bis(diphenylphosphino)ferrocene]dichloridepalladium (Pd(dppf)Cl₂), [1,1'-bis(diphenylphosphino)ferrocene]dichloridepalladium dichloromethane complex, bis(triphenylphosphine)palladium dichloride(Pd(PPh₃)Cl₂), and tris(dibenzylideneacetone)dipalladium (Pd₂(dba)₃). The ligand is one or more of 2-dicyclohexylphosphino-2,6'-dimethoxybiphenyl (SPhos), 4,5-bis(diphenylphosphino)-9,9-dimethylxanthene (XantPhos), 2-dicyclohexylphosphino-2,4,6-triisopropylbiphenyl (XPhos), 2-dicyclohexylphosphino-2'-(*N*,*N*-dimethylamino)biphenyl (DavePhos), 1,1'-bis(diphenylphosphino)ferrocene (Dppf) and 1,1'-binaphthyl-2.2'-diphemyl phosphine (BINAP), preferably 4,5-bis(diphenylphosphino)-9,9-dimethylxanthene (XantPhos); The condensing agent is one or more of dicyclohexylcarbodiimide (DCC), diisopropylcarbodiimide (DIC), 1-(3-dimethylaminopropyl)-3-ethylcarbodiimide (EDC), 1-(3-dimethylaminopropyl)-3-ethylcarbodiimide (EDCI), 2-(7-oxidobenzotriazol)-*N*,*N*,*N*'*,N*'-tetramethyluronium hexafluorophosphate (HATU), 2-(1*H*-benzotriazol-1-yl)-1,1,3,3-tetramethyluronium tetrafluoroborate (TBTU), 1-hydroxybenzotriazole (HOBt) and 1-propylphosphonic anhydride(T3P).

The above mentioned reaction is preferably carried out in a solvent, and the solvent used is one or more of *N*,*N*-dimethylformamide, dimethyl sulfoxide, 1,4-dioxane, water, tetrahydrofuran, dichloromethane, 1,2-dichloroethane, acetic acid, methanol, ethanol, toluene, petroleum ether, ethyl acetate, n-hexane, acetone, diethyl ether and diethylene glycol.

In a third aspect, the present disclosure provides a pharmaceutical composition, and the pharmaceutical composition comprises the polycyclic amide derivative, the stereoisomer, the tautomer, and the pharmaceutically acceptable salt as described above;

Preferably, the pharmaceutical composition further comprises a pharmaceutically acceptable carrier and/or an excipient.

In a fourth aspect, the present disclosure provides an application of the polycyclic amide derivative, the stereoisomer, the tautomer, and the pharmaceutically acceptable salt according to the first aspect, or the pharmaceutical composition according to the third aspect in the preparation of medicaments for treating cancers, wherein the cancers are preferably blood cancers including acute myeloid leukemia, multiple myeloma, chronic lymphocytic leukemia, follicular lymphoma, etc., and solid tumors including breast cancer, prostate cancer, ovarian cancer, hepatocellular carcinoma, pancreatic cancer, renal cancer, gastric cancer, colorectal cancer, lung cancer, etc.

The present disclosure also provides a method for using the polycyclic amide derivative as a CDK9 inhibitor to treat cancers, by administering an effective amount of the compound to a subject suffering from cancer.

These compounds of the present disclosure can be further administered in combination with a therapeutically effective amount of one or more reagents for the treatment of cancer, wherein examples of the reagents include, for example, radiation, alkylating agents, angiogenesis inhibitors, anti-mitotic agents, anti-proliferative agents, aurora kinase inhibitors, cell death activators(for example, inhibitors of Bcl-2, BclxL, Bcl-w, Bfl-1, or Mcl-1), activators of the death receptor pathway, Bcr-Abl kinase inhibitors, BET(bromodomain protein) inhibitors, inhibitors of the Ras signaling pathway(for example, inhibitors of MEK, Raf or Ras), antibodies, BiTE(bispecific T-cell engager) antibodies, antibody-drug conjugates, biological response modifiers, cyclin-dependent kinase inhibitors, cell cycle inhibitors, cyclooxygenase-2 inhibitors, DVD (dual variable domain) antibodies, leukemia viral oncogene homolog (ErbB2) receptor inhibitors, growth factor inhibitors, heat shock protein (HSP)-90 inhibitors, histone deacetylase (HDAC) inhibitors, hormone therapies, immunodrugs, inhibitors of apoptosis proteins(IAP), kinase inhibitors, tumor kinesin inhibitors, Jak2 inhibitors, mammalian target of rapamycin inhibitors, microRNAs, mitogen-activated extracellular signal-regulated kinase inhibitors, poly ADP (adenosine diphosphate)-ribose polymerase (PARP) inhibitors, platinum chemotherapeutic agents, polo-like kinase (Plk) inhibitors, phosphoinositol-3 kinase inhibitors, proteasome inhibitors, small interfering ribonucleic acid(siRNA), topoisomerase inhibitors, ubiquitin ligase inhibitors, and the like, as well as one or more combinations of these agents.

As used herein, the term "effective amount" refers to an amount of a compound or composition sufficient to significantly and positively alter the symptoms and/or conditions to be treated (eg, to provide a positive clinical response). An effective amount of an active ingredient used in a pharmaceutical composition will vary with the particular symptom being treated, the severity of the symptom, the duration of the treatment, the nature of the synchronous treatment, the one or more specific active ingredients used, the one or more pharmaceutically acceptable excipients/carriers used, the knowledge and professional skills of the attending physician, and the like.

In particular, an effective amount of the compound of formula (I) used in cancer treatment is an amount sufficient to relieve cancer symptoms of human, to slow down the progression of cancer, or to reduce the risk of symptom deterioration in patients suffering from cancer.

### Explanation of the Terms

Unless stated to the contrary, some of the terms used in the description and the claims herein are defined as follows:
"Alkyl" refers to a saturated aliphatic hydrocarbon group, including a saturated linear or branched monovalent hydrocarbon group of 1-20 carbon atoms, or 1-10 carbon atoms, or 1-6 carbon atoms, or 1-4 carbon atoms, or 1-3 carbon atoms, or 1-2 carbon atoms, wherein the alkyl group may be independently optionally substituted with one or more substituents described herein. Further examples of alkyl groups include, but are not limited to, methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, and the like.

The alkyl group may be optionally substituted or unsubstituted.

"Alkenyl" refers to a linear or branched monovalent hydrocarbon group of 2-12 carbon atoms, or 2-8 carbon atoms, or 2-6 carbon atoms, or 2-4 carbon atoms, wherein at least one C-C is an sp² double bond, wherein the alkenyl group may be independently optionally substituted with one or more substituents described herein, wherein specific examples include, but are not limited to, vinyl, allyl, butenyl, and the like. The alkenyl group may be optionally substituted or unsubstituted.

"Cycloalkyl" refers to a saturated or partially unsaturated monocyclic or polycyclic ringlike hydrocarbon substituent, and the cycloalkyl group comprises 3-20 carbon atoms, preferably 3-12 carbon atoms, more preferably 3-6 carbon atoms. Non-limiting examples of monocyclic cycloalkyl include, but are not limited to, cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like; the polycyclic cycloalkyl groups include spirocyclic, fused-ring, and bridged-ring cycloalkyl groups. The cycloalkyl group may be optionally substituted or unsubstituted.

"Spirocycloalkyl" refers to a polycyclic group of 5-18 members with two or more cyclic structures and one carbon atom (referred to as spiro atom) shared between the monocyclic rings, and one or more double bonds are contained in the rings, but none of the rings have a completely conjugated π-electron aromatic system. Preferably, it is 6-14 membered, more preferably 7-10 membered. The spirocycloalkyl group is divided into single spirocyclic, double spirocyclic or polyspirocyclic alkyl according to the number of spiro atoms shared between rings, preferably it is single spirocyclic or double spirocyclic alkyl groups, and preferably it is 4 membered/5 membered, 4 membered/6 membered, 5membered/5 membered or 5 membered/6 membered. Non-limiting examples of "spirocycloalkyl" include, but are not limited to:

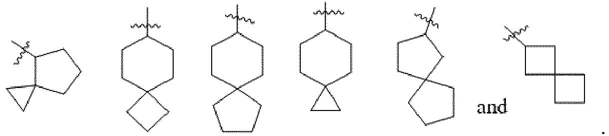

"Fused cycloalkyl" refers to an all-carbon polycyclic group of 5-18 members with two or more cyclic structures sharing a pair of carbon atoms with each other, and one or more rings may contain one or more double bonds, but none of the rings have a completely conjugated π-electron aromatic system. Preferably it is 6-12 members, more preferably 7-10 members. It can be divided into bicyclic, tricyclic, tetracyclic or polycyclic fused cycloalkyl group according to the number of constituting rings, preferably it is bicyclic or tricyclic, and more preferably it is 5 membered/5 membered or 5 membered/6 membered bicyclic alkyl groups. Non-limiting examples of "fused cycloalkyl" include, but are not limited to:

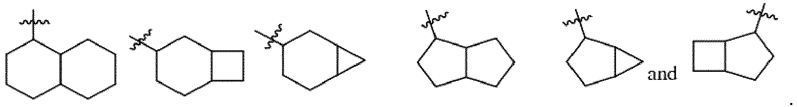

"Bridged cycloalkyl" refers to an all-carbon polycyclic group of 5-18 members with two or more cyclic structures sharing two non-directly-bonded carbon atoms with each other, and one or more rings may contain one or more double bonds, but none of the rings have a completely conjugated π-electron aromatic system. Preferably it is 6-12 members, more preferably 7-10 members. It can be divided into bicyclic, tricyclic, tetracyclic or polycyclic bridged cycloalkyl group according to the number of constituting rings, preferably it is bicyclic, tricyclic or tetracyclic, and more preferably it is bicyclic or tricyclic. Non-limiting examples of "bridged cycloalkyl" include, but are not limited to:

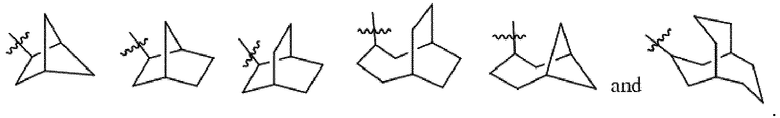

The cycloalkyl ring may be fused to an aryl, heteroaryl, or heterocyclic ring, wherein the ring bonded to the parent structure is the cycloalkyl group, and non-limiting examples include indanyl, tetrahydronaphthyl, benzocycloheptyl, and the like.

"Heterocyclyl", "heterocycle" or "heterocyclic" may be used interchangeably in the present disclosure, all referring to a saturated or partially unsaturated monocyclic, bicyclic, or tricyclic non-aromatic heterocyclic group comprising 3-12 ring atoms, wherein at least one ring atom is a heteroatom, such as oxygen, nitrogen, sulfur atom, and the like. It is preferred to have a 5-7 membered single ring or 7-10 membered double or triple rings, which may contain 1, 2, or 3 atoms selected from nitrogen, oxygen, and/or sulfur. Examples of "heterocyclyl" include, but are not limited to, morpholinyl, oxetanyl, thiomorpholinyl, tetrahydropyranyl, 1,1-dioxo-thiomorpholinyl, piperidinyl, 2-oxo-piperidinyl, pyrrolidinyl, 2-oxo-pyrrolidinyl, piperazine-2-one, 8-oxa-3-aza-bicyclo[3.2.1]octyl and piperazinyl. The heterocyclic ring may be fused to an aryl, heteroaryl, or cycloalkyl ring, wherein the ring bonded to the parent structure is the heterocyclyl group. The heterocyclyl group may be optionally substituted or unsubstituted. "Spiroheterocyclyl" refers to a polycyclic group of 5-18 members with two or more cyclic structures sharing an atom with each other between the monocyclic rings, and one or more double bonds are contained in the rings, but none of the rings have a completely conjugated π-electron aromatic system, wherein one or more ring atoms are selected from nitrogen, oxygen, sulfur, or S(O)ₘ heteroatoms, with the remaining ring atoms being carbon, m=1 or 2. Preferably it is 6-14 membered, more preferably 7-10 membered. The spiroheterocyclyl group is divided into single spiroheterocyclyl, double spiroheterocyclyl or polyspiroheterocyclyl according to the number of spiro atoms shared between rings, and preferably it is single spiroheterocyclyl or double spiroheterocyclyl. More preferably it is 4 membered/4 membered, 4 membered/5 membered, 4 membered/6 membered, 5 membered/5 membered or 5 membered/6 membered single spiroheterocyclyl. Non-limiting examples of "spiroheterocyclyl" include, but are not limited to:

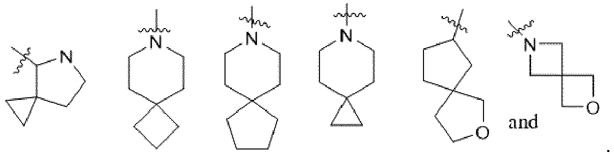

"Fused heterocyclyl" refers to an all-carbon polycyclic group comprising two or more cyclic structures sharing a pair of atoms with each other, and one or more rings may contain one or more double bonds, but none of the rings have a completely conjugated π-electron aromatic system, wherein one or more ring atoms are selected from nitrogen, oxygen, sulfur, or S(O)ₘ heteroatoms, with the remaining ring atoms being carbon, m=1 or 2. Preferably it is 6-14 membered, more preferably 7-10 membered. It can be divided into bicyclic, tricyclic, tetracyclic or polycyclic fused heterocyclic group according to the number of constituting rings, preferably it is bicyclic or tricyclic, and more preferably it is 5 membered/5 membered or 5 membered/6 membered bicyclic fused heterocyclyl. Non-limiting examples of "fused heterocyclyl" include, but are not limited to:

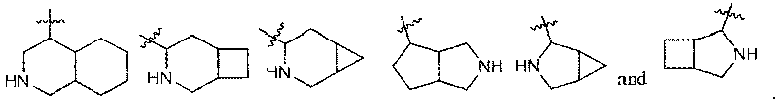

"Bridged heterocyclyl" refers to an polycyclic group of 5-18 members with two or more cyclic structures sharing two non-directly-bonded atoms with each other, and one or more rings may contain one or more double bonds, but none of the rings have a completely conjugated π-electron aromatic system, wherein one or more ring atoms are selected from nitrogen, oxygen, sulfur, or S(O)ₘ heteroatoms, with the remaining ring atoms being carbon, m=1 or 2. Preferably it is 6-14 membered, more preferably 7-10 membered. It can be divided into bicyclic, tricyclic, tetracyclic or polycyclic bridged heterocyclyl according to the number of constituting rings, preferably it is bicyclic, tricyclic, or tetracyclic, and more preferably it is bicyclic or tricyclic. Non-limiting examples of "bridge heterocyclyl" include, but are not limited to:

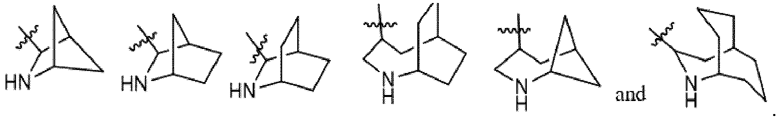

"Aryl" refers to a carbocyclic aromatic system comprising one or two rings, wherein the rings can be bonded together in a fused manner. The term "aryl" includes aromatic groups such as phenyl, naphthyl, tetrahydronaphthyl. Preferred aryl groups are C₆-C₁₀ aryl groups, more preferably aryl groups are phenyl and naphthyl, and most preferably it is phenyl. The aryl group may be substituted or unsubstituted. The "aryl" group may be fused with heteroaryl, heterocyclyl, or cycloalkyl, wherein the ring bonded to the parent structure is the aryl ring. Non-limiting examples include, but is not limited to:

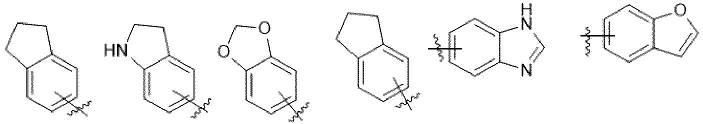

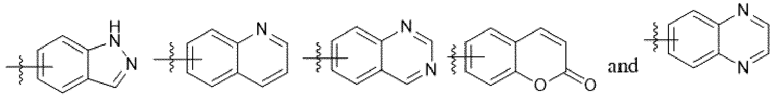

"Heteroaryl" refers to an aromatic 5-6 membered monocyclic or 9-10 membered bicyclic ring, which may contain 1 to 4 atoms selected from nitrogen, oxygen, and/or sulfur. Examples of "heteroaryl" include, but are not limited to, furyl, pyridyl, 2-oxo-1,2-dihydropyridyl, pyridazinyl, pyrimidinyl, pyrazinyl, thienyl, isoxazolyl, oxazolyl, oxadiazolyl, imidazolyl, pyrrolyl, pyrazolyl, triazolyl, tetrazolyl, thiazolyl, isoxazolyl, 1,2,3-thiadiazolyl, benzodioxolyl, benzimidazolyl, indolyl, isoindolyl, 1,3-dioxo-isoindolyl, quinolinyl, indazolyl, benzoisothiazolyl, benzoxazolyl, and benzisoxazolyl. Heteroaryl may be optionally substituted or unsubstituted. The heteroaryl ring may be fused to aryl, heterocyclyl, or cycloalkyl ring, wherein the ring bonded to the parent structure is the heteroaryl ring. Non-limiting examples include, but are not limited to:

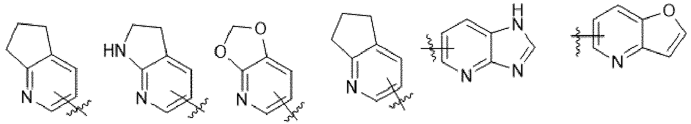

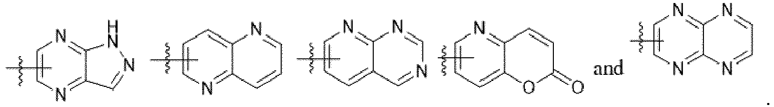

"Alkoxy" refers to a group of (alkyl-O-). Wherein, the alkyl group refers to the related defination herein. C₁-C₆ alkoxy is preferred. Its examples include, but are not limited to, methoxy, ethoxy, n-propoxy, isopropoxy, n-butoxy, isobutoxy, tert-butoxy, and the like.

"Haloalkyl" refers to an alkyl group having one or more halogen substituents, wherein the alkyl group has the meaning as described in the present disclosure. Examples of haloalkyl include, but are not limited to, fluoromethyl, difluoromethyl, trifluoromethyl, perfluoroethyl, 1,1-dichloroethyl, 1,2-dichloropropyl, and the like.

"Hydroxyl" refers to -OH group.

"Halogen" refers to fluorine, chlorine, bromine, and iodine, preferably fluorine, chlorine, and bromine.

"Amino" refers to -NH₂.

"Cyano" refers to -CN.

"Nitro" refers to -NO₂.

"Benzyl" refers to -CH₂-phenyl.

"Carboxyl" refers to -C(O)OH.

"Acetyl" refers to -C(O)CH₃ or Ac.

"Carboxylate" refers to -C(O)O(alkyl) or (cycloalkyl), wherein the definition of alkyl and cycloalkyl is as described above.

"Optional" means that the event described may, but need not, occur. For example, the description

"AR¹ is optionally substituted with one to more R^{c}" contains the case where the AR¹ group may be substituted with 1 to more R^{c} or not substituted with R^{c}.

"Substituted" means that one or more hydrogen atoms, preferably at most 5, more preferably 1-3 hydrogen atoms in a group are substituted with a corresponding number of substituents independently from each other. It goes without saying that the substituents are only at their possible chemical positions, and those skilled in the art can determine (through experimentation or theory) possible or impossible substitutions without excessive effort. For example, an amino or hydroxyl group with free hydrogen bonded to carbon atoms with an unsaturated (eg, olefinic) bond may be unstable.

As described herein, "substitution" or "substituted", as not particularly pointed out, means that the group can be substituted with one or more groups selected from the followings: alkyl, alkenyl, alkynyl, alkoxy, alkylthio, alkylamino, halogen, sulfhydryl, hydroxyl, nitro, cyano, cycloalkyl, heterocyclyl, aryl, heteroaryl, cycloalkoxy, heterocycloalkoxy, cycloalkylthio, heterocyclenylthio, amino, haloalkyl, hydroxyalkyl, carboxyl, carboxylate, =O, -C(O)R^{b}, -OC(O)R^{b}, -NR^{b}R^{b}, -C(O)NR^{b}R^{b}, -NR^{b}C(O)R^{b}, -S(O)NR^{b}R^{b}, or -S(O)₂NR^{b}R^{b}, wherein the definition of R^{b} is as described in general formula (I).

The use of stereochemical definitions and conventions in the present disclosure generally refers to the following document:
S.P.Parker,Ed.,McGraw-Hill Dictionary of Chemical Terms(1984)McGraw-HillBook Company,New York;and Eliel,E.and Wilen,S.,"Stereochemistry of Organic Compounds", John Wiley&Sons,Inc.,New York,1994. The compounds of the present disclosure may contain asymmetric centers or chiral centers, and therefore there are different stereoisomers. All stereoisomeric forms of the compounds disclosed herein include, but not limited to, diastereomers, enantiomers and atropisomers, as well as mixtures thereof such as racemic mixtures, forming part of the present invention. Diastereomeric mixtures can be separated into their individual diastereoisomers on the basis of their physical and chemical differences by chromatography, crystallization, distillation, sublimation or the like. Enantiomers can be separated by converting the enantiomeric mixture into a diastereomeric mixture in a manner of reacting with an appropriate optically active compound (e.g., chiral auxiliary such as a chiral alcohol or Mosher's acid chloride), separating the diastereoisomers and converting the individual diastereoisomers to the corresponding pure enantiomers. The intermediates and compounds of the present disclosure may exist in different tautomeric forms and all such forms are encompassed within the scope of the present disclosure. Many organic compounds exist in optically active forms, i.e., they have the ability to rotate the plane of plane-polarized light. In describing an optically active compound, the prefixes D and L, or R and S, are used to denote the absolute configuration of the molecule about its chiral center(s). The prefixes d and l, or (+) and (-) are employed to designate the sign of rotation of plane-polarized light by the compound, (-) or l meaning that the compound is levorotatory, (+) or d meaning that the compound is dextrorotatory. The atoms or atomic groups of these stereoisomers are bonded in the same order, but their three-dimensional structures are different. A specific stereoisomer may be an enantiomer, and a mixture of such isomers is often called an enantiomeric mixture. A 50:50 mixture of enantiomers is referred to as a racemic mixture or a racemate, which may result in no stereoselection or stereospecificity in a chemical reaction process. The term "racemic mixture" or "racemate" refers to an equimolar mixture of two enantiomers, devoid of optical activity.

"Tautomer" or "tautomeric form" refers to structural isomers of different energies which are interconvertible via a low energy barrier. For example, proton tautomers (i.e., prototropic tautomers) include interconversions via migration of a proton, such as keto-enol and imine-enamine isomerizations. Valence tautomers include interconversions by reorganization of the bonding electrons. Unless otherwise indicated, structure formulas depicted in the present disclosure include all isomeric forms (e.g., enantiomeric, diastereomeric, and geometric): for example, R and S configurations with asymmetric center, (Z) and (E) isomers of double bond, and (Z) and (E) conformational isomers. Therefore, a single stereochemical isomer as well as its enantiomer, diastereomer, or geometric isomer mixtures of the compounds of the present disclosure are within the scope of the present disclosure.

"Pharmaceutically acceptable salt" refers to a salt of the compound of the present disclosure that has safety and efficacy when used in humans or animals. The salt of the compound can be obtained by obtaining a corresponding addition salt with a sufficient amount of base or acid in a pure solution or a suitable inert dissolution. Pharmaceutically acceptable base addition salts include sodium, potassium, calcium, ammonium, organic ammonia, or magnesium salts, etc., pharmaceutically acceptable acid addition salts include inorganic acid salts and organic acid salts including hydrochloric acid, hydrobromic acid, carbonic acid, hydrogen carbonate, phosphoric acid, monohydrogen phosphate, dihydrogen phosphate, sulfuric acid, monohydrogen sulfate, acetic acid, maleic acid, malonic acid, succinic acid, fumaric acid, phthalic acid, benzenesulfonic acid, p-toluenesulfonic acid, citric acid, methanesulfonic acid, and the like (see Berge et al.,"Pharmaceutical Salts", Journal of Pharmaceutical Science 66:1-19(1977)).

Compared with the prior art, the polycyclic amide derivative used as a CDK9 inhibitor provided by the present disclosure has the following beneficial effects:
The present disclosure provides a novel structure CDK9 inhibitor. Test results show that the polycyclic amide derivative exhibits excellent CDK9 kinase inhibitory activity, exhibits excellent safety and selectivity at the same time, and can be used for preparing medicaments for treating cancers, especially blood cancers including acute myeloid leukemia, multiple myeloma, chronic lymphocytic leukemia, follicular lymphoma, etc., and solid tumors including breast cancer, prostate cancer, ovarian cancer, hepatocellular carcinoma, pancreatic cancer, renal cancer, gastric cancer, colorectal cancer, lung cancer and other diseases.

### BRIEF DESCRIPTION OF THE DRAWINGS

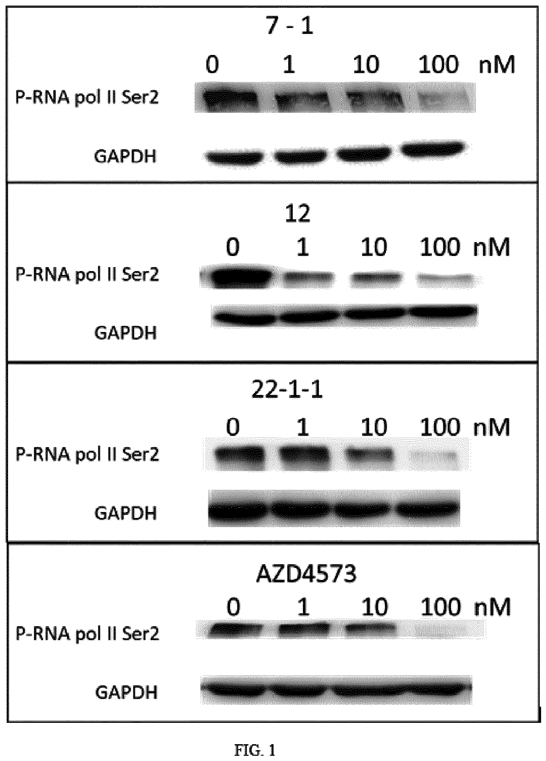

FIG. 1 is an SDS-PAGE electrophoresis detection diagram of the inhibition effect of the compounds 7-1, 12, 22-1-1, and AZD4573 on the phosphorylation of Mv 4-11 cell RNA pol II Ser2 according to the present disclosure.

### DETAILED DESCRIPTION OF THE PREFERRED EMBODIMENTS

The method of the present disclosure is described below through specific embodiments, so that the technical solution of the present disclosure is easier to understand and master, but the present disclosure is not limited thereto. The ¹H NMR spectrum in the following embodiments was determined by a Bruker instrument (400 MHz), and the chemical shift was expressed in ppm. Tetramethylsilane internal standard (0.00 ppm) was used. Expression of ¹H NMR: s=singlet, d=doublet, t=triplet, q=quartet, m=multiplet, br=broadening, dd=doublet of doublet, dt = doublet of triplet. If a coupling constant is provided, its unit is Hz.

The mass spectrum was determined by LC/MS instrument, and the ionization mode was ESI. High performance liquid chromatograph model: Agilent 1260, Thermo Fisher U3000; column model: Waters xbrige C18 (4.6*150 mm, 3.5 µm); mobile phase: A:ACN, B:Water (0.1% H₃PO₄), flow rate: 1.0 mL/min; gradient: 5% A for 1 min, increase to 20%A within 4 min, increase to 80%A within 8 min, 80%A for 2min, back to 5%A within 0.1 min; wavelength: 220 nm; column temperature box: 35°C.

Thin-layer chromatography silica gel plate uses Yantai Huanghai HSGF 254 or Qingdao GF254 silica gel plate, the size of the silica gel plate used for thin layer chromatography (TLC) is 0.2 mm to 0.3 mm, and the size for product separating and purifing thin-layer chromatography is 0.4 mm to 0.5 mm.

Column chromatography generally uses Yantai Huanghai silica gel 200-300 mesh silica gel as a carrier.

In the following embodiments, unless otherwise indicated, all temperatures are in degrees Celsius; unless otherwise indicated, all starting materials and reagents are commercially available or synthesized according to known methods, and commercially available starting materials and reagents are directly used without further purification; unless otherwise indicated, commercially available manufacturers include, but are not limited to, National Medicine Group, J&K Scientific Ltd., TCI (Shanghai) Development Co. Ltd., Shanghai Bide Pharmatech Ltd., Meryer (Shanghai) Chemical Technology Co., Ltd., et al.
CD₃OD: deuterated methanol
CDCl₃: deuterated chloroform.
DMSO-*d*₆: deuterated dimethyl sulfoxide
Pd₂(dba)₃: tris(dibenzylideneacetone)dipalladium
Pd(dppf)Cl₂: [1,1'-bis(diphenylphosphino)ferrocene]palladium dichloride
XantPhos: 4,5-bis-diphenylphosphine-9,9-dimethylxanthene
XPhos: 2-dicyclohexylphosphine -2,4,6-triisopropylbiphenyl
HATU: 2-(7-oxidobenzotriazol)-*N*,*N*,*N'*,*N'*-tetramethyluronium hexafluorophosphate
TLC: Thin Layer Chromatography
HPLC: High Performance Liquid Chromatography
Purity: purity
&: and
The hydrogen atmosphere means that the reaction flask is connected to a hydrogen balloon with a volume of about 1 L.

If no special statement is given in the embodiments, the solution in the reaction refers to an aqueous solution.

If no special statement is given in the embodiments, the temperature of the reaction is room temperature, and it is 20°C to 30°C.

Thin Layer Chromatography (TLC) was used for the monitoring of the reaction process in the embodiments. The expanding agent used in the reaction, the eluent system of column chromatography used for purifying the compound or the developing agent system of the thin-layer chromatography includes: A: petroleum ether and ethyl acetate system; B: dichloromethane and methanol system; C: n-hexane: ethyl acetate; wherein the volume ratio of the solvents is different depending on the different polarity of the compound, and a small amount of acidic or alkaline reagent such as acetic acid, triethylamine or the like can also be added for adjustment.

### Preparation of Intermediates

### Intermediate 1

### 5,5-dimethyl-3-(4,4,5,5-tetramethyl-l,3,2-dioxaborolan-2-yl)-5,6-dihydro-4H-pyrrolo[1,2-b]pyraz ole IN -1

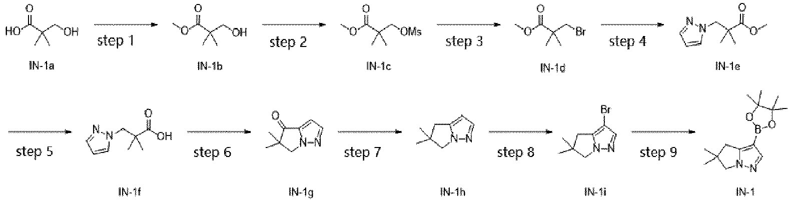

### Step 1: methyl 2,2-dimethyl-3-hydroxypropanoate IN-1b

2,2-dimethyl-3-hydroxypropionic acid **IN-1a** (100.0 g, 0.85 mol) was dissolved in methanol (1 L), concentrated sulfuric acid (91.1 g, 0.36 mol) was added dropwise at room temperature, the temperature was increased to 75°C for reaction for 4 hours, and the reaction was complete shown by TLC. The reaction solution was concentrated, a saturated sodium bicarbonate aqueous solution was added to adjust the pH to weak base, it was extracted with ethyl acetate, and the organic phases were combined, washed with saturated salt water, dried over anhydrous sodium sulfate, and concentrated to obtain the light yellow transparent liquid title compound **IN-1b** (93.0 g, crude product), which was directly used for the next step.

### Step 2: 2,2-dimethyl-3-((methylsulfonyl)oxy)propionic acid methyl ester IN-1c

Compound **IN-1b** (93.0 g, crude product) was dissolved in dichloromethane (500 mL), triethylamine (85.9 g, 0.85 mol) was added, the temperature was lowered to -5°C, under nitrogen protection, methylsulfonyl chloride (88.2 g, 0.77 mol) was added dropwise, after the addition the temperature was lifted to room temperature for reaction for 2 hours, and the reaction was complete shown by TLC. The reaction solution was poured into water and extracted with ethyl acetate, the organic phases were combined, washed with saturated salt water, dried over anhydrous sodium sulfate and concentrated, and the crude product was separated and purified by silica gel column chromatography to obtain the light yellow transparent liquid title compound **IN-1c** (121.0 g, crude product), which was directly used for the next step.

### Step 3: 3-bromo-2,2-dimethylpropionic acid methyl ester IN-1d

Compound **IN-1c** (121.0 g, crude product) was dissolved in *N*,*N*-dimethylformamide (800 mL), lithium bromide (101.0 g, 1.16 mol) was added at room temperature, the temperature was lifted to 100°C for reaction overnight, and the reaction was complete shown by TLC. The reaction solution was cooled to room temperature, poured into water and extracted with ethyl acetate, the organic phases were combined, washed with saturated salt water, dried over anhydrous sodium sulfate and concentrated, and the crude product was separated and purified by silica gel column chromatography to obtain the light yellow transparent liquid title compound **IN-1d** (85.0 g, three-step yield 51%).

¹H NMR (400 MHz, CDCl₃) δ 3.69 (s, 3H), 3.48 (s, 2H), 1.29 (s, 6H).

### Step 4: 2,2-dimethyl-3-(1H-pyrazol-1-yl)propionic acid methyl ester IN-1e

Compound **IN-1d** (85.0 g, 0.44 mol) was dissolved in *N*,*N*-dimethylformamide (500 mL), cesium carbonate (173.0 g, 0.53 mol) and imidazole (32.6 g, 0.48 mol) were added at room temperature, the temperature was lifted to 85°C for reaction overnight, and the reaction was complete shown by TLC. The reaction solution was cooled to room temperature, poured into water and extracted with ethyl acetate, the organic phases were combined, washed with saturated salt water, dried over anhydrous sodium sulfate and concentrated, and the crude product was separated and purified by silica gel column chromatography to obtain the light yellow transparent liquid title compound **IN-1e** (41.0 g, yield 52%).

LC-MS: m/z=183.2 [M+H]⁺

### Step 5: 2,2-dimethyl-3-(1H-pyrazol-1-yl)propionic acid IN-1f

Compound **IN-1e** (41.0 g, 0.22 mol) was dissolved in a mixed solvent of tetrahydrofuran (150 mL) and methanol (100 mL), a water (100 mL) solution of sodium hydroxide (18.0 g, 0.45 mol) was added, it was stirred at room temperature for 1 hour, and the reaction was complete shown by TLC. The reaction solution was concentrated to remove tetrahydrofuran and methanol, added with water and extracted with ethyl acetate, and the organic phases were combined, washed with saturated salt water, dried over anhydrous sodium sulfate and concentrated to obtain the white solid title compound **IN-1f** (33.0 g, crude product), which was directly used for the next step.

¹H NMR (400 MHz, DMSO-*d*₆) δ 12.44 (s, 1H), 7.61 (t, *J* = 1.6 Hz, 1H), 7.41 (d, *J* = 1.6 Hz, 1H), 6.21 (t, *J* = 2.0 Hz, 1H), 4.24 (s, 2H), 1.06 (s, 6H).

### Step 6: 5,5-dimethyl-5,6-dihydro-4H-pyrrolo[1,2-b]pyrazol-4-one IN-1g

Compound **IN-1f** (33.0 g, crude product) was dissolved in anhydrous tetrahydrofuran (300 mL), under nitrogen protection, the temperature was lowered to -65°C, n-butyllithium (160 mL, 0.4 mol, 2.5 M tetrahydrofuran solution) was slowly added dropwise, after the dropwise addition the temperature was lifted to -45°C for reaction for 1 hour, then the temperature was lifted to room temperature, it was stirred overnight, and the reaction was complete shown by TLC. The reaction solution was slowly poured into water and extracted with ethyl acetate, the organic phases were combined, washed with saturated salt water, dried over anhydrous sodium sulfate and concentrated, and the crude product was separated and purified by silica gel column chromatography to obtain the light yellow liquid title compound **IN-1g** (18.1 g, two-step yield 55%).

### Step 7: 5,5-dimethyl-5,6-dihydro-4H-pyrrolo[1,2-b]pyrazole IN-1h

Compound **IN-1g** (18.1 g, 0.12 mol) was dissolved in diethylene glycol (300 mL), hydrazine hydrate (30 mL, 0.6 mol, 85%) was added at room temperature, the temperature was lifted to 180°C (internal temperature 156°C) for reaction for 2 hours, the temperature was lowered to 150°C, potassium hydroxide (23.6 g, 0.42 mol) was slowly added, after the addition the temperature was lifted to 180°C for reaction for 5 hours, and the reaction was complete shown by TLC. The reaction solution was cooled to room temperature, added with water, adjusted to pH=6-7 with a hydrochloric acid (3M) solution and extracted with ethyl acetate,, the organic phases were combined, wash with saturated salt water, dried over anhydrous sodium sulfate and concentrated, and the crude product was separated and purified by silica gel column chromatography to obtain the light yellow liquid title compound **IN-1h** (7.8 g, yield 48%).

### Step 8: 3-bromo-5,5-dimethyl-5,6-dihydro-4H-pyrrolo[1,2-b]pyrazole IN-1i

Compound **IN-1h** (7.1 g, 52.1 mmol) was dissolved in dichloromethane (60 mL), N-bromosuccinimide (9.3 g, 52.2 mmol) was added, the reaction was carried out overnight at room temperature, and the reaction was complete shown by TLC. The reaction solution was added with water and extracted with dichloromethane, the organic phases were combined, washed with saturated salt water, dried over anhydrous sodium sulfate and concentrated, and the crude product was separated and purified by silica gel column chromatography to obtain the yellow liquid title compound **IN-1i** (7.8 g, yield 48%).

### Step 9: 5,5-dimethyl-3-(4,4,5,5-tetramethyl-1,3,2-dioxaborolan-2-yl)-5,6-dihydro-4H-pyrrolo[1,2-b]pyraz ole IN-1

Compound **IN-1i** (5.5 g, 25.6 mmol) was dissolved in 1,4-dioxane (50 mL), bis(pinacolato)diboron (9.7 g, 38.2 mmol), potassium acetate (5.0 g, 51.0 mmol) and Pd(dppf)Cl₂ dichloromethane complex (244 mg, 0.3 mmol) were added, under nitrogen protection, the temperature was lifted to 85°C for reaction overnight, and the reaction was complete shown by TLC. The reaction solution was cooled to room temperature, added with water and extracted with ethyl acetate, the organic phases were combined, washed with saturated salt water, dried over anhydrous sodium sulfate and concentrated, and the crude product was separated and purified by silica gel column chromatography to obtain the light yellow liquid title compound **IN-1** (2.4 g, yield 36%).

¹H NMR (400 MHz, CDCl₃) δ 7.77 (s, 1H), 3.88 (s, 2H), 2.79 (s, 2H), 1.26 (s, 12H), 1.24 (s, 6H).

### Intermediate 2

### 7-bromo-2,2-dimethyl-2,3-dihydro-1H-pyrrolizine-5-carbonitrile IN-2

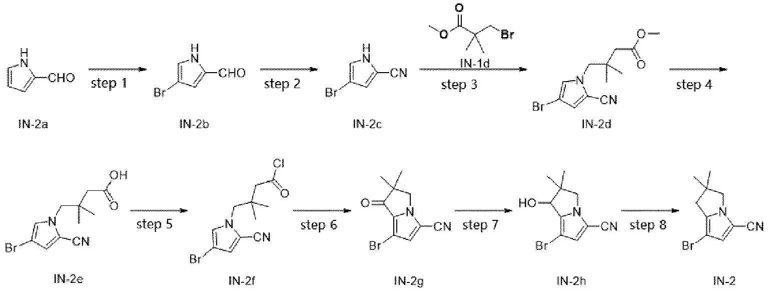

### Step 1: 4-bromo-1H-pyrrole-2-carbaldehyde IN-2b

1*H*-pyrrole-2-carbaldehyde **IN-1a** (10.0 g, 105.3 mmol) was dissolved in tetrahydrofuran (100 mL), under nitrogen protection, it was cooled to 0°C, N-bromosuccinimide (19.7 g, 110.5 mmol) was added, the reaction was kept at 0°C for 30 minutes, and the disappearance of the starting material was shown by TLC. The reaction solution was concentrated to remove organic solvent, diluted with water and extracted with ethyl acetate, and the organic phases were combined, washed with saturated salt water, dried over anhydrous sodium sulfate and concentrated, and the crude product was purified by silica gel column to obtain the white solid title compound **IN-2b** (12.6 g, yield 69%).

LC-MS: m/z=174.0[M+H]⁺

### Step 2: 4-bromo-1H-pyrrole-2-carbonitrile IN-2e

Compound **IN-2b** (5.0 g, 28.7 mmol) was dissolved in water (150 mL), O-hydroxylamine sulfonic acid (11.4 g, 100.6 mmol) was added, it was stirred overnight at room temperature, and the disappearance of the starting material was shown by TLC (the starting material and the product were distinguished by chromogenic agent for aldehyde and ketone). The reaction solution was cooled to 0°C, adjusted to pH=13-14 with potassium hydroxide solution (4N) and extracted with dichloromethane, the organic phases were combined, washed with saturated salt water, dried over anhydrous sodium sulfate and concentrated, and the crude product was purified by silica gel column to obtain the white solid title compound **IN-2c** (4.2 g, yield 85.0%).

LC-MS: m/z=168.9[M-H]⁻

### Step3: 4-(4-bromo-2-cyano-1H-pyrrol-1-yl)-3,3-dimethylbutyric acid methyl ester IN-2d

Compound **IN-2c** (1.0 g, 5.8 mmol) was dispersed in *N*,*N*-dimethylformamide (15 mL), potassium carbonate (1.6 g, 11.6 mmol) and Compound **IN-1d** (1.7 g, 8.8 mmol) were added at room temperature, the temperature was lifted to 85°C, it was stirred overnight, and the complete reaction of the starting material was shown by TLC. The reaction solution was cooled to room temperature, diluted with water and extracted with ethyl acetate, the organic phases were combined, washed with water, washed with saturated salt water, dried over anhydrous sodium sulfate and concentrated, and the crude product was purified by silica gel column chromatography to obtain the yellow liquid title compound **IN-2d** (1.7 g, yield 97%).

### Step 4: 4-(4-bromo-2-cyano-1H-pyrrol-1-yl)-3,3-dimethylbutyric acid IN-2e

Compound **IN-2d** (1.7 g, 5.7 mmol) was dispersed in tetrahydrofuran (15 mL) and methanol (15 mL), it was cooled to 0°C, a sodium hydroxide aqueous solution (3 mL, 12 mmol, 4 M) was added, the temperature was slowly lifted to room temperature, it was stirred for 30 minutes, and the complete reaction of the starting material was shown by TLC. The reaction solution was concentrated to remove the organic solvent, diluted with water and extracted with ethyl acetate, the organic phase was discarded, the aqueous phase was adjusted to be pH acidic with dilute hydrochloric acid (1N) and extracted with ethyl acetate, and the organic phases were combined, washed with saturated salt water, dried over anhydrous sodium sulfate and concentrated to obtain the yellow oil-like title compound **IN-2e** (1.4 g, crude product), which was directly used for the next step.

### Step 5: 4-(4-bromo-2-cyano-1H-pyrrol-1-yl)-3,3-dimethylbutyryl chloride IN-2f

Compound **IN-2e** (1.4 g, crude product) was dispersed in dichloromethane (30 mL), N,N-dimethylformamide (1 mL) was added at room temperature, it was cooled to 0°C, oxalyl chloride (980 mg, 7.7 mmol) was added dropwise, after the dropwise addition the temperature was slowly lifted to room temperature, it is stirred for 30 minutes, and the complete reaction of the starting material was shown by TLC (methanol quenching point plate). The reaction solution was concentrated to obtain the yellow oil-like title compound **IN-2f** (1.5 g, crude product), which was directly used for the next step.

### Step 6: 7-bromo-2,2-dimethyl-1-oxo-2,3-dihydro-1H-pyrrolizine-5-carbonitrile IN-2g

Aluminum trichloride (1.4 g, 5.8 mmol) was dispersed in dichloromethane (15m), it was cooled to 0°C, a dichloromethane (15 mL) solution of Compound **IN-2f** (1.5 g, crude product) was added dropwise under nitrogen protection, after the dropwise addition the temperature is naturally lifted to room temperature, it was stirred overnight, and the complete reaction of the starting material was shown by TLC. The reaction solution was poured into ice water for quenching, hydrochloric acid (1N) was added to adjust the pH to be acidic, it was extracted with dichloromethane, the organic phases were combined, washed with saturated salt water, dried over anhydrous sodium sulfate and concentrated, and the crude product was purified by silica gel column chromatography to obtain the white solid title compound **IN-2g** (900 mg, three-step yield 62%).

¹H NMR (400 MHz, CDCl₃) δ 7.00 (s, 1H), 4.17 (s, 2H), 1.38 (s, 6H).

### Step 7: 7-bromo-1-hydroxy-2,2-dimethyl-2,3-dihydro-1H-pyrrolizine-5-carbonitrile IN-2h

Compound **IN-2g** (870 mg, 3.4 mmol) was dispersed in methanol (15mL), it was cooled to 0°C, sodium borohydride (330 mg, 8.7 mmol) was added, the temperature was lifted to room temperature, it was stirred for 1 hour, and the complete reaction of the starting material was shown by TLC. The reaction solution was quenched with water, concentrated to remove methanol and extracted with ethyl acetate, and the organic phases were combined, washed with saturated salt water, dried over anhydrous sodium sulfate and concentrated to obtain the title compound **IN-2h** (1.0 g, crude product), which was directly used for the next step.

### Step 8: 7-bromo-2,2-dimethyl-2,3-dihydro-1H-pyrrolizine-5-carbonitrile IN-2

Compound **IN-2h** (1.0 g, crude product) was dispersed in trifluoroacetic acid (20 mL), it was cooled to 0°C, with nitrogen replacement, triethylsilane (1.4 g, 12 mmol) was added dropwise, after the addition it was stirred at 0°C for 1 hour, and the complete reaction of the starting material was shown by TLC. The reaction solution was concentrated, adjusted to be pH alkaline with a saturated sodium bicarbonate aqueous solution and extracted with ethyl acetate, and the organic phases were combined, washed with saturated salt water, dried over anhydrous sodium sulfate and concentrated to obtain the yellow liquid title compound **IN-2** (370 mg, crude product), which was directly used for the next step.

LC-MS: m/z=239.3[M+H] ⁺

### Intermediate 3

### (1R,3S)-3-((tert-butoxycarbonyl)amino)cyclohexane-1-carboxylic acid benzyl ester (assumed) IN-3-1

### (1S,3R)-3-((tert-butoxycarbonyl) amino)cyclohexane-1-carboxylic acid benzyl ester (assumed) IN-3-2

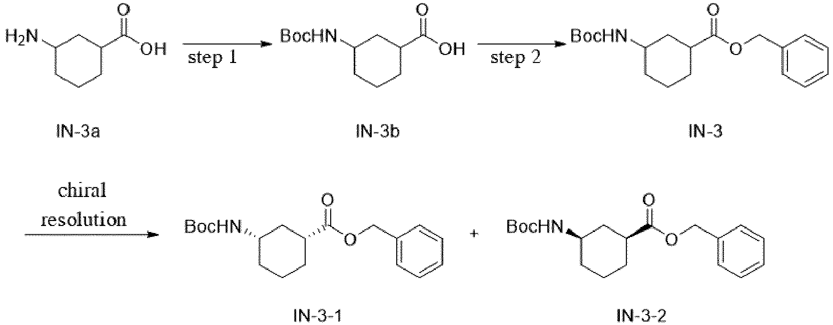

### Step 1: 3-((tert-butoxycarbonyl)amino)cyclohexane-1-carboxylic acid IN-3b

3-aminocyclohexanecarboxylic acid **IN-3a** (10.0 g, 69.8 mmol) was dissolved in 1,4-dioxane (100 mL), a water (100 mL) solution of sodium hydroxide (4.2 g, 105 mmol) was added, di-tert-butyl dicarbonate (19.8 g, 90.7 mmol) was added after all the solids were dissolved, it was stirring overnight at room temperature, and the reaction was complete shown by TLC. The reaction solution was added with hydrochloric acid (3M) to adjust pH=3-4 and extracted with ethyl acetate, and the organic phases were combined, washed with saturated salt water, dried over anhydrous sodium sulfate and concentrated to obtain the white solid title compound **IN-3b** (16.0 g, crude product), which was directly used for the next step.

### Step 2: (1R,3S)-3-((tert-butoxycarbonyl)amino)cyclohexane-1-carboxylic acid benzyl ester (assumed) IN-3-1 & (1S,3R)-3-((tert-butoxycarbonyl)amino)cyclohexane-1-carboxylic acid benzyl ester (assumed) IN-3-2

Compound **IN-3b** (20.2 g, crude product) was dissolved in N,N-dimethylformamide (200 mL), it was cooled to 0°C, cesium carbonate (40.3 g, 123.7 mmol) was added, under nitrogen protection, benzyl bromide (14.8 g, 86.5 mmol) was added dropwise slowly, after the dropwise addition the temperature was slowly lifted to room temperature for reaction for 2 hours, and the reaction was complete shown by TLC. The reaction solution was added with water and extracted with ethyl acetate, the organic phases were combined, washed with saturated salt water, dried over anhydrous sodium sulfate and concentrated, and the crude product was separated and purified by silica gel column chromatography to obtain the white solid compound **IN-3** (24.9 g, two-step yield 82%). 5.9 g was taked to chiral resolution (DAICEL AD-H, 30*250mm, Sum, 30mL/min, IPA:Hexane=5:95) to obtain Compound **IN-3-1** (peak 1, RT 21.2 min) (2.7 g, yield 46%) and Compound **IN-3-2** (peak 2, RT 29.1 min) (1.5 g, +27.1°, ee=100%, yield 25%). According to the RT data of the literature, Compound **IN-3-2** is the intermediate of the desired configuration.

LC-MS: m/z=234.1 [M-Boc+H]⁺

### Intermediate 4

### 5-oxo-octahydro-cyclopentadiene-2-carboxylic acid methyl ester IN-4

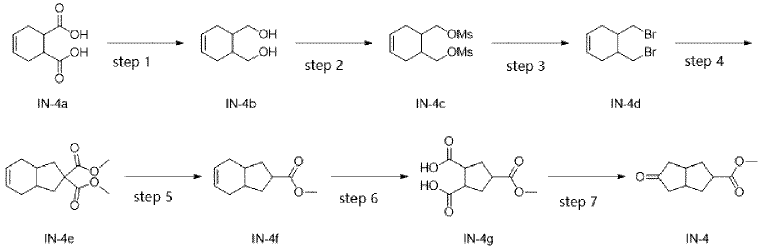

### Step 1: cyclohex-4-ene-1,2-diyldimethanol IN-4b

4-cyclohexene-1,2-dicarboxylic acid **IN-4a** (20.0 g, 117.5 mmol) was dissolved in tetrahydrofuran (400 mL), it was cooled to 0°C, lithium aluminum hydride (17.9 g, 471.7 mmol) was added in batches, after the addition the temperature was lifted to 80°C, it was stirred for 2 hours, and the reaction was complete shown by TLC. The reaction solution was cooled to 0°C, water (18 mL) and a sodium hydroxide aqueous solution (18 mL, 1.5N) were added in sequence, it was stirred for 10 minutes, water (50 mL) was added, it was stirred for 10 minutes and filtered, the filter cake was washed with tetrahydrofuran, and the filtrate was concentrated to obtain the light yellow liquid title compound **IN-4b** (16.5 g, crude product), which was directly used for the next step.

¹H NMR (400 MHz, DMSO-*d₆*) δ 5.57 (s, 2H), 4.41 (t, *J* = 4.2 Hz, 2H), 3.42-3.37 (m, 2H), 3.28-3.22 (m, 2H), 1.99-1.85 (m, 6H).

### Step 2: cyclohex-4-ene-1,2-diylbis(methylene)dimesylate IN-4c

Compound **IN-4b** (16.5 g, crude product) was dissolved in dichloromethane (200 mL), it was cooled to 0°C, triethylamine (31.7 g, 313.3 mmol) and methylsulfonyl chloride (33.0 g, 288.1 mmol) were added in sequence, the temperature was lifted to room temperature, it was stirred for 2 hours, and the reaction was complete shown by TLC. The reaction solution was quenched with water and extracted with dichloromethane, and the organic phases were combined, washed with water, washed with saturated salt water, dried over anhydrous sodium sulfate and concentrated to obtain the title compound **IN-4c** (33.0 g, crude product), which was directly used for the next step.

### Step 3: 4,5-bis(bromomethyl)cyclohex-1-ene IN-4d

Compound **IN-4c** (33.0 g, crude product) was dissolved in *N*,*N*-dimethylformamide (200 mL), lithium bromide (28.5 g, 328.2 mmol) was added at room temperature, the temperature was lifted to 100°C, it was stirred for 2 hours, and the reaction was complete shown by TLC. The reaction solution was thermally filtered, the filter cake was washed with ethyl acetate, the filtrate was added with water, extracted with ethyl acetate, washed with saturated salt water, dried over anhydrous sodium sulfate and concentrated, and the crude product was purified by silica gel column chromatography to obtain the light yellow liquid title compound **IN-4d** (16.0 g, three-step yield 51%).

¹H NMR (400 MHz, CDCl₃) δ 5.63 (s, 2H), 3.42-3.39 (m, 2H), 3.34-3.30 (m, 2H), 2.39-2.35 (m, 2H), 2.27-2.21 (m, 2H), 2.09-2.03 (m, 2H).

### Step 4: 1,3,3a,4,7,7a-hexahydro-2H-indene-2,2-dicarboxylic acid dimethyl ester IN-4e

Compound **IN-4d** (15.0 g, 56.0 mmol) was dissolved in *N*,*N*-dimethylformamide (200 mL), dimethyl malonate (15 g, 113.5 mmol) and potassium carbonate (31.2 g, 225.7 mmol) were added at room temperature, the temperature was lifted to 110°C, it was stirred overnight, and the reaction was complete shown by TLC. The reaction solution was cooled to room temperature, added with water and extracted with ethyl acetate, and the organic phases were combined, washed with saturated salt water, dried over anhydrous sodium sulfate and concentrated to obtain the light yellow liquid title compound **IN-4e** (7.5 g, yield 56%).

¹H NMR (400 MHz, CDCl₃) δ 5.63 (s, 2H), 3.72 (d, *J* =3.2 Hz, 6H), 2.36-2.31 (m, 2H), 2.18-2.07 (m, 6H), 1.88-1.80 (m, 2H).

### Step 5: 2,3,3a,4,7,7a-hexahydro-1H-indene-2-carboxylic acid methyl ester IN-4f

Compound **IN-4e** (7.5 g, 31.5 mmol) was dissolved in dimethyl sulfoxide (20 mL) and water (2 mL), sodium chloride (3.75 g, 64.2 mmol) was added at room temperature, the temperature was lifted to 170 °C, it was stirred overnight and the reaction was complete shown by TLC. The reaction solution was lowered to room temperature, added with water and extracted with ethyl acetate, and the organic phases were combined, washed with saturated salt water, dried over anhydrous sodium sulfate and concentrated to obtain the light yellow liquid title compound **IN-4f** (4.0 g, crude product), which was directly used for the next step of reaction.

¹H NMR (400 MHz, CDCl₃) δ 5.66-5.60 (m, 2H), 3.67 (d, *J* = 2.0 Hz, 6H), 3.02-2.83 (m, 2H), 2.18-2.13 (m, 4H), 2.01-1.68 (m, 2H).

### Step 6: 4-(methoxycarbonyl)cyclopentane-1,2-dicarboxylic acid IN-4g

Compound **IN-4f** (5.3 g, crude product) was dissolved in acetonitrile/ethyl acetate (50 mL/50 mL), a sodium periodate (27.3 g, 127.6 mol) aqueous solution (100 mL) and ruthenium trichloride (122 mg, 0.59 mmol) were added, and the reaction was carried out overnight at room temperature. The disappearance of starting material was shown by TLC. The reaction solution was filtered, the pH of the filtrate was adjusted to 1-2 with hydrochloric acid (1N), it was extracted with ethyl acetate, the organic phases were combine, washed with saturated salt water, dried over anhydrous sodium sulfate and concentrated to obtain a light yellow solid crude product, and the crude product was triturated with petroleum ether/dichloromethane to obtain the white solid title compound **IN-4g** (6.2 g, crude product), which is directly used for the next step of reaction.

### Step 7: 5-oxo-octahydro-pentadiene-2-carboxylic acid methyl ester IN-4

Compound **IN-4g** (2.7 g, crude product) was dissolved in acetic anhydride (16mL), the temperature was lifted to 130 °C for reaction for 2h, anhydrous sodium acetate (816 mg, 9.95 mmol) was added, and the reaction was carried out overnight at 120 °C. The reaction solution was cooled to 0 °C, quenched with methanol, then adjusted to pH 8-9 with a saturated sodium carbonate aqueous solution and extracted with ethyl acetate, the organic phases were combined, washed with saturated salt water, dried over anhydrous sodium sulfate and concentrated, and the crude product was purified by silica gel column chromatography to obtain the colorless liquid title compound **IN-4** (1.3 g, three-step yield 23%).

### Intermediate 5

### 5-bromo-7-isocyano-2,2-dimethyl-2,3-dihydro-1H-pyrrolizine IN-5

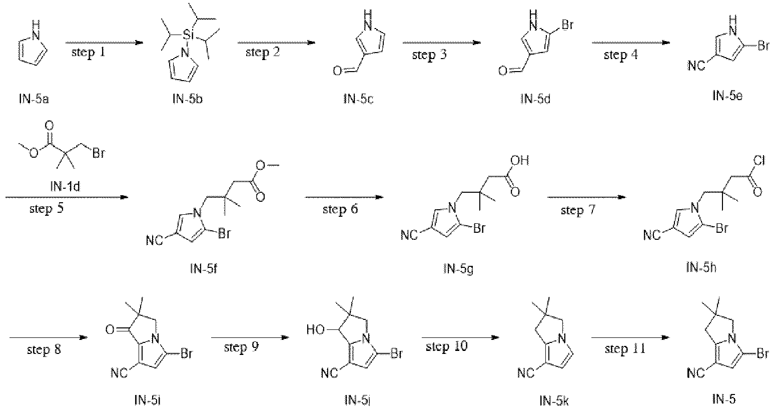

### Step 1: 1-(triisopropylsilyl)pyrrole IN-5b

Sodium hydride (13.7 g, 342 mmol, 60%) was dispersed into tetrahydrofuran (400 mL), it was cooled to 0°C, under nitrogen protection, pyrrole **IN-5a** (17.4 g, 259 mmol) was added dropwise, after the addition the temperature was lifted to room temperature, it was stirred for 1 hour, it was cooled to 0°C again, triisopropylchlorosilane (50.0 g, 259 mmol) was added dropwise, after the dropwise addition the temperature was lifted to room temperature, it was stirred for 1 hour, and the complete reaction of starting material was shown by TLC. The reaction solution was slowly poured into ice water and extracted with ethyl acetate, and the organic phases were combined, washed with water, washed with saturated salt water, dried over anhydrous sodium sulfate and concentrated to obtain the title compound **IN-5b** (63.0 g, crude product), which is directly used for the next step of reaction.

### Step 2: pyrrole-3-carboxaldehyde IN-5c

*N*,*N*-dimethylformamide (18.6 g, 256 mmol) was dissolved in dichloromethane (500 mL), oxalyl chloride (32.53 g, 256 mmol) was added dropwise at room temperature, after the addition it was stirred at room temperature for 2 hours, and a white solid was produced. Compound **IN-5b** (52.0 g, 233 mmol) was dissolved in dichloromethane (10 mL) and added dropwise to the above reaction, after the addition it was heated and refluxed for 2 hours, it was cooled to room temperature and stirred overnight, and the reaction was complete shown by TLC. The reaction solution was concentrated to remove dichloromethane, added with water and extracted with ethyl acetate, the organic phases were combined, washed with saturated salt water, dried over anhydrous sodium sulfate and concentrated, and the crude product was purified by silica gel column chromatography to obtain the brown liquid title compound **IN-5c** (7.0 g, two-step yield 34%).

### Step 3: 5-bromopyrrole-3-carboxaldehyde IN-5d

Compound **IN-5c** (7.0 g, 73.6 mmol) was dissolved in tetrahydrofuran (150 mL), under nitrogen protection, it was cooled to -65°C, a N,N-dimethylformamide (30 mL) solution of N-bromosuccinimide (13.7 g, 77.0 mmol) was added dropwise, the temperature was kept at -65°C, and the reaction was tracked by TLC until it was complete. The reaction solution was diluted with ethyl acetate, washed with water, washed with saturated salt water, dried over anhydrous sodium sulfate and concentrated, and the crude product was purified by silica gel column chromatography to obtain the title compound **IN-5d** (3.6 g, yield 28%).

### Step 4: 2-bromo-4-isocyano-1H-pyrrole IN-5e

Hydroxylammoniumsulfonate (9.3 g, 82.2 mmol) was dispersed in water (40 mL), it was cooled to 0°C, Compound **IN-5d** (3.6 g, 20.7 mmol) was added, the temperature was naturally lifted to room temperature, it was stirred overnight, and the complete reaction of the starting materials was shown by TLC. The reaction solution was filtered, the filter cake was washed with water, the pH of the filtrate was adjusted to be neutral with a saturated sodium bicarbonate aqueous solution, it was extracted with dichloromethane, the organic phases were combined, washed with saturated salt water, dried over anhydrous sodium sulfate and concentrated, and the crude product was purified by silica gel column chromatography to obtain the light yellow solid title compound **IN-5e** (3.0 g, yield 85%).

### Step 5: 4-(2-bromo-4-isocyano-1H-pyrrol-1-yl)-3,3-dimethylbutyric methyl ester IN-5f

Compound **IN-5e** (4.0 g, 23.4 mmol) was dispersed in *N*,*N*-dimethylformamide (40 mL), Compound **IN-1d** (7.4 g, 35.3 mmol) and potassium carbonate (6.5 g, 47.0 mmol) were added at room temperature, under nitrogen protection, it was heated to 80°C and stirred overnight, and the complete reaction of the starting materials was shown by TLC. The reaction solution was diluted with water and extracted with ethyl acetate, the organic phases were combined, washed with water, washed with saturated salt water, dried over anhydrous sodium sulfate and concentrated, and the crude product was purified by silica gel column chromatography to obtain the yellow oil title compound **IN-5f** (4.2 g, yield 57%).

### Step 6: 4-(2-bromo-4-isocyano-1H-pyrrol-1-yl) -3,3-dimethylbutyric acid IN-5g

Compound **IN-5f** (4.1 g, 13.1 mmol) was dispersed in tetrahydrofuran (20 mL) and methanol (20 mL), sodium hydroxide aqueous solution (7 mL, 28 mmol, 4 M) was added, it was stirred at room temperature for 1 hour, and the complete reaction of the starting materials was shown by TLC. The reaction solution was concentrated to remove the solvent, added with water (50 mL) and extracted with ethyl acetate, the organic phase was discarded, the aqueous phase was adjusted to pH about 1 with dilute hydrochloric acid (1N) and extracted with ethyl acetate, and the organic phases were combined, washed with saturated salt water, dried over anhydrous sodium sulfate and concentrated to obtain the yellow solid title compound **IN-5g** (3.5 g, crude product), which was directly used for the next step of reaction.

¹H NMR (400 MHz, DMSO-*d*₆) δ 12.49 (s, 1H), 7.64 (d, *J* =2.0 Hz, 1H), 6.70 (d, *J* = 2.0 Hz, 1H), 4.15 (s, 2H), 3.38 (s, 2H), 1.11 (s, 6H).

### Step 7: 4-(2-bromo-4-isocyano-1H-pyrrol-1-yl)-3,3-dimethylbutyryl chloride IN-5h

Compound **IN-5g** (3.5 g, crude product) was dispersed in dichloromethane (80 mL) and N,N-dimethylformamide (0.5 mL), it was cooled to 0°C, oxalyl chloride (2.5 g, 19.7 mmol) was added dropwise under nitrogen protection, after the dropwise addition it was stirred at 0°C for 1 hour, and the complete reaction of the starting materials was shown by TLC. The reaction solution was concentrated to obtain the title compound **IN-5h** (4.0 g, crude product), which was directly used for the next step.

### Step 8: 5-bromo-7-isocyano-2,2-dimethyl-2,3-dihydro-1H-pyrrolizin-1-one IN-5i

Aluminum chloride (3.7 g, 27.7 mmol) was dispersed in dichloromethane (80 mL), it was cooled to 0°C. under nitrogen protection, a dichloromethane (80 mL) solution of Compound **IN-5h** (4.0 g, 13.2 mmol) was added dropwise, after the dropwise addition the temperature was naturally lifted to room temperature, it was stirred overnight, and the complete reaction of the starting materials was shown by TLC. The reaction solution was quenched with water and extracted with dichloromethane, the organic phases were combined, washed with saturated salt water, dried over anhydrous sodium sulfate and concentrated, and the crude product was purified by silica gel column chromatography to obtain the yellow solid title compound **IN-5i** (2.5 g, three-step yield 76%).

¹H NMR (400 MHz, CDCl₃) δ 6.81 (s, 1H), 4.02 (s, 2H), 1.38 (s, 6H).

### Step 9: 5-Bromo-7-isocyano-2,2-dimethyl-2,3-dihydro-1H-pyrrolizin-1-ol IN-5j

Compound **IN-5i** (2.5 g, 9.9 mmol) was dispersed in methanol (50 mL), it was cooled to 0°C, sodium borohydride (940 mg, 24.8 mmol) was added in batches, after the addition the temperature was lifted to room temperature, it was stirred for 1 hour, and the reaction was complete shown by LCMS. The reaction solution was quenched with water, concentrated to remove methanol, diluted with ethyl acetate, washed with water, washed with saturated salt water, dried over anhydrous sodium sulfate and concentrated to obtain the title compound **IN-5j** (2.0 g, crude product), which was directly used for the next step.

LC-MS: m/z=237.1[M-H₂O]⁺

### Step 10: 7-isocyano-2,2-dimethyl-2,3-dihydro-1H-pyrrolizine IN-5k

Compound **IN-5j** (2.0 g, crude product) was dispersed in trifluoroacetic acid (30 mL), it was cooled to 0°C, under nitrogen protection, triethylsilane (2.7 g, 23.2 mmol) was added dropwise, after addition it was stirred at 0°C for 1 hour, and the complete reaction of the starting materials was shown by TLC. The reaction solution was concentrated to remove the solvent, diluted with water and extracted with ethyl acetate, the organic phases were combined, washed with saturated salt water, dried over anhydrous sodium sulfate and concentrated, and the crude product was purified by silica gel column chromatography to obtain the yellow solid title compound **IN-5k** (1.2 g, two-step yield 76%).

### Step11: 5-bromo-7-isocyano-2,2-dimethyl-2,3-dihydro-1H-pyrrolizine IN-5

Compound **IN-5k** (1.2 g, 7.5 mmol) was dispersed in dichloromethane (20 mL), it was cooled to 0°C, N-bromosuccinimide (1.2 g, 6.7 mmol) was added in batches, after the addition it was stirred at 0°C for 20 minutes, and the complete reaction of the starting materials was shown by TLC. The reaction solution was diluted with water and extracted with dichloromethane, the organic phases were combined, washed with saturated salt water, dried over anhydrous sodium sulfate and concentrated, and the crude product was purified by silica gel column chromatography to obtain the yellow liquid title compound **IN-5** (1.6 g, yield 89%).

¹H NMR (400 MHz, CDCl₃) δ 6.34 (s, 1H), 3.68 (s, 2H), 2.84 (s, 2H), 1.28 (s, 6H).

### Intermediate 6

### 7-carbamoyl-2-azaspiro[4.4]nonane-2-carboxylic acid tert-butyl ester IN-6-1,2

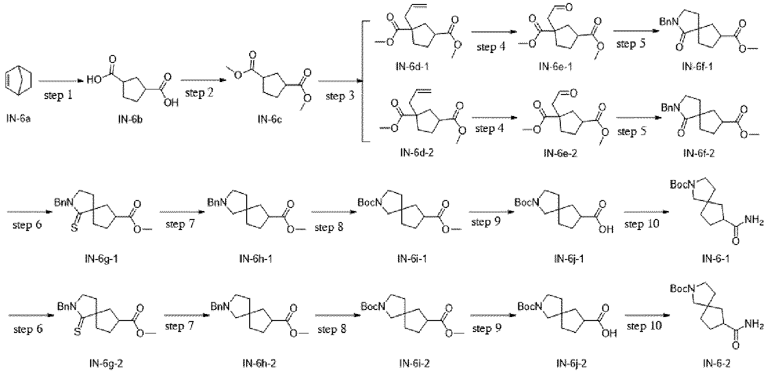

### Step 1: cyclopentane-1,3-dicarboxylic acid IN-6b

Norbornene **IN-6a** (20.0 g, 0.21 mol) was dissolved in acetonitrile/ethyl acetate (400 mL/400 mL), an aqueous solution (600 mL) of sodium periodate (181.8 g, 0.85 mol) and ruthenium trichloride (969 mg, 4.67 mmol) were added in sequence, and the reaction was carried out overnight at room temperature. The disappearance of the starting material was shown by TLC. The reaction solution was filtered, the pH of the filtrate was adjusted to 11-12 with a sodium hydroxide (5N) solution, it was extracted with ethyl acetate, the organic phase was discarded, the pH of the aqueous phase was adjusted to 1-2 with dilute hydrochloric acid (1N), it was extracted with ethyl acetate, and the organic phases were combined, washed with saturated salt water, dried over anhydrous sodium sulfate and concentrated to obtain the light yellow solid title compound **IN-6b** (18.5 g, crude product), which was directly used for the next step.

### Step 2: cyclopentane-1,3-dicarboxylate IN-6c

Compound **IN-6b** (18.5 g, crude product) was dissolved in methanol (200 mL), concentrated sulfuric acid (5 mL) was added at room temperature, and the temperature was lifted to 70°C for reaction overnight. The reaction solution was concentrated, diluted with water, extracted with ethyl acetate, washed with saturated salt water, dried over anhydrous sodium sulfate and concentrated, and the crude product was purified by silica gel column chromatography to obtain the light yellow liquid title compound **IN-6c** (18.6 g, two-step yield 47%).

¹H NMR (400 MHz, CDCl₃) δ 3.36 (s, 6H), 2.87-2.73 (m, 2H), 2.27-2.23 (m, 1H), 2.14-2.08 (m, 1H), 2.01-1.89 (m, 4H).

### Step 3: 1-allylcyclopentane-1,3-dicarboxylic acid dimethyl ester IN-6d-1,2

Compound **IN-6c** (40.0 g, 215 mmol) was dispersed into anhydrous tetrahydrofuran (500 mL), it was cooled to -60°C, under nitrogen protection, lithium diisopropylamide (120 mL, 240 mmol, 2M tetrahydrofuran solution) was added dropwise, after the addition it was stirred for 30 minutes at -60°C, a tetrahydrofuran (20 mL) solution of bromopropene (5.8 g, 47.9 mmol) was added dropwise, after the addition it was stirred for 30 minutes at -60°C, and the reaction was complete shown by TLC. The reaction solution was quenched with a saturated ammonium chloride solution and extracted with ethyl acetate, and the organic phases were combined, washed with saturated salt water, dried over anhydrous sodium sulfate and concentrated, and the crude product was purified by silica gel column chromatography to obtain the low-polarity colorless liquid title compound **IN-6d-1** (12.0 g, yield 25%) and the high-polarity title compound **IN-6d-2** (15.0 g, yield 31%).

### Compound IN-6d-1

¹H NMR (400 MHz, CDCl₃) δ 5.74-5.64 (m, 1H), 5.07-5.02 (m, 2H), 3.68 (s, 6H), 2.94-2.86 (m, 1H), 2.50-2.36 (m, 3H), 2.17-2.10 (m, 1H), 2.01-1.85 (m, 2H), 1.80-1.67 (m, 2H).

### Compound IN-6d-2

¹H NMR (400 MHz, CDCl₃) δ 5.65-5.58 (m, 1H), 4.98 (d, *J* = 12.4 Hz, 2H), 3.60 (s, 6H), 2.82-2.74 (m, 1H), 2.33-2.27 (m, 3H), 2.20-2.13 (m, 1H), 1.92-1.85 (m, 3H), 1.57-1.49 (m, 1H). Step 4: 1-(2-oxoethyl)cyclopentane-1,3-dicarboxylic acid dimethyl ester **IN-6e-1,2**

Compound **IN-6d-1** (3.3 g, 14.6 mmol) was dissolved in acetonitrile (30 mL) and water (30 mL), at 0°C, ruthenium trichloride (138 mg, 0.66 mmol) and sodium periodate (5.66 g, 26.5 mmol) were added, it was stirred for 30 minutes, and the reaction was complete shown by TLC. The reaction solution was filtered, the filtrate was concentrated to remove the organic phase, the aqueous phase was extracted with ethyl acetate, the organic phase were combined, washed with saturated salt water, dried over anhydrous sodium sulfate and concentrated, and the crude product was purified by silica gel column chromatography to obtain the colorless liquid title compound **IN-6e-1** (1.2 g, yield 36%).

Compound **IN-6d-2** (15.0 g, 66.3 mmol) was dissolved in acetonitrile (200 mL) and water (200 mL), at 0°C, ruthenium trichloride (690 mg, 3.3 mmol) and sodium periodate (28.3 g, 132.3 mmol) were added, it was stirred for 30 minutes, and the reaction was complete shown by TLC. The reaction solution was filtered, the filtrate was concentrated to remove the organic phase, the aqueous phase was extracted with ethyl acetate, the organic phases were combined, washed with saturated salt water, dried over anhydrous sodium sulfate and concentrated, and the crude product was purified by silica gel column chromatography to obtain the colorless liquid title compound **IN-6e-2** (7.5 g, yield 49%).

¹H NMR (400 MHz, CDCl₃) δ 9.71 (s, 1H), 3.69 (d, *J* = 2.8 Hz, 6H), 2.93-2.84 (m, 1H), 2.77 (s, 2H), 2.55-2.49 (m, 1H), 2.37-2.30 (m, 1H), 2.10-1.92 (m, 3H), 1.65-1.59 (m, 1H).

### Step 5: 2-benzyl-1-oxo-2-azaspiro[4.4]nonane-7-carboxylic acid methyl ester IN-6f-1,2

Compound **IN-6e-1** (1.2 g, 5.26 mmol) was dissolved in methanol (15 mL), benzylamine (0.85 g, 7.88 mmol) was added, it was stirred at room temperature for 30 minutes, sodium cyanoborohydride (1.0 g, 15.9 mmol) was added, it was stirred overnight at room temperature, and the reaction was complete shown by TLC. The reaction solution was quenched with water, concentrated to remove methanol, diluted with ethyl acetate, washed with saturated salt water, dried over anhydrous sodium sulfate and concentrated, and the crude product was purified by silica gel column chromatography to obtain the colorless liquid title compound **IN-6f-1** (1.1 g, yield 73%).

Compound **IN-6e-2** (7.5 g, 32.9 mmol) was dissolved in methanol (80 mL), benzylamine (5.3 g, 49.5 mmol) was added, it was stirred at room temperature for 30 minutes, sodium cyanoborohydride (6.2 g, 98.7 mmol) was added, it was stirred overnight at room temperature, and the reaction was complete shown by TLC. The reaction solution was quenched with water, concentrated to remove methanol and diluted with ethyl acetate, the organic phases were combined, washed with saturated salt water, dried over anhydrous sodium sulfate and concentrated, and the crude product was purified by silica gel column chromatography to obtain the colorless liquid title compound **IN-6f-2** (7.0 g, yield 74%).

LC-MS: m/z=288.3[M+H]⁺

### Step 6: 2-benzyl-1-thio-2-azaspiro[4.4]nonane-7-carboxylic acid methyl ester IN-6g-1,2

Compound **IN-6F-1** (1.2 g, 4.18 mmol) was dissolved in toluene (6 mL), Lawesson reagent (2.53 g, 6.26 mmol) was added at room temperature, the temperature was lifted to 100°C for reaction for 2 hours, and the disappearance of the starting material was shown by TLC. The reaction solution was cooled to room temperature, quenched with water, extracted with ethyl acetate, washed with saturated salt water, concentrated and chromatographed on silica gel column to obtain the colorless oil title compound **IN-6g-1** (932 mg, yield 73%).

Compound **IN-6f-2** (2.0 g, 6.96 mmol) was dissolved in toluene (6 mL), Lawesson reagent (4.22 g, 10.43 mmol) was added at room temperature, the temperature was lifted to 100°C for reaction for 2 hours, and the disappearance of the starting material was shown by TLC. The reaction solution was cooled to room temperature, quenched with water, extracted with ethyl acetate, washed with saturated salt water, concentrated and chromatographed on silica gel column to obtain the colorless oil title compound **IN-6g-2** (1.6 g, yield 76%).

LC-MS: m/z=304.2[M+H]⁺

### Step 7: 2-benzyl-2-azaspiro[4.4]nonane-7-carboxylic acid methyl ester IN-6h-1,2

Compound **IN-6g-1** (932 mg, 3.07 mmol) was dissolved in tetrahydrofuran (6 mL), Raney nickel was added at room temperature, the temperature was lifted to 65°C for reaction for 2 hours, and the reaction was complete shown by TLC. The reaction solution was filtered with diatomite, and the filtrate is concentrated to obtain the colorless oil title compound **IN-6h-1** (755 mg, crude product), which was directly used for the next step.

Compound **IN-6g-2** (1.6 g, 5.27 mmol) was dissolved in tetrahydrofuran (6 mL), Raney nickel was added at room temperature, the temperature was lifted to 65°C for reaction for 2 hours, and the reaction was complete shown by TLC. The reaction solution was filtered with diatomite, and the filtrate was concentrated to obtain the colorless oil title compound **IN-6h-2** (1.2 g, crude product), which was directly used for the next step.

LC-MS: m/z=274.2[M+H]⁺

### Step 8: 7-methyl-2-azaspiro[4.4]nonane-2,7-dicarboxylic acid di-tert-butyl ester IN-6i-1,2

Compound **IN-6h-1** (755 mg, crude product) was dissolved in methanol (10 mL), di-tert-butyl dicarbonate (0.71 g, 3.25 mmol) and palladium/carbon (100 mg, 10%) were added, the reaction was carried out overnight at room temperature in a hydrogen atmosphere, and the reaction was complete shown by TLC. The reaction solution was filtered with diatomite, the filtrate was concentrated, and the crude product was purified by silica gel column chromatography to obtain the colorless liquid title compound **IN-6i-1** (747 mg, two-step yield 86%).

Compound **IN-6h-2** (1.2 g, crude product) was dissolved in methanol (10 mL), di-tert-butyl dicarbonate (1.15 g, 5.27 mmol) and palladium/carbon (200 mg, 10%) were added, the reaction was carried out overnight at room temperature in a hydrogen atmosphere, and the reaction was complete shown by TLC. The reaction solution was filtered with diatomite, the filtrate was concentrated, and the crude product was purified by silica gel column chromatography to obtain the colorless liquid title compound **IN-6i-2** (1.1 g, two-step yield 74%).

¹H NMR (400 MHz, CDCl₃) δ 3.68 (s, 3H), 3.44-3.34 (m, 2H), 3.27-3.21 (m, 2H), 2.93-2.86 (m, 1H), 2.06-1.87 (m, 4H), 1.77-1.71 (m, 3H), 1.56-1.53 (m, 1H), 1.46 (s, 9H).

### Step 9: 2-(tert-butoxycarbonyl)-2-azaspiro[4.4]nonane-7-carboxylic acid IN-6j-1,2

The compound **IN-6i-1** (747 mg, 2.64 mmol) was dissolved in methanol/tetrahydrofuran/water (8 mL/3 mL/3 mL), sodium hydroxide (158 mg, 3.95 mmol) was added, the reaction was carried out for 3 hours at room temperature, and the reaction was complete shown by TLC. The reaction solution was added with water and extracted with ethyl acetate, the organic phase was discarded, the pH of the aqueous phase was adjusted to about 4 with dilute hydrochloric acid (1N), it was extracted with ethyl acetate, and the organic phases were combined, washed with saturated salt water, dried over anhydrous sodium sulfate and concentrated to obtain the colorless oily liquid title compound **IN-6j-1** (725 mg, crude product), which was directly used for the next step. Compound **IN-6i-2** (1.1 g, 3.88 mmol) was dissolved in methanol/tetrahydrofuran/water (12 mL/6 mL/6 mL), sodium hydroxide (233 mg, 5.83 mmol) was added, the reaction was carried out for 3 hours at room temperature, and the reaction was complete shown by TLC. The reaction solution was added with water and extracted with ethyl acetate, the organic phase was discarded, the pH of the aqueous phase was adjusted to about 4 with dilute hydrochloric acid (1N),it was extracted with ethyl acetate, and the organic phases were combined, washed with saturated salt water, dried over anhydrous sodium sulfate and concentrated to obtain the colorless oily liquid title compound **IN-6j-2** (830 mg, crude product), which was directly used for the next step.

### Step 10: 7-carbamoyl-2-azaspiro[4.4]nonane-2-carboxylic acid tert-butyl ester IN-6-1,2

Compound **IN-6j-1** (300 mg, crude product) was dissolved in N,N-dimethylformamide (6 mL), HATU (838 mg, 2.20 mmol), *N*,*N*-diisopropylethylamine (475 mg, 3.68 mmol) and ammonium chloride (149 mg, 2.78 mmol) were added in sequence, and the reaction was carried out overnight at room temperature. The reaction was complete shown by TLC. The reaction solution was quenched with water and extracted with ethyl acetate, the organic phases were combined, washed with saturated salt water, dried over anhydrous sodium sulfate and concentrated, and the crude product was purified by silica gel column chromatography to obtain the white solid title compound **IN-6-1** (260 mg, two-step yield 86%).

Compound **IN-6j-2** (830 mg, crude product) was dissolved in *N*,*N*-dimethylformamide (6 mL), HATU (1.4 g, 3.68 mmol), *N*,*N*-diisopropylethylamine (793 mg, 6.14 mmol) and ammonium chloride (249 mg, 4.66 mmol) were added in sequence, and the reaction was carried out at room temperature overnight. The reaction was complete shown by TLC. The reaction solution was quenched with water and extracted with ethyl acetate, the organic phases were combined, washed with saturated salt water, dried over anhydrous sodium sulfate and concentrated, and the crude product was purified by silica gel column chromatography to obtain the white solid title compound **IN-6-2** (760 mg, two-step yield 73%).

¹H NMR (400 MHz, CDCl₃) δ 5.40 (s, 2H), 3.44-3.23 (m, 4H), 2.81-2.72 (m, 1H), 2.06-1.87 (m, 4H), 1.81-1.69 (m, 3H), 1.61-1.53 (m, 1H), 1.46 (s, 9H).

### Embodiment 1

### (1S,3R)-3-acetamido-N-(5-chloro-4-(5-cyano-2,2-dimethyl-2,3-dihydro-1H-pyrrolizin-7-yl)pyridin -2-yl)cyclohexane-1-carboxamide 1

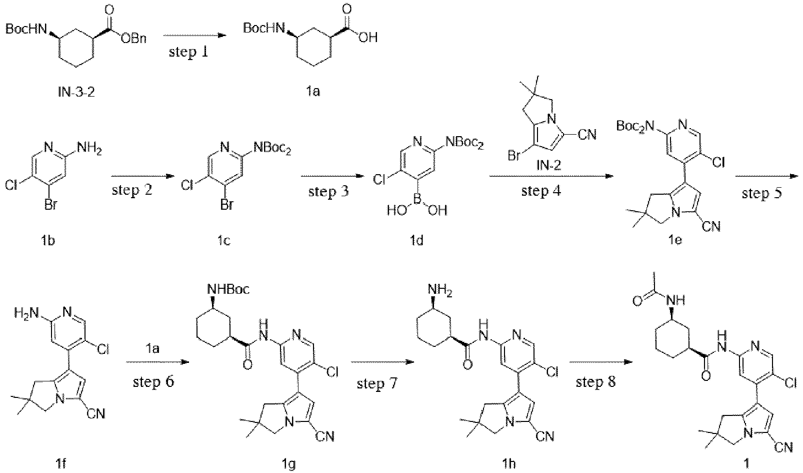

### Step 1: (1S,3R)-3-tert-butoxycarbonylamino cyclohexane carboxylic acid 1a

Intermediate **IN-3-2** (1.5 g, 4.5 mmol) was dissolved in methanol (25mL), palladium/carbon (150 mg, 10%) was added, it was stirred overnight at room temperature in hydrogen atmosphere, and the disappearance of the starting material was shown by TLC. The reaction solution was filtered with diatomite, and the filtrate was concentrated to obtain the white solid title compound **1a** (1.1 g, crude product), which was directly used for the next step.

### Step 2: (4-bromo-5-chloropyridin-2-yl)iminodicarboxylic acid di-tert-butyl ester 1c

4-bromo-5-chloropyridin-2-amine **1b** (3.0 g, 14.5 mmol) was dispersed in a mixed solvent (100 mL) of tert-butanol/acetone (1:1), triethylamine (6.2 g, 61.3 mmol), di-tert-butyl dicarbonate (12.7 g, 58.2 mmol) and 4-dimethylaminopyridine (catalytic amount) were added, it was stirred at room temperature for 3 hours, and the complete reaction of the starting material was shown by TLC. The reaction solution was concentrated to remove the organic solvent, diluted with water and extracted with ethyl acetate, the organic phases were combined, washed with saturated salt water, dried over anhydrous sodium sulfate and concentrated, and the crude product was purified by silica gel column chromatography to obtain the white solid title compound **1c** (7.5 g, crude product), which was directly used for the next step.

¹H NMR (400 MHz, DMSO-*d*₆) δ 8.65 (s, 1H), 8.03 (s, 1H), 1.41 (s, 18H).

### Step 3: (2-((di-tert-butoxycarbonyl)amino)-5-chloropyridin-4-yl)boronic acid 1d

Compound **1c** (1.0 g, crude product) was dispersed in 1,4-dioxane (20 mL), bis(pinacolato)diboron (950 mg, 3.7 mmol), potassium acetate (730 mg, 7.4 mmol) and Pd(dppf)Cl₂ dichloromethane complex (catalytic amount) were added in sequence, under nitrogen protection, the temperature was lifted to 90°C, it was stirred for 1 hour, and the disappearance of the starting material was shown by TLC. The reaction solution was cooled to room temperature and filtered, the filter cake was washed with ethyl acetate,the filtrate was diluted with water and extracted with ethyl acetate, the organic phases were combined, washed with water, washed with saturated salt water, dried over anhydrous sodium sulfate and concentrated to obtain the brown oil title compound **1d** (2.5 g, crude product), which was directly used for the next step.

LC-MS: m/z=373.1[M+H]⁺

### Step 4: (5-chloro-4-(5-cyano-2,2-dimethyl-2,3-dihydro-1H-pyrrolizin-7-yl)pyridin-2-yl)iminodicarboxylic acid di-tert-butyl ester 1e

Compound **1d** (250 mg, crude product) and Intermediate **IN-2** (1.2 g, 1.6 mmol) were dispersed in 1,4-dioxane (5 mL) and water (2 mL), sodium carbonate (220 mg, 2.1 mmol) and Pd(dppf)Cl₂ (catalytic amount) were added in sequence, under nitrogen protection, the temperature was lifted to 100°C, it was stirred for 1 hour, and the complete reaction of the starting material was shown by TLC. The reaction solution was cooled to room temperature and filtered, the filter cake was washed with ethyl acetate, the filtrate was diluted with water and extracted with ethyl acetate, the organic phases were combined, washed with saturated salt water, dried over anhydrous sodium sulfate and concentrated, and the crude product was purified by silica gel column chromatography to obtain the white solid title compound **1e** (300 mg, three-step yield 26%).

LC-MS: m/z=387.2[M+H-Boc]⁺

### Step 5: 7-(2-amino-5-chloropyridin-4-yl)-2,2-dimethyl-2,3-dihydro-1H-pyrrolizine-5-carbonitrile 1f

Compound **1e** (300 mg, 0.62 mmol) was dispersed in dichloromethane (6 mL), trifluoroacetic acid (3 mL) was added, it was stirred at room temperature for 1 hour, and the complete reaction of the starting material was shown by TLC. The reaction solution was concentrated, quenched with a saturated sodium bicarbonate aqueous solution and extracted with ethyl acetate, and the organic phases were combined, washed with saturated salt water, dried over anhydrous sodium sulfate, and concentrated to obtain the yellow solid title compound **If** (250 mg, crude product), which was directly used for the next step.

LC-MS: m/z=287.1[M+H]⁺

### Step 6: (1R,3S)-3-((5-chloro-4-(5-cyano-2,2-dimethyl-2,3-dihydro-1H-pyrrolizin-7-yl)pyridin-2-yl)carba moyl)cyclohexyl carbamic acid tert-butyl ester 1g

Compound **If** (250 mg, crude product) and compound **1a** (317 mg, 1.3 mmol) were dispersed in ethyl acetate (10 mL), pyridine (413 mg, 5.2 mmol) and 1-propylphosphonic anhydride (2.2 g, 6.9 mmol, 50% ethyl acetate solution) were added in sequence at room temperature, the temperature was lifted to 85°C, it was stirred overnight, and the complete reaction of the starting material was shown by TLC. The reaction solution was added with a saturated sodium carbonate aqueous solution to adjust pH to be alkaline and extracted with ethyl acetate, the organic phases were combined, washed with saturated salt water, dried over anhydrous sodium sulfate and concentrated, and the crude product was was purified by Prep-TLC to obtain the white solid title compound **1g** (210 mg, two-step yield 67%).

LC-MS: m/z=512.3[M+H]⁺

### Step 7: (1S,3R)-3-amino-N-(5-chloro-4-(5-cyano-2,2-dimethyl-2,3-dihydro-1H-pyrrolizin-7-yl)pyridin-2-yl)cyclohexane-1-carboxamide 1h

Compound **1g** (210 mg, 0.41 mmol) was dispersed in dichloromethane (4 mL), trifluoroacetic acid (2 mL) was added, it was stirred at room temperature for 1 hour, and the complete reaction of the starting material was shown by TLC. The reaction solution was concentrated, quenched with a saturated sodium bicarbonate aqueous solution and extracted with ethyl acetate, the organic phases were combined, washed with saturated salt water, dried over anhydrous sodium sulfate and concentrated to obtain the yellow solid title compound **Ih** (220 mg, crude product),which was directly used for the next step.

### Step 8: (1S,3R)-3-acetamido-N-(5-chloro-4-(5-cyano-2,2-dimethyl-2,3-dihydro-1H-pyrrolizin-7-yl)pyridin -2-yl)cyclohexane-1-carboxamide 1

Compound **Ih** (220 mg, crude product) was dispersed in dichloromethane (4 mL), triethylamine (108 mg, 1.1 mmol) and acetic anhydride (82 mg, 0.8 mmol) were added in sequence, it was stirred at room temperature for 15 minutes, and the complete reaction of the starting material was shown by TLC. The reaction solution was added with a saturated sodium bicarbonate aqueous solution and extracted with dichloromethane, the organic phases were combined, washed with saturated salt water, dried over anhydrous sodium sulfate and concentrated, and the crude product was purified by silica gel column chromatography (dichloromethane/methanol 50:1-30:1) to obtain the white solid title compound **1** (160 mg, two-step yield 86%).

LC-MS: m/z=454.2[M+H]⁺

¹H NMR (400 MHz, DMSO-*d*₆) δ 10.58 (s, 1H), 8.35 (s, 1H), 8.19 (s, 1H), 7.75 (d, *J* = 7.6 Hz, 1H), 7.39 (s, 1H), 3.94 (s, 2H), 3.61-3.50 (m, 1H), 2.85 (s, 2H), 2.65-2.56 (m, 1H), 1.90-1.86 (m, 1H), 1.81-1.70 (m, 6H), 1.30-1.27 (m, 2H), 1.23 (s, 7H), 1.12-1.02 (m, 1H). (96.49% purity by HPLC)

### Embodiment 2

### 7-(2-((1S,3R)-3-acetylaminocyclohexane-1-carboxamido)-5-chloropyridin-4-yl)-2,2-dimethyl-2,3-dihydro-1H-pyrrolizine-5-carboxamide 2

Compound **1** (50 mg, 0.11 mmol) was dispersed in dimethyl sulfoxide (1 mL), potassium carbonate (30 mg, 0.22 mmol) and hydrogen peroxide (1 mL, 30%) were added in sequence, it was stirred at room temperature for 1 hour, and the complete reaction of the starting material was shown by TLC. The reaction solution was quenched with water and extracted with ethyl acetate, the organic phases were combined, washed with water, washed with saturated salt water, dried over anhydrous sodium sulfate and concentrated, and the crude product was purified by Prep-TLC to obtain the white solid title compound **2** (35.4 mg, yield 68%).

LC-MS: m/z= 472.2[M+H]⁺

¹H NMR (400 MHz, DMSO-*d*₆) δ 10.52 (s, 1H), 8.31 (s, 1H), 8.21 (s, 1H), 7.77 (d, *J* = 8.0 Hz, 1H), 7.59 (br, 1H), 7.34 (s, 1H), 6.92 (br, 1H), 4.03 (s, 2H), 3.62-3.53 (m, 1H), 2.78 (s, 2H), 2.62-2.58 (m, 1H), 1.90-1.87 (m, 1H), 1.82-1.72 (m, 6H), 1.28-1.23 (m, 3H), 1.20 (s, 6H), 1.10-1.05 (m, 1H). (99.33% purity by HPLC)

### Embodiment 3

### (1S,3R)-3-acetamido-N-(5-chloro-4-(5-((S)-1-hydroxyethyl)-2,2-dimethyl-2,3-dihydro-1H-pyrroliz in-7-yl)pyridin-2-yl)cyclohexane-1-carboxamide (assumed) 3-1

### (1S,3R)-3-acetamido-N-(5-chloro-4-(5-((R)-1-hydroxyethyl)-2,2-dimethyl-2,3-dihydro-1H-pyrroli zin-7-yl)pyridin-2-yl)cyclohexane-1-carboxamide (assumed) 3-2

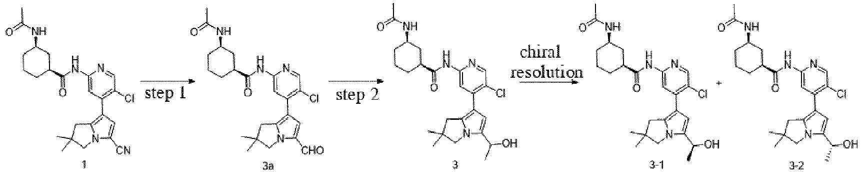

### Step 1: (1S,3R)-3-acetamido-N-(5-chloro-4-(5-formyl-2,2-dimethyl-2,3-dihydro-1H-pyrrolizin-7-yl)pyridi n-2-yl)cyclohexane-1-carboxamide 3a

Compound **1** (100 mg, 0.22 mmol) was dissolved in dichloromethane (1 mL), it was cooled to -60°C, diisobutyl aluminum hydride (0.35 mL, 0.52 mmol, 1.5 M tetrahydrofuran solution) was added dropwise, after the addition the reaction was carried out at -60°C for 4 hours, and the reaction was complete shown by TLC. The reaction solution was poured into ice water, added with diluted hydrochloric acid (1N) to adjust the pH to be acidic and extracted with ethyl acetate, the organic phases were combined, washed with saturated salt water, dried over anhydrous sodium sulfate and concentrated, and the crude product was purified by silica gel column chromatography to obtain the title compound **3a** (76 mg, yield 76%).

LC-MS: m/z=457.2 [M+H]⁺

### Step 2: (1S,3R)-3-acetamido-N-(5-chloro-4-(5-((S)-1-hydroxyethyl)-2,2-dimethyl-2,3-dihydro-1H-pyrroliz in-7-yl)pyridin-2-yl)cyclohexane-1-carboxamide (assumed) 3-1 &

### (1S,3R)-3-acetamido-N-(5-chloro-4-(5-((R)-1-hydroxyethyl)-2,2-dimethyl-2,3-dihydro-1H-pyrroli zin-7-yl)pyridin-2-yl)cyclohexane-1-carboxamide (assumed) 3-2

Compound **3a** (75 mg, 0.16 mmol) was dissolved in anhydrous tetrahydrofuran (1.5 mL), methylmagnesium bromide (0.5 mL, 1.5 mmol, 3M tetrahydrofuran solution) was added dropwise at room temperature, after the addition the reaction was carried out at room temperature for 1 hour, and the reaction was complete shown by TLC. The reaction solution was poured into a saturated ammonium chloride solution for quenching and extracted with ethyl acetate, the organic phases were combined, washed with saturated salt water, dried over anhydrous sodium sulfate and concentrated, and the crude product was separated and purified by neutral alumina column chromatography to obtain the light yellow solid title compound **3** (46 mg, yield 61%). Chiral resolution (NANOMICRO OD-5H, 30*250 mm, 5 um, 30mL/min, EtOH:Hexane=5:95) to obtain the light yellow solid compound **3-1** (peak 1, RT 10.3 min) (19.3 mg, yield 42%) and the light yellow solid compound **3-2** (peak 2, RT 20.4 min) (12.9 mg, yield 28%).

### Compound 3-1

LC-MS: m/z= 473.2[M+H]⁺

¹H NMR(400 MHz, DMSO-*d*₆) δ 10.45 (s, 1H), 8.25 (s, 1H), 8.18 (s, 1H), 7.80 (d, *J* = 7.6 Hz, 1H), 6.42 (s, 1H), 5.06 (d, *J* = 5.2 Hz, 1H), 4.72-4.65 (m, 1H), 3.79 (q, *J* = 6.8 Hz, 2H), 3.61-3.52 (m, 2H), 2.81-2.73 (m, 2H), 2.63-2.56 (m, 1H), 1.92-1.84 (m, 1H), 1.78 (s, 5H), 1.40 (d, *J* = 6.4 Hz, 3H), 1.34-1.29 (m, 4H), 1.23 (d, *J* = 3.6 Hz, 6H). (97.79% purity by HPLC)

### Compound 3-2

LC-MS: m/z= 473.2[M+H]⁺

¹H NMR(400 MHz, DMSO-*d*₆) δ 10.45 (s, 1H), 8.25 (s, 1H), 8.18(s, 1H), 7.79 (d, *J* = 8.0 Hz, 1H), 6.42 (s, 1H), 5.04 (d, *J* = 5.2 Hz, 1H), 4.72-4.66 (m, 1H), 3.80 (q, *J* = 6.8 Hz, 2H), 3.60-3.50 (m, 2H), 2.82-2.73 (m, 2H), 2.63-2.56 (m, 1H), 1.92-1.84 (m, 1H), 1.78 (s, 5H), 1.40 (d, *J* = 6.4 Hz, 3H), 1.34-1.27 (m, 4H), 1.24 (d, *J* = 3.2 Hz, 6H). (98.33% purity by HPLC)

### Embodiment 4

### (3aR,5s,6aS)-2-acetyl-N-(5-chloro-4-(5,5-dimethyl-5,6-dihydro-4H-pyrrolo[1,2-b]pyrazol-3-yl)pyr idin-2-yl)octahydrocyclopenta[c]pyrrole-5-carboxamide (assumed) 4-1

### (3aR,5r,6aS)-2-acetyl-N-(5-chloro-4-(5,5-dimethyl-5,6-dihydro-4H-pyrrolo[1,2-b]pyrazol-3-yl)pyr idin-2-yl)octahydrocyclopenta[c]pyrrole-5-carboxamide (assumed) 4-2

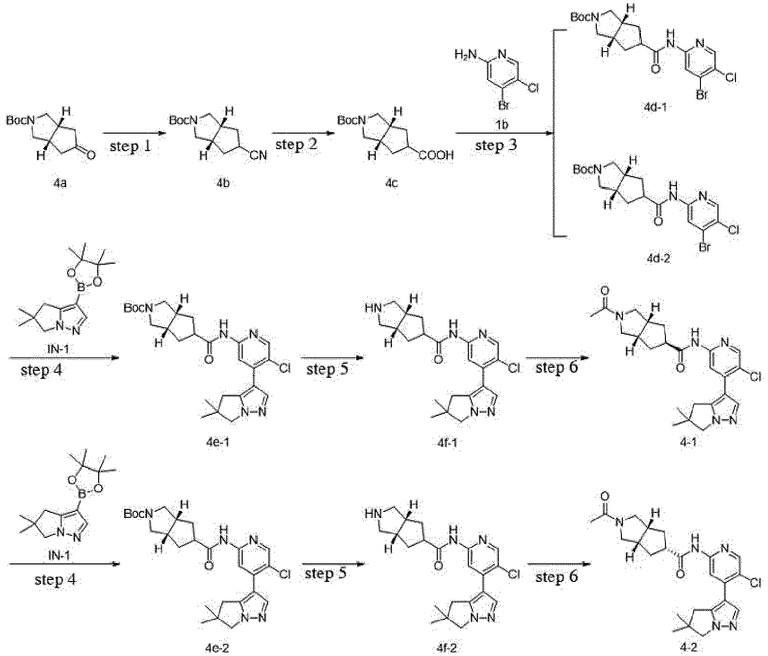

Step 1: (3a*R*,6a*S*)-5-cyanohexahydrocyclopenta[*c*]pyrrol-2(1*H*)-carboxylic acid tert-butyl ester **4b** (3a*R*,6a*S*)-5-oxohexahydrocyclopenta[*c*]pyrrole-2(1*H*)-carboxylic acid tert-butyl ester **4a** (3.0 g, 13.32 mmol) and p-toluenesulfonylmethyl isocyanide (992 mg, 33.29 mmol) were dissolved in ethylene glycol dimethyl ether (20 mL), under nitrogen protection, it was cooled to 0°C, ethanol (1.53 g, 33.29 mmol) and potassium tert-butoxide (4.88 g, 43.53 mmol) were added in sequence, and the temperature was lifted to 60°C for reaction overnight. The reaction was complete detected by TLC. The reaction solution was cooled to room temperature, added with water and extracted with ethyl acetate, the organic phases were combined, washed with water, washed with saturated salt water, dried over anhydrous sodium sulfate and concentrated, and the crude product was purified by silica gel column chromatography to obtain the title compound **4b** (1.22 g, yield 39%). ¹H NMR (400 MHz, CDCl₃) δ 3.54-3.50 (m, 2H), 3.16-3.13 (m, 2H), 3.00-2.93 (m, 1H), 2.87 (s, 2H), 2.20-2.14 (m, 2H), 1.94-1.90 (m, 2H), 1.44 (s, 9H).

### Step 2: (3aR,6aS)-2-(tert-butoxycarbonyl)octahydrocyclopenta[c]pyrrole-5-carboxylic acid 4c

Compound **4b** (590 mg, 2.50 mmol) was dissolved in ethylene glycol (6 mL) and water (1.2 mL), potassium hydroxide (421 mg, 7.50 mmol) was added at room temperature and the temperature was lifted to 115°C for reaction for 2 hours. The reaction was complete detected by TLC. The reaction solution was cooled to room temperature, added with water and extracted with ethyl acetate, the organic phase was discarded, the aqueous phase was adjusted to pH = 3 or so with dilute hydrochloric acid (1N) and extracted with ethyl acetate, the organic phases were combined, washed with water, washed with saturated salt water, dried over anhydrous sodium sulfate and concentrated to obtain the yellow oily liquid title compound **4c** (602 mg, crude product), which was directly used for the next step.

LC-MS: m/z=256.2 [M+H]⁺

### Step 3: (3aR,6aS)-5-((4-bromo-5-chloropyridin-2-yl)carbamoyl)hexahydrocyclopenta[c]pyrrole-2(1H)-car boxylic acid tert-butyl ester 4d-1,2

Compound **4c** (470 mg, crude product) and 4-bromo-5-chloro-2-aminopyridine **1b** (318 mg, 1.53 mmol) were dissolved in ethyl acetate (14 mL), pyridine (726 mg, 9.18 mmol) was added at room temperature, then 1-propylphosphonic anhydride (3.9 g, 6.12 mmol, 50% ethyl acetate solution) was added dropwise, and the reaction solution was stirred at room temperature overnight. The reaction was complete detected by TLC. The reaction solution was quenched with water and extracted with ethyl acetate, the organic phases were combined, washed with saturated salt water, dried over anhydrous sodium sulfate and concentrated, and the crude product was purified by silica gel column chromatography to obtain the low-polarity title compound **4d-1** (454 mg, yield 66%) and the high-polarity title compound **4d-2** (309 mg, yield 45%).

LC-MS: m/z=446.1[M+H]⁺

### Step 4:

(3a*R*,6a*S*)-5-((5-chloro-4-(5,5-dimethyl-5,6-dihydro-4*H*-pyrrolo[1,2-*b*]pyrazol-3-yl)pyridin-2-yl)c arbamoyl)hexahydrocyclopenta[*c*]pyrrole-2(1*H*)-carboxylic acid tert-butyl ester **4e-1,2** Compound **4d-1** (200 mg, 0.45 mmol) and Intermediate **IN-1** (171 mg, 0.68 mmol) were dissolved in 1,4-dioxane (6 mL) and water (2 mL), potassium carbonate (125 mg, 0.90 mmol) and Pd(dppf)Cl₂ dichloromethane complex (catalytic amount) were added in sequence at room temperature, and the temperature was lifted to 90°C to react overnight under nitrogen protection. The reaction was complete shown by TLC. The reaction solution was cooled to room temperature, added with water and extracted with ethyl acetate, the organic phases were combined, washed with saturated salt water, dried over anhydrous sodium sulfate and concentrated, and the crude product was purified by silica gel column chromatography to obtain the white solid title compound **4e-1** (109 mg, yield 48%).

LC-MS: m/z=500.3[M+H]⁺

Compound **4d-2** (309 mg, 0.70 mmol) and Intermediate **IN-1** (276 mg, 1.05 mmol) were dissolved in 1,4-dioxane (6 mL) and water (2 mL), potassium carbonate (194 mg, 1.4 mmol) and Pd(dppf)Cl₂ dichloromethane complex (catalytic amount) were added in sequence at room temperature, and the temperatue was lifted to 90°C for reaction overnight under nitrogen protection. The reaction was complete shown by TLC. The reaction solution was cooled to room temperature, added with water and extracted with ethyl acetate, the organic phases were combined, washed with saturated salt water, dried over anhydrous sodium sulfate and concentrated, and the crude product was purified by silica gel column chromatography to obtain the white solid title compound **4e-2** (72 mg, yield 32%).

LC-MS: m/z=500.3[M+H]⁺

### Step 5: (3aR,6aS)-N-(5-chloro-4-(5,5-dimethyl-5,6-dihydro-4H-pyrrolo[1,2-b]pyrazol-3-yl)pyridin-2-yl)o ctahydrocyclopenta[c]pyrrole-5-carboxamide 4f-1,2

Compound **4e-1** (109 mg, 0.22 mmol) was dissolved in dichloromethane (6 mL), trifluoroacetic acid (1 mL) was added dropwise, it was stirred at room temperature for 1 hour, and the disappearance of the starting material was shown by TLC. The reaction solution was concentrated, and the residue was dissolved in ethyl acetate, washed sequentially with saturated sodium carbonate and saturated salt water, dried over anhydrous sodium sulfate and concentrated to yield the light yellow solid title compound **4f-1** (80 mg, crude product), which was directly used for the next step.

LC-MS: m/z=400.2[M+H]⁺

Compound **4e-2** (72 mg, 0.14 mmol) was dissolved in dichloromethane (6 mL), trifluoroacetic acid (1 mL) was added dropwise, it was stirred at room temperature for 1 hour, and the disappearance of the starting material shown by TLC. The reaction solution was concentrated, and the residue was dissolved in ethyl acetate, washed sequentially with saturated sodium carbonate and saturated salt water, dried over anhydrous sodium sulfate and concentrated to yield the light yellow solid title compound **4f-2** (56 mg, crude product), which was directly used for the next step.

LC-MS: m/z=400.2[M+H]⁺

### Step 6: (3aR,5s,6aS)-2-acetyl-N-(5-chloro-4-(5,5-dimethyl-5,6-dihydro-4H-pyrrolo[1,2-b]pyrazol-3-yl)pyr idin-2-yl)octahydrocyclopenta[c]pyrrole-5-carboxamide (assumed) 4-1 &

### (3aR,5r,6aS)-2-acetyl-N-(5-chloro-4-(5,5-dimethyl-5,6-dihydro-4H-pyrrolo[1,2-b]pyrazol-3-yl)pyr idin-2-yl)octahydrocyclopenta[c]pyrrole-5-carboxamide (assumed) 4-2

Compound **4f-1** (30 mg, crude product) and triethylamine (16 mg, 0.16 mmol) were dissolved in dichloromethane (4 mL), acetic anhydride (12 mg, 0.12 mmol) was added dropwise, it was stirred at room temperature for half an hour, and the disappearance of the starting material was shown by TLC. The reaction solution was quenched with water and extracted with dichloromethane, the organic phases were combined, washed with saturated salt water, dried over anhydrous sodium sulfate and concentrated, and the crude product was purified by Prep-TLC to obtain the white solid title compound **4-1** (27 mg, two-step yield 75%).

LC-MS: m/z=442.2[M+H]⁺

¹H NMR (400 MHz, DMSO-*d₆*) δ 10.59 (s, 1H), 8.35 (s, 1H), 8.25 (s, 1H), 7.99 (s, 1H), 3.94 (s, 2H), 3.66-3.62 (m, 1H), 3.51-3.46 (m, 1H), 3.24-3.12 (m, 3H), 2.93 (s, 2H), 2.86-2.80 (m, 1H), 2.77-2.71 (m, 1H), 2.00-1.92 (m, 5H), 1.81-1.72 (m, 2H), 1.26 (s, 6H). (97.34% purity by HPLC) Compound **4f-2** (56 mg, crude product) and triethylamine (28 mg, 0.27 mmol) were dissolved in dichloromethane (4 mL), acetic anhydride (21 mg, 0.21 mmol) was added dropwise, it was stirred at room temperature for half an hour, and the disappearance of the starting material was shown by TLC. The reaction solution was quenched with water and extracted with dichloromethane, the organic phases were combined, washed with saturated salt water, dried over anhydrous sodium sulfate and concentrated, and the crude product was purified by Prep-TLC to obtain the white solid title compound **4-2** (40 mg, two-step yield 63%).

LC-MS: m/z=442.2[M+H]⁺

¹H NMR (400 MHz, DMSO-*d₆*) δ 10.60 (s, 1H), 8.34 (s, 1H), 8.25 (s, 1H), 7.99 (s, 1H), 3.93 (s, 2H), 3.58-3.54 (m, 2H), 3.33-3.30 (m, 1H), 3.28-3.24 (m, 1H), 3.10-3.06 (m, 1H), 2.88 (s, 2H), 2.70-2.60 (m, 2H), 2.17-2.10 (m, 2H), 1.93 (s, 3H), 1.62-1.53 (m, 2H), 1.26 (s, 6H). (96.95% purity by HPLC)

### Embodiment 5

### (3aR,6aS)-2-(L-alanyl)-N-(5-chloro-4-(5,5-dimethyl-5,6-dihydro-4H-pyrrolo[1,2-b]pyrazol-3-yl)p yridin-2-yl)octahydrocyclopenta[c]pyrrole-5-carboxamide 5

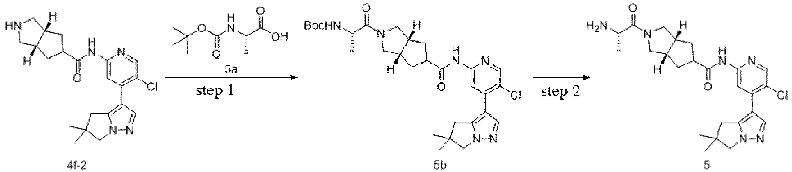

### Step 1: ((2S)-1-((3aR,6aS)-5-((5-chloro-4-(5,5-dimethyl-5,6-dihydro-4H-pyrrolo[1,2-b]pyrazol-3-yl)pyridi n-2-yl)carbamoyl)hexahydrocyclopenta[c]pyrrol-2(1H)-yl)-1-oxopropane-2-yl)carbamic acid tert-butyl ester 5b

Compound **4f-2** (70 mg, 0.18 mmol) and Boc-L-alanine **5a** (66 mg, 0.35 mmol) were dissolved in dichloromethane (8 mL), HATU (103 mg, 0.27 mmol) and N, N-diisopropylethylamine (46 mg, 0.36 mmol) were added in sequence, and the reaction was carried out at room temperature for 2 hours. The reaction was complete shown by TLC. The reaction solution was quenched with water and extracted with dichloromethane, the organic phases were combined, washed with saturated salt water, dried over anhydrous sodium sulfate and concentrated, and the crude product was purified by Prep-TLC to obtain the white solid title compound **5b** (100 mg, yield 100%).

### Step 2: (3aR,6aS)-2-(L-alanyl)-N-(5-chloro-4-(5,5-dimethyl-5,6-dihydro-4H-pyrrolo[1,2-b]pyrazol-3-yl)p yridin-2-yl)octahydrocyclopenta[c]pyrrole-5-carboxamide 5

Compound **5b** (100 mg, 0.18 mmol) was dissolved in dichloromethane (6 mL), trifluoroacetic acid (2 mL) was added, and the reaction was carried out at room temperature for 2 hours. The reaction was complete shown by TLC. The reaction solution was added with a saturated sodium bicarbonate solution and extracted with dichloromethane, the organic phases were combined, washed with saturated salt water, dried over anhydrous sodium sulfate and concentrated, and the crude product was purified by Prep-TLC to obtain the white solid title compound **5** (40 mg, yield 48%).

LC-MS: m/z=471.2[M+H]⁺

¹H NMR (400 MHz, DMSO-*d₆*) δ 10.63 (d, J = 3.2 Hz, 1H), 8.36 (s, 1H), 8.25 (s, 1H), 7.99 (s, 1H), 3.94 (s, 2H), 3.70-3.39 (m, 7H), 3.14-3.06 (m, 1H), 2.88 (s, 2H), 2.72-2.60 (m, 2H), 2.20-2.11 (m, 2H), 1.66-1.54 (m, 2H), 1.26 (s, 6H), 1.10 (dd, J = 6.8, 10.4 Hz, 3H). (98.97% purity by HPLC)

### Embodiment 6

### (3aR,6aS)-2-(D-alanyl)-N-(5-chloro-4-(5,5-dimethyl-5,6-dihydro-4H-pyrrolo[1,2-b]pyrazol-3-yl)p yridin-2-yl)octahydrocyclopenta[c]pyrrole-5-carboxamide 6

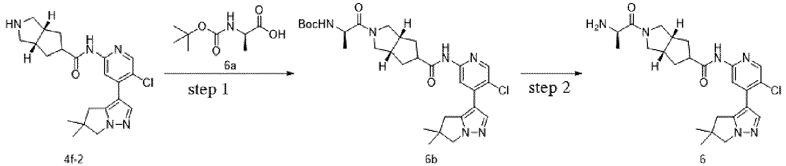

### Step 1: ((2R)-1-((3aR,6aS)-5-((5-chloro-4-(5,5-dimethyl-5,6-dihydro-4H-pyrrolo[1,2-b]pyrazol-3-yl)pyrid in-2-yl)carbamoyl)hexahydrocyclopenta[c]pyrrol-2(1H)-yl)-1-oxopropane-2-yl)carbamic acid tert-butyl ester 6b

Compound **4f-2** (70 mg, 0.18 mmol) and Boc-*D*-alanine **6a** (66 mg, 0.35 mmol) were dissolved in dichloromethane (8 mL), HATU (103 mg, 0.27 mmol) and *N,N*-diisopropylethylamine (46 mg, 0.36 mmol) were added in sequence, and the reaction was carried out at room temperature for 2 hours. The reaction was complete shown by TLC. The reaction solution was quenched with water and extracted with dichloromethane, the organic phases were combined, washed with saturated salt water, dried over anhydrous sodium sulfate and concentrated, and the crude product was purified by Prep-TLC to obtain the white solid title compound **6b** (90 mg, yield 90%).

### Step 2: (3aR,6aS)-2-(D-alanyl)-N-(5-chloro-4-(5,5-dimethyl-5,6-dihydro-4H-pyrrolo[1,2-b]pyrazol-3-yl)p yridin-2-yl)octahydrocyclopenta[c]pyrrole-5-carboxamide 6

Compound **6b** (90 mg, 0.16 mmol) was dissolved in dichloromethane (6 mL), trifluoroacetic acid (2 mL) was added, and the reaction was carried out at room temperature for 2 hours. The reaction was complete shown by TLC. The reaction solution was added with a saturated sodium bicarbonate solution and extracted with dichloromethane, the organic phases were combined, washed with saturated salt water, dried over anhydrous sodium sulfate and concentrated, and the crude product was purified by Prep-TLC to obtain the white solid title compound **6** (40 mg, yield 54%).

LC-MS: m/z=471.2 [M+H]⁺

¹H NMR (400 MHz, DMSO-*d₆*+D₂O) δ 8.35 (s, 1H), 8.22 (d, J = 3.6 Hz, 1H), 7.99 (d, J = 1.2 Hz, 1H), 3.93 (s, 2H), 3.86-3.82 (m, 0.5H), 3.71-3.67 (m, 0.5H), 3.51-3.29 (m, 4H), 3.16-3.04 (m, 1H), 2.87 (s, 2H), 2.74-2.64 (m, 2H), 2.19-2.14 (m, 2H), 1.66-1.55 (m, 2H), 1.25-1.22 (m, 9H). (97.18% purity by HPLC)

### Embodiment 7

### (3aR,5s,6aS)-2-acetyl-N-(5-chloro-4-(5-cyano-2,2-dimethyl-2,3-dihydro-1H-pyrrolizin-7-yl)pyridi n-2-yl)octahydrocyclopenta[c]pyrrole-5-carboxamide (assumed) 7-1

### (3aR,5r,6aS)-2-acetyl-N-(5-chloro-4-(5-cyano-2,2-dimethyl-2,3-dihydro-1H-pyrrolizin-7-yl)pyridi n-2-yl)octahydrocyclopenta[c]pyrrole-5-carboxamide (assumed) 7-2

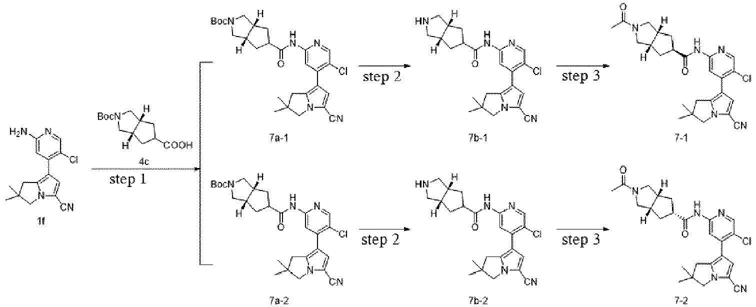

### Step 1: (3aR,6aS)-5-((5-chloro-4-(5-cyano-2,2-dimethyl-2,3-dihydro-1H-pyrrolizin-7-yl)pyridin-2-yl)carb amoyl)hexahydrocyclopenta[c]pyrrole-2(1H)-carbamic acid tert-butyl ester 7a-1,2

Compound **4c** (420 mg, 1.64 mmol) and Compound **If** (393 mg, 1.37 mmol) were dissolved in ethyl acetate (20 mL), pyridine (651 mg, 8.23 mmol) and 1-propylphosphonic anhydride (3.5 g, 5.50 mmol, 50% ethyl acetate solution) were added, and it was stirred at room temperature overnight. The complete reaction of the starting material was detected by TLC. The reaction solution was quenched with water and extracted with ethyl acetate, the organic layer was washed with saturated salt water, dried over anhydrous sodium sulfate and concentrated, and the crude product was purified by silica gel column chromatography to obtain the low-polarity white solid compound **7a-1** (110 mg, yield 15%) and the high-polarity white solid compound **7a-2** (105 mg, yield 15%).

### Step 2: (3aR,6aS)-N-(5-chloro-4-(5-cyano-2,2-dimethyl-2,3-dihydro-1H-pyrrolizin-7-yl)pyridin-2-yl)octa hydrocyclopenta[c]pyrrole-5-carboxamide 7b-1,2

Compound **7a-1** (110 mg, 0.21 mmol) was dissolved in dichloromethane (5 mL), trifluoroacetic acid (2 mL) was added, it was stirred at room temperature for 1 hour, and the reaction was complete shown by TLC. The reaction solution was concentrated to obtain the title compound **7b-1** (crude product), which was directly used for the next step.

Compound **7a-2** (105 mg, 0.20 mmol) was dissolved in dichloromethane (5 mL), trifluoroacetic acid (2 mL) was added, it was stirred at room temperature for 1 hour, and the reaction was complete shown by TLC. The reaction solution was concentrated to obtain the title compound **7b-1** (crude product), which was directly used for the next step.

### Step 3:

### (3aR,5s,6aS)-2-acetyl-N-(5-chloro-4-(5-cyano-2,2-dimethyl-2,3-dihydro-1H-pyrrolizin-7-yl)pyridi n-2-yl)octahydrocyclopenta[c]pyrrole-5-carboxamide (assumed) 7-1 &

### (3aR,5r,6aS)-2-acetyl-N-(5-chloro-4-(5-cyano-2,2-dimethyl-2,3-dihydro-1H-pyrrolizin-7-yl)pyridi n-2-yl)octahydrocyclopenta[c]pyrrole-5-carboxamide (assumed) 7-2

Compound **7b-1** (crude product) was dissolved in dichloromethane (5 mL), triethylamine (2 mL) and acetic anhydride (28 mg, 0.27 mmol) were added in sequence, it was stirred at room temperature for 1 hour, and the reaction was complete shown by TLC. The reaction solution was concentrated, dissolved with ethyl acetate, washed with water, washed with saturated salt water, dried over anhydrous sodium sulfate and concentrated, and the crude product was purified by Prep-TLC to obtain the white solid title compound **7-1** (75 mg, yield 77%).

LC-MS: m/z=466.2 [M+H]⁺

¹H NMR (400 MHz, DMSO-*d₆*) δ 10.64 (s, 1H), 8.37 (s, 1H), 8.22 (s, 1H), 7.42 (s, 1H), 3.95 (s, 2H), 3.65 (dd, J = 10.8, 8.4 Hz, 1H), 3.49 (dd, J = 10.8, 8.4 Hz ,1H), 3.24-3.12 (m, 3H), 2.87 (s, 2H), 2.84-2.75 (m, 2H), 2.02-1.94 (m, 5H), 1.81-1.72 (m, 2H), 1.24 (s, 6H). (98.73% purity by HPLC)

Compound **7b-2** (crude product) was dissolved in dichloromethane (5 mL), triethylamine (2 mL) and acetic anhydride (26 mg, 0.25 mmol) were added in sequence, it was stirred at room temperature for 1 hour, and the reaction was complete shown by TLC. The reaction solution was concentrated, dissolved with ethyl acetate, washed with water, washed with saturated salt water, dried over anhydrous sodium sulfate and concentrated, and the crude product was purified by Prep-TLC to obtain the white solid title compound **7-2** (67 mg, two-step yield 72%).

LC-MS: m/z=466.2 [M+H]⁺

¹H NMR (400 MHz, DMSO-*d₆*) δ 10.66 (s, 1H), 8.37 (s, 1H), 8.22 (s, 1H), 7.41 (s, 1H), 3.95 (s, 2H), 3.56 (dd, J = 10.4, 7.6 Hz, 1H), 3.42-3.35 (m, 2H), 3.26 (dd, J = 12.4, 4.4 Hz, 1H), 3.14-3.05 (m, 1H), 2.86 (s, 2H), 2.73-2.58 (m, 2H), 2.18-2.11 (m, 2H), 1.96 (s, 3H), 1.66-1.53 (m, 2H), 1.24 (s, 6H). (96.66% purity by HPLC)

### Embodiment 8

### (3aR,5s,6aS)-2-acetyl-N-(4-(5-carbamoyl-2,2-dimethyl-2,3-dihydro-1H-pyrrolizin-7-yl)-5-chlorop yridin-2-yl)octahydrocyclopenta[c]pyrrole-5-carboxamide (assumed) 8-1

### (3aR,5r,6aS)-2-acetyl-N-(4-(5-carbamoyl-2,2-dimethyl-2,3-dihydro-1H-pyrrolizin-7-yl)-5-chlorop yridin-2-yl)octahydrocyclopenta[c]pyrrole-5-carboxamide (assumed) 8-2

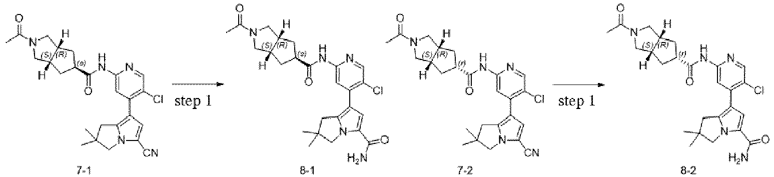

Compound **7-1** (150 mg, 0.32 mmol) was dissolved in dimethyl sulfoxide (2 mL), potassium carbonate (89 mg, 0.64 mmol) and hydrogen peroxide (2 mL, 30%) were added, it was stirred at room temperature for 1 hour, and the complete reaction of the starting material was shown by TLC. The reaction solution was quenched with water and extracted with ethyl acetate, the organic phases were combined, washed with water, washed with saturated salt water, dried over anhydrous sodium sulfate and concentrated, and the crude product was separated and purified by silica gel column chromatography to obtain the white solid title compound **8-1** (78 mg, yield 50%). LC-MS: m/z=484.2[M+H]⁺

¹H NMR (400 MHz, DMSO-*d₆*) δ 10.57 (s, 1H), 8.32 (s, 1H), 8.23 (s, 1H), 7.63 (br, 1H), 7.34 (s, 1H), 6.96 (br, 1H), 4.03 (s, 2H), 3.67-3.62 (m, 1H), 3.52-3.47 (m, 1H), 3.24-3.12 (m, 3H), 2.87-2.71 (m, 4H), 2.02-1.92 (m, 5H), 1.81-1.73 (m, 2H), 1.20 (s, 6H). (97.16% purity by HPLC) Compound 7-2 (230 mg, 0.49 mmol) was dissolved in dimethyl sulfoxide (3 mL), potassium carbonate (150 mg, 1.08 mmol) and hydrogen peroxide (3 mL, 30%) were added, it was stirred at room temperature for 1 hour, and the complete reaction of the starting material was shown by TLC. The reaction solution was quenched with water and extracted with ethyl acetate, the organic phases were combined, washed with water, washed with saturated salt water, dried over anhydrous sodium sulfate and concentrated, and the crude product was separated and purified by silica gel column chromatography to obtain the white solid title compound **8-2** (132 mg, yield 56%). LC-MS: m/z=484.2[M+H]⁺

¹H NMR (400 MHz, DMSO-*d₆*) δ 10.59 (s, 1H), 8.32 (s, 1H), 8.23 (s, 1H), 7.63 (br, 1H), 7.34 (s, 1H), 6.95 (br, 1H), 4.03 (s, 2H), 3.59-3.54 (m, 1H), 3.42-3.32 (m, 2H), 3.28-3.34 (m, 1H), 3.11-3.07 (m, 1H), 2.77 (s, 2H), 2.72-2.58 (m, 2H), 2.17-2.11 (m, 2H), 1.93 (s, 3H), 1.66-1.53 (m, 2H), 1.20 (s, 6H). (97.88% purity by HPLC)

### Embodiment 9

### 7-(2-((1S,3R)-3-acetylaminocyclohexane-1-carboxamido)-5-chloropyridin-4-yl)-N,N,2,2-tetrameth yl-2,3-dihydro-1H-pyrrolizine-5-carboxamide 9

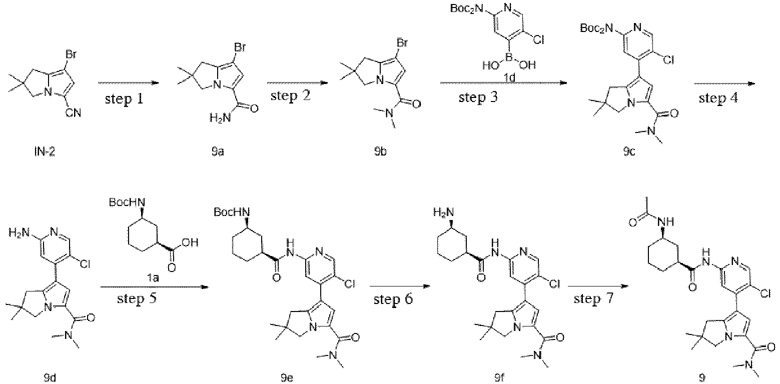

### Step 1: 7-bromo-2,2-dimethyl-2,3-dihydro-1H-pyrrolizine-5-carboxamide 9a

Intermediate **IN-2** (1.50 g, 6.27 mmol) was dissolved in methanol (10 mL) and water (4 mL), sodium hydroxide (0.50 g, 12.50 mmol) was added at room temperature, the temperature was lifted to 80°C for reaction for 3 hours, and the reaction was complete shown by TLC. The reaction solution was cooled to room temperature, concentrated, added with water,the solid was precipitated and filtered, and the filter cake was washed and dried to obtain the white solid title compound **9a** (1.2 g, yield 74%).

### Step 2: 7-bromo-N,N,2,2-tetramethyl-2,3-dihydro-1H-pyrrolizine-5-carboxamide 9b

Compound **9a** (550 mg, 2.1 mmol) was dissolved in anhydrous tetrahydrofuran (10 mL), sodium hydride (154 mg, 3.85 mmol, 60%) was added at 0°C, it was stirred for 10 minutes, methyl iodide (1.2 g, 8.45 mmol) was added, and the temperature was lifted to room temperature for reaction for 2 hours. The reaction was complete shown by TLC. The reaction solution was quenched with water and extracted with ethyl acetate, the organic phases were combined, washed with saturated salt water, dried over anhydrous sodium sulfate and concentrated, and the crude product was chromatographed on silica gel column to obtain the yellow solid title compound **9b** (500 mg, yield 83%).

¹H NMR (400 MHz, DMSO-*d₆*) δ 6.58 (s, 1H), 3.92 (s, 2H), 3.04 (s, 6H), 2.54 (s, 2H), 1.17 (s, 6H).

### Step 3: (5-chloro-4-(5-(dimethylcarbamoyl)-2,2-dimethyl-2,3-dihydro-1H-pyrrolizin-7-yl)pyridin-2-yl)im inodicarboxylic acid di-tert-butyl ester 9c

Compound **9b** (300 mg, 1.05 mmol) was dissolved in dioxane (10 mL) and water (3 mL), Compound 1d (525 mg, 1.41 mmol), sodium carbonate (223 mg, 2.10 mmol) and Pd(dppf)Cl₂ (39 mg, 0.05 mmol) were added in sequence at room temperature, under nitrogen protection, the temperature was lifted to 100°C for reaction for 2 hours. The reaction was complete shown by TLC. The reaction solution was cooled to room temperature, added with water and extracted with ethyl acetate, the organic phases were combined, washed with water, washed with saturated salt water, dried over anhydrous sodium sulfate and concentrated, and the crude product was separated and purified by silica gel column chromatography to obtain the white solid title compound **9c** (300 mg, yield 54%).

### Step 4: 7-(2-amino-5-chloropyridin-4-yl)-N,N,2,2-tetramethyl-2,3-dihydro-1H-pyrrolizine-5-carboxamide 9d

Compound **9c** (300 mg, 0.69 mmol) was dissolved in dichloromethane (6 mL), trifluoroacetic acid (2 mL) was added, the reaction was carried out for 3 hours at room temperature, and the starting material was remained shown by TLC. The reaction solution was added with saturated sodium bicarbonate solution to adjust the alkalinity and extracted with dichloromethane, the organic phases were combined, washed with water, washed with saturated salt water, dried over anhydrous sodium sulfate and concentrated, and the crude product was separated and purified by silica gel column chromatography to obtain the white solid title compound **9d** (80 mg, yield 43%).

### Step 5: ((1R,3S)-3-((5-chloro-4-(5-(dimethylcarbamoyl)-2,2-dimethyl-2,3-dihydro-1H-pyrrolizin-7-yl)pyri din-2-yl)carbamoyl)cyclohexyl)carbamic acid tert-butyl ester 9e

Compound **9d** (78 mg, 0.23 mmol) and compound **1a** (68 mg, 0.28 mmol) were dissolved in ethyl acetate (10 mL), 1-propylphosphonic anhydride (293 mg, 0.46 mmol, 50% ethyl acetate solution) and pyridine (73 mg, 0.92 mmol) were added at room temperature, and the temperature was lifted to 80°C for reaction for 3 hours. the reaction was complete shown by TLC. The reaction solution was cooled to room temperature, added with water and extracted with ethyl acetate, and the organic phases were combined, washed with water, washed with saturated salt water, dried over anhydrous sodium sulfate and concentrated to obtain the title compound **9e** (177 mg, crude product), which was directly used for the next step.

### Step 6: 7-(2-((1S,3R)-3-aminocyclohexane-1-carboxamido)-5-chloropyridin-4-yl)-N,N,2,2-tetramethyl-2,3 -dihydro-1H-pyrrolizine-5-carboxamide 9f

Compound **9e** (177 mg, crude product) was dissolved in dichloromethane (6 mL), trifluoroacetic acid (2 mL) was added, and the reaction was carried out for 1 hour at room temperature. The reaction was complete shown by TLC. The reaction solution was quenched with water and extracted with ethyl acetate, and the organic phases were combined, washed with water, washed with saturated salt water, dried over anhydrous sodium sulfate and concentrated to obtain the title compound **9f** (130 mg, crude product),which was directly used for the next step.

### Step 7: 7-(2-((1S,3R)-3-acetylaminocyclohexane-1-carboxamido)-5-chloropyridin-4-yl)-N,N,2,2-tetrameth yl-2,3-dihydro-1H-pyrrolizine-5-carboxamide 9

Compound **9f** (130 mg, crude product) was dissolved in tetrahydrofuran (8 mL), acetic anhydride (34 mg, 0.33 mmol) and triethylamine (42 mg, 0.42 mmol) were added, and the reaction was carried out at room temperature for 1 hour. The reaction was complete shown by TLC. The reaction solution was quenched with water and extracted with ethyl acetate, the organic phases were combined, washed with water, washed with saturated salt water, dried over anhydrous sodium sulfate and concentrated, and the crude product was separated and purified by silica gel column chromatography to obtain the white solid title compound **9** (70 mg, three-step yield 61%). LC-MS: m/z=500.3[M+H]⁺

¹H NMR(400 MHz, DMSO-*d₆*) δ 10.55 (s, 1H), 8.33 (s, 1H), 8.22 (s, 1H), 7.79 (d, J = 7.6 Hz, 1H), 6.99 (s, 1H), 3.96 (s, 2H), 3.59-3.55 (m, 1H), 3.11 (s, 6H), 2.80 (s, 2H), 2.64-2.58 (m, 1H), 1.91-1.88 (m, 2H), 1.78-1.76 (m, 6H), 1.32-1.29 (m, 2H), 1.21 (s, 6H), 1.12-1.05 (m, 1H). (96.72% purity by HPLC)

### Embodiment 10

### (1S,3R)-3-acetamido-N-(4-(5-cyano-2,2-dimethyl-2,3-dihydro-1H-pyrrolizin-7-yl)-5-fluoropyridin -2-yl)cyclohexane-1-carboxamide 10

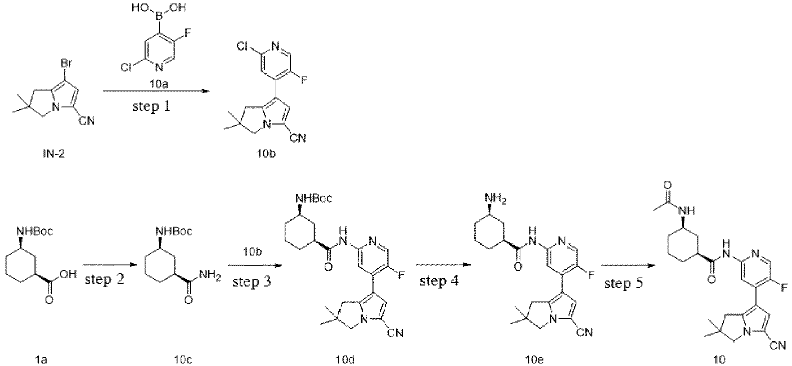

### Step 1: 7-(2-chloro-5-fluoropyridin-4-yl)-2,2-dimethyl-2,3-dihydro-1H-pyrrolizine-5-carbonitrile 10b

Intermediate **IN-2** (500 mg, 2.09 mmol) and 2-chloro-5-fluoropyridine-4-boronic acid **10a** (550 mg, 3.14 mmol) were dissolved in a mixed solvent of 1,4-dioxane (10 mL) and water (2 mL), sodium carbonate (332 mg, 3.13 mmol) and Pd(dppf)Cl₂ (153 mg, 0.21 mmol) were added at room temperature, the temperature was lifted to 90°C for reaction for 3 hours under nitrogen protection, and the reaction was complete shown by TLC. The reaction solution was cooled to room temperature, added with water and extracted with ethyl acetate, the organic phases were combined, washed with saturated salt water, dried over anhydrous sodium sulfate and concentrated, and the crude product was separated and purified by silica gel column chromatography to obtain the title compound **10b** (120 mg, yield 20%).

### Step 2: ((1R,3S)-3-carbamoyl cyclohexyl)carbamic acid tert-butyl ester 10c

Compound **1a** (400 mg, 1.6 mmol) was dissolved in dry *N*,*N*-dimethylformamide (10 mL), HATU (950 mg, 2.5 mmol) and *N,N*-diisopropylethylamine (413 mg, 3.2 mmol) were added, it was stirred for 15 minutes, ammonium chloride (101 mg, 1.9 mmol) was added, it was stirred overnight at room temperature, and the reaction was complete shown by TLC. The reaction solution was poured into water, the solid was precipitated and filtered, and the filter cake was washed and dried to obtain the white-like solid title compound **10c** (330 mg, crude product), which was directly used for the next step.

### Step 3: ((1R,3S)-3-((4-(5-cyano-2,2-dimethyl-2,3-dihydro-1H-pyrrolizin-7-yl)-5-fluoropyridin-2-yl)carba moyl)cyclohexyl)carbamic acid tert-butyl ester 10d

Compound **10b** (120 mg, 0.41 mmol) and Compound **10c** (149 mg, 0.61 mmol) were dissolved in 1,4-dioxane (10 mL), Pd₂(dba)₃ (38 mg, 0.04 mmol), XPhos (39 mg, 0.08 mmol) and potassium tert-butoxide (92 mg, 0.82 mmol) were added at room temperature, and the temperature was lifted to 100°C for reaction overnight. The reaction was complete shown by TLC. The reaction solution was cooled to room temperature, added with water and extracted with ethyl acetate, the organic phases were combined, washed with saturated salt water, dried over anhydrous sodium sulfate and concentrated, and the crude product was separated and purified by silica gel column chromatography to obtain the title compound **10d** (120 mg, yield 59%).

### Step 4: (1S,3R)-3-amino-N-(4-(5-cyano-2,2-dimethyl-2,3-dihydro-1H-pyrrolizin-7-yl)-5-fluoropyridin-2-y l)cyclohexane-1-carboxamide 10e

Compound **10d** (120 mg, 0.24 mmol) was dissolved in dichloromethane (6 mL), trifluoroacetic acid (2 mL) was added, and the reaction was carried out for 1 hour at room temperature. The reaction was complete shown by TLC. The reaction solution was quenched by adding a saturated sodium bicarbonate solution and extracted with dichloromethane, and the organic phases were combined, washed with saturated salt water, dried over anhydrous sodium sulfate and concentrated to obtain the title compound **10e** (100 mg, crude product), which was directly used for the next step.

### Step 5: (1S,3R)-3-acetamido-N-(4-(5-cyano-2,2-dimethyl-2,3-dihydro-1H-pyrrolizin-7-yl)-5-fluoropyridin -2-yl)cyclohexane-1-carboxamide 10

Compound **10e** (100 mg, crude product) was dissolved in tetrahydrofuran (8 mL), acetic anhydride (31 mg, 0.30 mmol) and potassium carbonate (52 mg, 0.38 mmol) were added, the reaction was carried out for 1 hour at room temperature, and the reaction was complete shown by TLC. The reaction solution was added with water and extracted with ethyl acetate, the organic phases were combined, washed with saturated salt water, dried over anhydrous sodium sulfate and concentrated, and the crude product was purified by Prep-TLC to obtain the title compound **10** (58 mg, two-step yield 55%).

LC-MS: m/z=438.2 [M+H]⁺

¹H NMR (400 MHz, DMSO-*d₆*) δ 10.51 (s, 1H), 8.30-8.28 (m, 2H), 7.78 (d, *J* = 8.0 Hz, 1H), 7.36 (d, *J=* 2.0 Hz, 1H), 3.95 (s, 2H), 3.59-3.54 (m, 1H), 2.94 (s, 2H), 2.63-2.58 (m, 1H), 1.90-1.85 (m, 1H), 1.78 (s, 6H), 1.36-1.25 (m, 9H), 1.13-1.05 (m, 1H). (99.62% purity by HPLC)

### Embodiment 11

### (1S,3R)-3-acetamido-N-(4-(5-cyano-2,2-dimethyl-2,3-dihydro-1H-pyrrolizin-7-yl)pyridin-2-yl)cyc lohexane-1-carboxamide 11

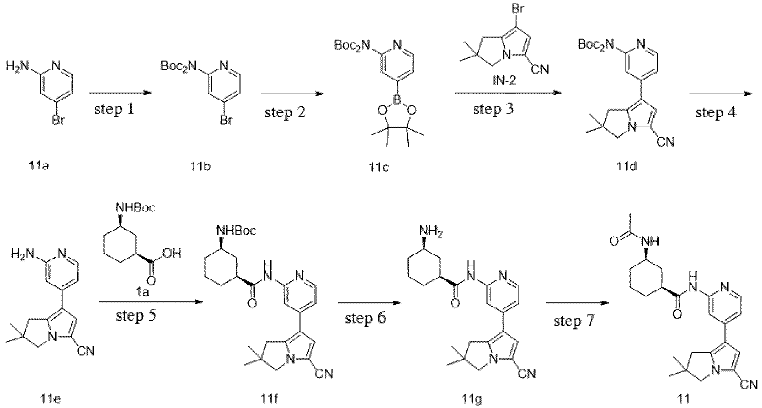

### Step 1: (4-bromopyridin-2-yl)iminodicarboxylic acid di-tert-butyl ester 11b

2-amino-4-bromopyridine **11a** (2.0 g, 11.56 mmol) and di-tert-butyl dicarbonate (6.5 g, 29.78 mmol) were dissolved in dichloromethane (20 mL), 4-dimethylaminopyridine (122 mg, 0.1 mmol) was added, the reaction was carried out overnight at room temperature, and the reaction was complete shown by TLC. The reaction solution was added with water and extracted with dichloromethane, the organic phases were combined, washed with saturated salt water, dried over anhydrous sodium sulfate and concentrated, and the crude product was chromatographed on silica gel column to obtain the white solid title compound **11b** (3.5 g, yield 81%).

### Step 2: (4-(4,4,5,5-tetramethyl-1,3,2-dioxaboran-2-yl)pyridin-2-yl)iminodicarboxylic acid di-tert-butyl ester 11c

Under nitrogen protection, Compound **11b** (3.5 g, 9.38 mmol) and bis(pinacolato)diboron (3.57 g, 14.06 mmol) were dissolved in 1,4-dioxane (50 mL), potassium acetate (2.30 g, 23.44 mmol) and Pd(dppf)Cl₂ (343 mg, 0.47 mmol) were added at room temperature, the temperature was lifted to 90°C, it was stirred overnight, and the reaction was complete shown by TLC. The reaction solution was cooled to room temperature, added with water and extracted with ethyl acetate, the organic phases were combined, washed with saturated salt water, dried over anhydrous sodium sulfate and concentrated, and the crude product was purified by silica gel column chromatography to obtain the white solid title compound **11c** (2.7 g, yield 68%).

### Step 3: (4-(5-cyano-2,2-dimethyl-2,3-dihydro-1H-pyrrolizin-7-yl)pyridin-2-yl)iminodicarboxylic acid di-tert-butyl ester 11d

Under nitrogen protection, compound **11c** (500 mg, 1.19 mmol) and Intermediate **IN-2** (340 mg, 1.42 mmol) were dissolved in 1,4-dioxane (6 mL) and water (2 mL), sodium carbonate (254 mg, 2.40 mmol) and Pd(dppf)Cl₂ (88 mg, 0.12 mmol) were added at room temperature, the temperature was lifted to 90°C for reaction for 3 hours, and the reaction was complete shown by TLC. The reaction solution was cooled to room temperature, added with water and extracted with ethyl acetate, the organic phases were combined, washed with saturated salt water, dried over anhydrous sodium sulfate and concentrated, and the crude product was purified by silica gel column chromatography to obtain the white solid title compound **11d** (380 mg, yield 70%).

### Step 4: 7-(2-aminopyridin-4-yl) -2,2-dimethyl-2,3-dihydro-1H-pyrrolizine-5-carbonitrile 11e

Compound **11d** (380 mg, 1.08 mmol) was dissolved in dichloromethane (9 mL), trifluoroacetic acid (3 mL) was added to react for 30 minutes at room temperature, and the reaction was complete shown by TLC. The reaction solution was quenched by adding a saturated sodium bicarbonate aqueous solution and extracted with dichloromethane, the organic phases were combined, washed with saturated salt water, dried over anhydrous sodium sulfate and concentrated, and the crude product was purified by silica gel column chromatography to obtain the white solid title compound **11e** (220 mg, yield 79%).

### Step 5: ((1R,3S)-3-((4-(5-cyano-2,2-dimethyl-2,3-dihydro-1H-pyrrolizin-7-yl)pyridin-2-yl)carbamoyl)cycl ohexyl)carbamic acid tert-butyl ester 11f

Compound **11e** (220 mg, 0.87 mmol), Compound 1**a** (233 mg, 0.96 mmol) and pyridine (206 mg, 2.60 mmol) were dissolved in ethyl acetate (10 mL), 1-propylphosphonic anhydride (1.11 g, 1.74 mmol, 50% ethyl acetate solution) was added, the temperature was lifted to 40°C for reaction overnight, and the reaction was complete shown by TLC. The reaction solution was quenched with water and extracted with ethyl acetate, the organic phases were combined, washed with saturated salt water, dried over anhydrous sodium sulfate and concentrated, and the crude product was purified by silica gel column chromatography to obtain the white title compound **11f** (234 mg, yield 56%).

### Step 6: (1S,3R)-3-amino-N-(4-(5-cyano-2,2-dimethyl-2,3-dihydro-1H-pyrrolizin-7-yl)pyridin-2-yl)cycloh exane-1-carboxamide 11g

Compound **11f** (234 mg, 0.49 mmol) was dissolved in dichloromethane (9 mL), trifluoroacetic acid (3 mL) was added, the reaction was carried out for 30 minutes at room temperature, and the reaction was complete shown by TLC. The reaction solution was added with a saturated sodium bicarbonate aqueous solution and extracted with dichloromethane, the organic phases were combined, washed with saturated salt water, dried over anhydrous sodium sulfate and concentrated, and the crude product was purified by silica gel column chromatography to obtain the white solid compound **11g** (121 mg, 65% yield).

### Step 7: (1S,3R)-3-acetamido-N-(4-(5-cyano-2,2-dimethyl-2,3-dihydro-1H-pyrrolizin-7-yl)pyridin-2-yl)cyc lohexane-1-carboxamide 11

Compound **11g** (121 mg, 0.32 mmol) was dissolved in dichloromethane (10 mL), triethylamine (66 mg, 0.65 mmol) and acetic anhydride (40 mg, 0.39 mmol) were added, the reaction was carried out for 1 hour at room temperature, and the reaction was complete shown by TLC. The reaction solution was added with a saturated sodium bicarbonate aqueous solution and extracted with dichloromethane, the organic phases were combined, washed with saturated salt water, dried over anhydrous sodium sulfate and concentrated, and the crude product was purified by silica gel column chromatography to obtain the white solid compound **11** (75 mg, yield 56%).

LC-MS: m/z=420.3[M+H]⁺

¹H NMR (400 MHz, DMSO-*d₆*) δ 10.42 (s, 1H), 8.23-8.22 (m, 2H), 7.80 (d, J = 8.0 Hz, 1H), 7.50 (s, 1H), 7.22 (dd, J = 4.2, 1.2 Hz, 1H), 3.91 (s, 2H), 3.58-3.53 (m, 1H), 2.97 (s, 2H), 2.64-2.59 (m, 1H), 1.90-1.87 (m, 1H), 1.80-1.76 (m, 6H), 1.32-1.23 (m, 9H), 1.10-1.05 (m, 1H). (95.42% purity by HPLC)

### Embodiment 12

### 7-(2-((1S,3R)-3-acetylaminocyclohexane-1-carboxamido)pyridin-4-yl)-2,2-dimethyl-2,3-dihydro-1H-pyrrolizine-5-carboxamide 12

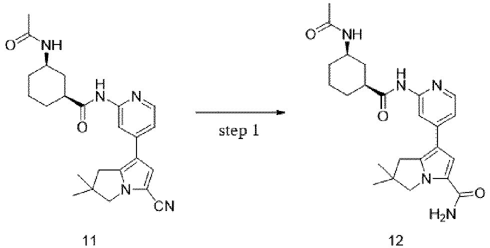

Compound **11** (75 mg, 0.18 mmol) and potassium carbonate (49 mg, 0.35 mmol) were dissolved in dimethyl sulfoxide (5 mL), hydrogen peroxide (60 mg, 30%) was added, the reaction was carried out for 2 hours at room temperature, and the reaction was complete shown by TLC. The reaction solution was added with a saturated sodium bicarbonate aqueous solution and extracted with dichloromethane, the organic phases were combined, washed with saturated salt water, dried over anhydrous sodium sulfate and concentrated, and the crude product was purified by silica gel column chromatography to obtain the white solid title compound **12** (15 mg, yield 19%). LC-MS: m/z=438.3[M+H]⁺

¹H NMR (400 MHz, DMSO-*d₆*) δ 10.34 (s, 1H), 8.23 (s, 1H), 8.19 (d, J = 5.2 Hz, 1H), 7.79 (d, J = 8.0 Hz, 1H), 7.64-7.56 (m, 1H), 7.33 (s, 1H), 7.06 (d, J = 4.8 Hz, 1H), 7.00-6.91 (m, 1H), 4.00 (s, 2H), 3.62-3.53 (m, 1H), 2.88 (s, 2H), 2.66-2.62 (m, 1H), 2.01-1.89 (m, 2H), 1.78 (s, 6H), 1.32-1.23 (m, 9H). (97.42% purity by HPLC)

### Embodiment 13

### 7-(2-((1S,3R)-3-aminocyclohexane-1-carboxamido)-5-chloropyridin-4-yl)-N,2,2-trimethyl-2,3-dih ydro-1H-pyrrolizine-5-carboxamide 13

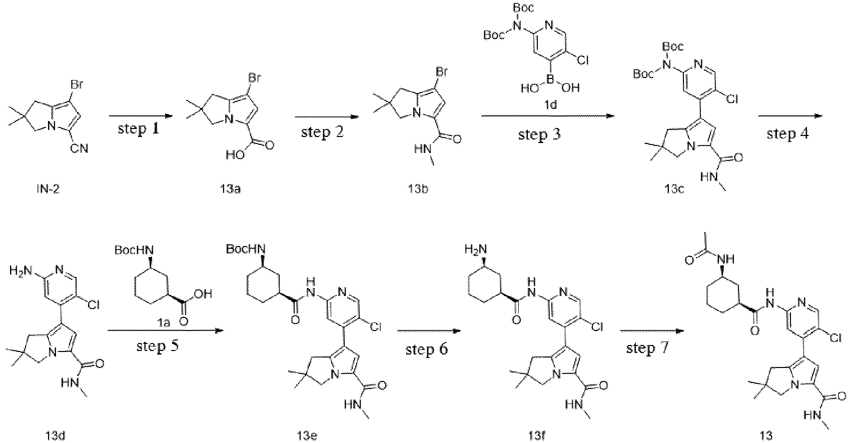

### Step 1: 7-bromo-2,2-dimethyl-2,3-dihydro-1H-pyrrolizine-5-carboxylic acid 13a

Intermediate **IN-2** (2.0 g, 8.36 mmol) was dissolved in ethanol (40 mL), sodium hydroxide (3.34 g, 83.52 mmol) was added at room temperature, the temperater was lifted to 80°C for reaction for 48 hours, and the reaction was complete shown by TLC. The reaction solution was cooled to room temperature, added with diluted hydrochloric acid (1N) to adjust acidity, diluted with water and extracted with ethyl acetate, the organic phases were combined, washed with water, washed with saturated salt water, dried over anhydrous sodium sulfate and concentrated, and the crude product was chromatographed on silica gel column to obtain the white solid title compound **13a** (1.50 g, yield 70%).

LC-MS: m/z=258.0[M+H]⁺

### Step 2: 7-bromo-N,2,2-trimethyl -2,3-dihydro-1H-pyrrolizine-5-carboxamide 13b

Compound **13a** (1.0 g, 3.87 mmol) and methylamine hydrochloride (392 mg, 5.80 mmol) were dissolved in dichloromethane (10 mL), HATU (2.21 g, 5.81 mmol) and N,N-diisopropylethylamine (1.0 g, 7.74 mmol) were added, the reaction was carried out at room temperature for 2 hours, and the reaction was complete shown by TLC. The reaction solution was quenched with water and extracted with ethyl acetate, the organic phases were combined, washed with water, washed with saturated salt water, dried over anhydrous sodium sulfate and concentrated, and the crude product was purified by silica gel column chromatography to obtain the white solid title compound **13b** (1.1 g, yield 100%).

LC-MS: m/z=271.1[M+H]⁺

### Step 3: (5-chloro-4-(2,2-dimethyl-5-(methylcarbamoyl)-2,3-dihydro-1H-pyrrolizin-7-yl)pyridin-2-yl)imin odicarboxylic acid di-tert-butyl ester 13c

Compound **13b** (1.1 g, 4.06 mmol), Compound **1d** (2.22 g, 5.96 mmol) and sodium carbonate (860 mg, 8.11 mmol) were dispersed into a mixed solvent of 1,2-dichloroethane (20 mL) and water (5 mL), Pd(dppf)Cl₂ (170 mg, 0.23 mmol) was added at room temperature, the temperature was lifted to 85°C for reaction for 3 hours under nitrogen protection, and the reaction was complete shown by TLC. The reaction solution was cooled to room temperature, quenched with water and extracted with ethyl acetate, the organic phases were combined, washed with water, washed with saturated salt water, dried over anhydrous sodium sulfate and concentrated, and the crude product was purified by silica gel column chromatography to obtain the white solid title compound **13c** (250 mg, yield 15%).

LC-MS: m/z=419.2[M+H-Boc]⁺

### Step 4: 7-(2-amino-5-chloropyridin-4-yl)-N,2,2-trimethyl-2,3-dihydro-1H-pyrrolizine-5-carboxamide 13d

Compound **13c** (250 mg, 0.48 mmol) was dissolved in dichloromethane (6 mL), trifluoroacetic acid (2 mL) was added, the reaction was carried out for 1 hour at room temperature, and the reaction was complete shown by TLC. The reaction solution was added with a saturated sodium bicarbonate solution to adjust the alkalinity and extracted with ethyl acetate, the organic phases were combined, washed with water, washed with saturated salt water, dried over anhydrous sodium sulfate and concentrated, and the crude product was purified by silica gel column chromatography to obtain the white solid title compound **13d** (170 mg, yield 89%).

### Step 5: ((1R,3S)-3-((5-chloro-4-(2,2-dimethyl-5-(methylcarbamoyl)-2,3-dihydro-1H-pyrrolizin-7-yl)pyridi n-2-yl)carbamoyl)cyclohexyl)carbamic acid tert-butyl ester 13e

Compound **13d** (100 mg, 0.31 mmol) and Compound **1a** (90 mg, 0.37 mmol) was dissolved in N,N-dimethylformamide (6 mL), 1-propylphosphonic anhydride (394 mg, 0.62 mmol, 50% N,N-dimethylformamide solution) and pyridine (147 mg, 1.86 mmol) were added at room temperature, the temperature was lifted to 80°C for reaction for 2 hours. The reaction was complete shown by TLC. The reaction solution was cooled to room temperature, added with water and extracted with ethyl acetate, the organic phases were combined, washed with water, washed with saturated salt water, dried over anhydrous sodium sulfate and concentrated, and the crude product was purified by silica gel column chromatography to obtain the white solid title compound **13e** (60 mg, yield 36%).

LC-MS: m/z=544.3[M+H]⁺

### Step 6:

### 7-(2-((1S,3R)-3-aminocyclohexane-1-carboxamido)-5-chloropyridin-4-yl)-N,2,2-trimethyl-2,3-dih ydro-1H-pyrrolizine-5-carboxamide 13f

Compound **13e** (60 mg, 0.11 mmol) was dissolved in dichloromethane (6 mL), trifluoroacetic acid (2 mL) was added, the reaction was carried out for 1 hour at room temperature, and the reaction was complete shown by TLC. It was added with a saturated sodium bicarbonate solution to adjust the alkalinity, extracted with ethyl acetate, washed with water, dried over anhydrous sodium sulfate, concentrated, and purified by silica gel column chromatography to obtain the white solid title compound **13f** (47 mg, yield 96%).

### Step 7: 7-(2-((1S,3R)-3-aminocyclohexane-1-carboxamido)-5-chloropyridin-4-yl)-N,2,2-trimethyl-2,3-dih ydro-1H-pyrrolizine-5-carboxamide 13

Compound **13f** (47 mg, 0.10 mmol) was dissolved in tetrahydrofuran (10 mL), acetic anhydride (14 mg, 0.14 mmol) and triethylamine (25 mg, 0.25 mmol) were added, and the reaction was carried out at room temperature for 1 hour. The reaction was complete shown by TLC. The reaction solution was quenched with water and extracted with ethyl acetate, the organic phases were combined, washed with water, washed with saturated salt water, dried over anhydrous sodium sulfate and concentrated, and the crude priduct was purified by Prep-TLC to obtain the white solid title compound **13** (35 mg, yield 65%).

LC-MS: m/z=486.2[M+H]⁺

¹H NMR(400 MHz, DMSO-*d₆*) δ 10.55 (s, 1H), 8.32 (s, 1H), 8.23 (s, 1H), 8.11-8.09 (m, 1H), 7.79 (d, J = 7.6 Hz, 1H), 7.30 (s, 1H), 4.04 (s, 2H), 3.60-3.52 (m, 1H), 2.79 (s, 2H), 2.71 (d, J = 4.4 Hz, 3H), 2.63-2.59 (m, 1H), 1.90-187 (m, 1H), 1.82-1.73 (m, 6H), 1.28-1.23 (m, 3H), 1.20 (s, 6H), 1.09-1.06 (m, 1H). (98.68% purity by HPLC)

### Embodiment 14

### (2r,3aR,5s,6aS)-5-acetamido-N-(5-chloro-4-(5-cyano-2,2-dimethyl-2,3-dihydro-1H-pyrrolizin-7-yl )pyridin-2-yl)octahydropentalene-2-carboxamide (assumed) 14-1-1

### (2r,3aR,5r,6aS)-5-acetamido-N-(5-chloro-4-(5-cyano-2,2-dimethyl-2,3-dihydro-1H-pyrrolizin-7-yl )pyridin-2-yl)octahydropentalene-2-carboxamide (assumed) 14-1-2

### (2s,3aR,5s,6aS)-5-acetamido-N-(5-chloro-4-(5-cyano-2,2-dimethyl-2,3-dihydro-1H-pyrrolizin-7-yl )pyridin-2-yl)octahydropentalene-2-carboxamide (assumed) 14-2-1

### (2s,3aR,5r,6aS)-5-acetamido-N-(5-chloro-4-(5-cyano-2,2-dimethyl-2,3-dihydro-1H-pyrrolizin-7-yl )pyridin-2-yl)octahydropentalene-2-carboxamide (assumed) 14-2-2

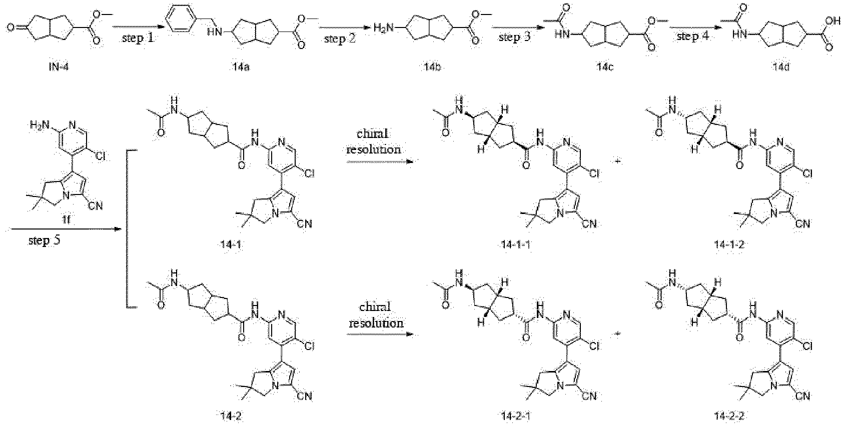

### Step 1: 5-(benzylamino)octahydropentalene-2-carboxylic acid methyl ester 14a

Intermediate **IN-4** (500 mg, 2.74 mmol) and benzylamine (353 mg, 3.29 mmol) were dispersed in 1,2-dichloroethane (15 mL), it was stirred at room temperature for 30 minutes, sodium triacetoxyborohydride (875 mg, 4.13 mmol) and acetic acid (165 mg, 2.75 mmol) were added, it was stirred at room temperature for 4 hours, and the reaction was complete shown by TLC. The reaction solution was quenched with water and extracted with dichloromethane, the organic phases were combined, washed with saturated salt water, dried over anhydrous sodium sulfate and concentrated, and the crude product was purified by silica gel column chromatography to obtain the white solid title compound **14a** (610 mg, yield 83%).

LC-MS: m/z=274.2 [M+H]⁺

### Step 2: 5-amino octahydropentalene-2-carboxylic acid methyl ester 14b

Compound **14a** (610 mg, 2.23 mmol) was dispersed in methanol (12 mL), palladium/carbon (200 mg, 10%) was added, and it was stirred at room temperature overnight under a hydrogen atmosphere. The complete reaction of the starting material was shown by TLC. The reaction solution was filtered with diatomite, and the filtrate was concentrated to obtain the oily liquid title compound **14b** (390 mg, crude product), which was directly used for the next step.

LC-MS: m/z=184.2 [M+H]⁺

### Step 3: 5-acetylamino octahydropentalene-2-carboxylic acid methyl ester 14c

Compound **14b** (390 mg, crude product) was dissolved in dichloromethane (10 mL), triethylamine (434 mg, 4.29 mmol) and acetic anhydride (326 mg, 3.19 mmol) were added, it was stirred at room temperature for 1 hour, and the complete reaction of the starting material was shown by TLC. The reaction solution was quenched with water and extracted with ethyl acetate, the organic phases were combined, washed with saturated salt water, dried over anhydrous sodium sulfate and concentrated, and the crude product was purified by silica gel column chromatography to obtain the yellow oil-like title compound **14c** (400 mg, two-step yield 80%).

### Step 4: 5-acetylamino octahydropentalene-2-carboxylic acid 14d

Compound **14c** (300 mg, 1.33 mmol) was dissolved in tetrahydrofuran (6 mL), a sodium hydroxide (0.8 mL, 4.0 mmol, 5N) aqueous solution was added, it was stirred overnight at room temperature, and the complete reaction of the starting material was shown by TLC. The reaction solution was diluted with water and extracted with ethyl acetate, the organic phase was discarded, the pH of the aqueous phase was adjusted to 2-3 with dilute hydrochloric acid (1N), it was extracted with ethyl acetate, and the organic phases were combined, washed with saturated salt water, dried over anhydrous sodium sulfate and concentrated to obtain the white solid title compound **14d** (220 mg, crude product), which was directly used for the next step.

### Step 5: (2r,3aR,5s,6aS)-5-acetamido-N-(5-chloro-4-(5-cyano-2,2-dimethyl-2,3-dihydro-1H-pyrrolizin-7-yl )pyridin-2-yl)octahydropentalene-2-carboxamide (assumed) 14-1-1 &

### (2r,3aR,5r,6aS)-5-acetamido-N-(5-chloro-4-(5-cyano-2,2-dimethyl-2,3-dihydro-1H-pyrrolizin-7-yl )pyridin-2-yl)octahydropentalene-2-carboxamide (assumed) 14-1-2 &

### (2s,3aR,5s,6aS)-5-acetamido-N-(5-chloro-4-(5-cyano-2,2-dimethyl-2,3-dihydro-1H-pyrrolizin-7-yl )pyridin-2-yl)octahydropentalene-2-carboxamide (assumed) 14-2-1 &

### (2s,3aR,5r,6aS)-5-acetamido-N-(5-chloro-4-(5-cyano-2,2-dimethyl-2,3-dihydro-1H-pyrrolizin-7-yl )pyridin-2-yl)octahydropentalene-2-carboxamide (assumed) 14-2-2

Compound **14d** (220 mg, crude product) and compound **1f** (250 mg, 0.87 mmol) were dispersed in ethyl acetate (10 mL), pyridine (0.4 mL, 4.97 mmol) and 1-propylphosphonic anhydride (2.22 g, 3.49 mmol, 50% ethyl acetate solution) were added in sequence at room temperature, the temperature was lifted to 85°C, it was stirred for 2 hours, and the complete reaction of the starting material was shown by TLC. The reaction solution was cooled to room temperature, quenched with water and extracted with ethyl acetate, the organic phases were combined, washed with saturated salt water, dried over anhydrous sodium sulfate and concentrated, and the crude product was purified by Prep-TLC to obtain the white solid low-polarity compound **14-1** (90 mg, yield 19%) and the high-polarity compound **14-2** (96 mg, yield 20%). Compound **14-1** was chirally resolved (NANOMICRO OD-5H, 30*250mm, 5um, 30mL/min, IPA:Hexane=20:80) to obtain Compound **14-1-1** (peak 1, RT 17.2 min) (60 mg, yield 67%) and Compound **14-1-2** (peak 2, RT 24.1 min) (15 mg, yield 16%). Compound **14-2** was chirally resolved (NANOMICRO OD-5H, 30*250mm, 5um, 30mL/min, IPA:Hexane=20:80) to obtain Compound **14-2-1** (peak 1, RT 30.0 min) (15 mg, yield 17%) and Compound **14-2-2** (peak 2, RT 42.0 min) (60 mg, yield 67%).

### Compound 14-1-1

LC-MS: m/z=480.2[M+H]⁺

¹H NMR(400 MHz, DMSO-*d₆*) δ 10.62 (s, 1H), 8.37 (s, 1H), 8.23 (s, 1H), 7.79 (d, J = 7.6 Hz, 1H), 7.42 (s, 1H), 4.09-4.04 (m, 1H), 3.95 (s, 2H), 3.05-3.01 (m, 1H), 2.87 (s, 2H), 2.40-2.34 (m, 2H), 2.14-2.03 (m, 4H), 1.76 (s, 3H), 1.54-1.47 (m, 2H), 1.24 (s, 6H), 1.18-1.13 (m, 2H). (98.65% purity by HPLC)

### Compound 14-2-2

LC-MS: m/z=480.2[M+H]⁺

¹H NMR(400 MHz, DMSO-*d₆*) δ 10.73 (s, 1H), 8.37 (s, 1H), 8.23 (s, 1H), 7.87 (d, J = 7.2 Hz, 1H), 7.42 (s, 1H), 3.95 (s, 2H), 3.77-3.71 (m, 1H), 3.12-3.06 (m, 1H), 2.86 (s, 2H), 2.46-2.42 (m, 2H), 2.10-2.03 (m, 2H), 1.80-1.75 (m, 5H), 1.68-1.63 (s, 2H), 1.23(s, 6H), 1.04-0.95 (m, 2H). (98.90% purity by HPLC)

Compound **14-1-2** and Compound **14-2-1** were of small amount and not sent for ¹H NMR.

### Embodiment 15

### (2s,3aR,5r,6aS)-N-(4-(5-carbamoyl-2,2-dimethyl-2,3-dihydro-1H-pyrrolizin-7-yl)-5-chloropyridin -2-yl)octahydropentalene-2,5-dicarboxamide (assumed) 15-1

### (2r,3aR,5s,6aS)-N-(4-(5-carbamoyl-2,2-dimethyl-2,3-dihydro-1H-pyrrolizin-7-yl)-5-chloropyridin -2-yl)octahydropentalene-2,5-dicarboxamide (assumed) 15-2

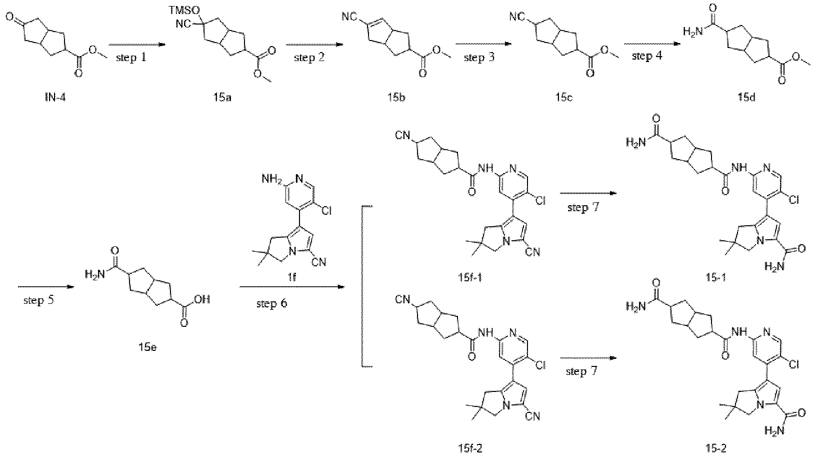

### Step 1: 5-cyano-5-((trimethylsilyl)oxy)octahydropentalene-2-carboxylic acid methyl ester 15a

Intermediate **IN-4** (600 mg, 3.29 mmol) was dispersed in chloroform (4 mL), zinc iodide (catalytic amount) and cyanotrimethylsilane (272 mg, 2.74 mmol) were added at room temperature, it was stirred overnight at room temperature, and the exhaustion of the starting material was shown by TLC. The reaction solution was poured into a saturated ammonium chloride aqueous solution for quenching and extracted with dichloromethane, the organic phases were combined, washed with saturated salt water, dried over anhydrous sodium sulfate and concentrated, and the crude product was purified by silica gel column chromatography to obtain the colorless oil title compound **15a** (820 mg, yield 89%).

¹H NMR (400 MHz, CDCl3) δ 3.67-3.66 (m, 3H), 2.91-2.63 (m, 3H), 2.48-2.31 (m, 2H), 2.23-2.16 (m, 1H), 1.97-1.85 (m, 2H), 1.79-1.72 (m, 2H), 1.63-1.52 (m, 1H), 0.24-0.22 (m, 9H).

### Step 2: 5-cyano-1,2,3,3a,4,6a-hexahydropentalene-2-carboxylic acid methyl ester 15b

Compound **15a** (820 mg, 2.91 mmol) was dispersed in pyridine (4 mL), phosphorus oxychloride (0.6 mL, 6.44 mmol) was added at room temperature, the temperature was lifted to 110°C, it was refluxed for 3 hours, and the exhaustion of the starting material was shown by TLC. The reaction solution was cooled to room temperature, quenched by slowly adding water and extracted with ethyl acetate, the organic phases were combined, washed with saturated salt water, dried over anhydrous sodium sulfate and concentrated to obtain the transparent oily liquid title compound **15b** (400 mg, yield 72%).

¹H NMR (400 MHz, CDCl3) δ 6.50-6.40 (m, 1H), 3.67-3.66 (m, 3H), 3.48-3.32 (m, 1H), 3.00-2.66 (m, 3H), 2.42-1.98 (m, 3H), 1.87-1.54 (m, 2H).

### Step 3: 5-cyano octahydropentalene-2-carboxylic acid methyl ester 15c

Compound **15b** (100 mg, 0.52 mmol) was dispersed in methanol (2 mL), palladium/carbon (40 mg, 10%) was added, it was stirred at room temperature for 2 hours, and the reaction was complete shown by TLC. The reaction solution was filtered with diatomite, the filtrate was concentrated, and the crude product was purified by silica gel column chromatography to obtain the transparent oily liquid title compound **15c** (30 mg, yield 30%).

¹H NMR (400 MHz, CDCl3) δ 3.67-3.66 (m, 3H), 2.87-2.72 (m, 2H), 2.61-2.50 (m, 2H), 2.39-2.16 (m, 4H), 1.79-1.61 (m, 4H).

### Step 4: 5-carbamoyl octahydropentalene-2-carboxylic acid methyl ester 15d

Compound **15c** (100 mg, 0.52 mmol) was dispersed in dimethyl sulfoxide (0.5 mL), potassium carbonate (145 mg, 1.05 mmol) and hydrogen peroxide (1 mL, 30%) were added in sequence, and it was stirred at room temperature for 30 minutes. The exhaustion of the starting material was shown by TLC. The reaction solution was quenched with water and extracted with ethyl acetate, and the organic phases were combined, washed with saturated salt water, dried over anhydrous sodium sulfate and concentrated to obtain the title compound **15d** (95 mg, crude product), which was directly used for the next step.

LC-MS: m/z=212.2 [M+H]⁺

### Step 5: 5-carbamoyl octahydropentalene-2-carboxylic acid 15e

Compound **15d** (150 mg, crude product) was dispersed in tetrahydrofuran (3 mL), sodium hydroxide (57 mg, 1.42 mmol) was added, it was stirred overnight at room temperature, and the reaction was complete shown by TLC. The reaction solution was added with water and extracted with ethyl acetate, the organic phases were discarded, the aqueous phase was adjusted to pH=3 with dilute hydrochloric acid (1N) and extracted with ethyl acetate, the organic phases were combined, washed with saturated salt water, dried over anhydrous sodium sulfate and concentrated to obtain the title compound **15e** (120 mg, crude product),which was directly used for the next step.

LC-MS: m/z=198.2 [M+H]⁺

### Step 6: N-(5-chloro-4-(5-cyano-2,2-dimethyl-2,3-dihydro-1H-pyrrolizin-7-yl)pyridin-2-yl)-5-cyano octahydropentalene-2-carboxamide 15f-1,2

Compound **15e** (120 mg, crude product) and Compound **If** (89 mg, 0.31 mmol) were dispersed in ethyl acetate (10 mL), pyridine (0.4 mL, 4.97 mmol) and 1-propylphosphonic anhydride (2.22 g, 3.49 mmol, 50% ethyl acetate solution) were added in sequence at room temperature, the temperature was lifted to 85°C, it was stirred for 2 hours, and the complete reaction of the starting material was shown by TLC. The reaction solution was added with water and extracted with ethyl acetate,the organic phases were combined, washed with saturated salt water, dried over anhydrous sodium sulfate and concentrated, and the crude product was purified by Prep-TLC (petroleum ether/ethyl acetate=2:1) to obtain the white solid low-polarity title compound **15f-1** (30 mg, yield 21%) and the high-polarity title compound **15f-2** (60 mg, yield 42%).

### Compound 15f-1

LC-MS: m/z=448.3 [M+H]⁺

¹H NMR (400 MHz, DMSO-*d₆*) δ 10.60 (s, 1H), 8.38 (s, 1H), 8.22 (s, 1H), 7.42 (s, 1H), 3.95 (s, 2H), 3.13-3.06 (m, 1H), 2.86 (s, 2H), 2.74-2.67 (m, 1H), 2.57-2.53 (m, 2H), 2.35-2.29 (m, 2H), 1.71-1.69 (m, 3H), 140-1.32 (m, 3H), 1.26 (s, 6H). (92.51% purity by HPLC)

### Compound 15f-2

LC-MS: m/z=448.3 [M+H]⁺

¹H NMR (400 MHz, DMSO-*d₆*) δ 10.59 (s, 1H), 8.36 (s, 1H), 8.22 (s, 1H), 7.41 (s, 1H), 3.94 (s, 2H), 3.03-3.00 (m, 2H), 2.86 (s, 2H), 2.49-2.48 (m, 2H), 2.21-2.18 (m, 2H), 2.10-2.09 (m, 2H), 1.65-1.60 (m, 4H), 1.26 (s, 6H). (92.98% purity by HPLC)

### Step 7: (2s,3aR,5r,6aS)-N-(4-(5-carbamoyl-2,2-dimethyl-2,3-dihydro-1H-pyrrolizin-7-yl)-5-chloropyridin -2-yl)octahydropentalene-2,5-dicarboxamide (assumed) 15-1 &

### (2r,3aR,5s,6aS)-N-(4-(5-carbamoyl-2,2-dimethyl-2,3-dihydro-1H-pyrrolizin-7-yl)-5-chloropyridin -2-yl)octahydropentalene-2,5-dicarboxamide (assumed) 15-2

Compound **15f-1** (20 mg, 0.045 mmol) was dispersed in dimethyl sulfoxide (0.5 mL), potassium carbonate (25 mg, 0.18 mmol) and hydrogen peroxide (0.75 mL, 30%) were added, it was stirred at room temperature for two days, and the reaction was complete shown by TLC. The reaction solution was quenched with water and extracted with ethyl acetate, the organic phases were combined, washed with saturated salt water, dried over anhydrous sodium sulfate and concentrated, and the crude product was purified by Prep-TLC to obtain the white solid title compound **15-1** (3.5 mg, yield 16%).

LC-MS: m/z=484.3[M+H]⁺

¹H NMR (400 MHz, DMSO-*d₆*) δ 10.61 (s, 1H), 8.31 (s, 1H), 8.24 (s, 1H), 7.62 (s, 1H), 7.34 (s, 1H), 7.23 (s, 1H), 6.96 (s, 1H), 6.72 (s, 1H), 4.03 (s, 2H), 3.14-3.05 (m, 1H), 2.77 (s, 2H), 2.39-2.33 (m, 1H), 2.01-1.97 (m, 3H), 1.74-1.65 (m, 4H), 1.35-1.30 (m, 3H), 1.20 (s, 6H). (94.53% purity by HPLC)

Compound **15f-2** (40 mg, 0.09 mmol) was dispersed in dimethyl sulfoxide (1 mL), potassium carbonate (50 mg, 0.36 mmol) and hydrogen peroxide (1.5 mL, 30%) were added, it was stirred for two days at room temperature, and the reaction was complete shown by TLC. The reaction solution was quenched with water and extracted with ethyl acetate, the organic phases were combined, washed with saturated salt water, dried over anhydrous sodium sulfate and concentrated, and the crude product was purified by Prep-TLC to obtain the white solid title compound **15-2** (15 mg, yield 34%).

LC-MS: m/z=484.3[M+H]⁺

¹H NMR (400 MHz, DMSO-*d₆*) δ 10.50 (s, 1H), 8.31 (s, 1H), 8.25 (s, 1H), 7.64 (s, 1H), 7.35 (s, 1H), 7.21 (s, 1H), 6.96 (s, 1H), 6.72 (s, 1H), 4.03 (s, 2H), 3.07-3.02 (m, 1H), 2.79 (s, 2H), 2.69-2.64 (m, 1H), 2.45-2.42 (m, 2H), 2.09-2.02 (m, 2H), 1.99-1.93 (m, 2H), 1.56-1.43 (m, 4H), 1.20 (s, 6H). (98.19% purity by HPLC)

### Embodiment 16

### (1S,3R)-3-acetamido-N-(5-chloro-4-(7-cyano-2,2-dimethyl-2,3-dihydro-1H-pyrrolizin-5-yl) pyridin-2-yl)cyclohexane-1-carboxamide 16

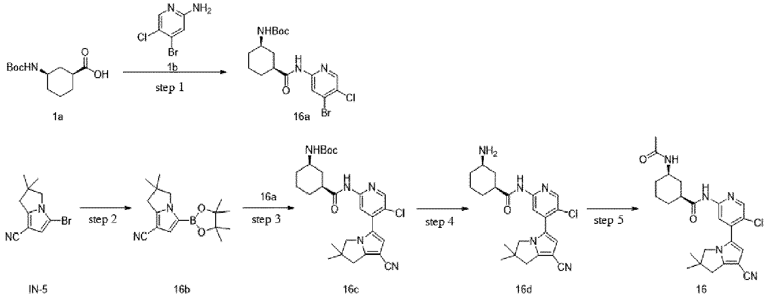

### Step 1: ((1R,3S)-3-((4-bromo-5-chloropyridin-2-yl)carbamoyl)cyclohexyl)carbamic acid tert-butyl ester 16a

4-bromo-5-chloropyridin-2-amine **1b** (200 mg, 0.96 mmol), Compound **1a** (246 mg, crude product) and pyridine (304 mg, 3.84 mmol) were dissolved in ethyl acetate (8 mL), 1-propylphosphonic anhydride (1.2 g, 1.88 mmol, 50% ethyl acetate solution) was added, the reaction was carried out at room temperature for 4 hours, most of the starting material was remained shown by TLC, the temperature was lifted to 60°C for reaction overnight, and most of the starting material was still remained shown by TLC. The reaction solution was cooled to room temperature, added with water, added with a saturated sodium carbonate solution to adjust the pH to 9 and extracted with ethyl acetate, the organic phases were combined, washed with saturated salt water, dried over anhydrous sodium sulfate and concentrated, and the crude product was chromatographed on silica gel column to obtain the white solid title compound **16a** (114 mg, yield 26%).

LC-MS: m/z=431.9[M+H]⁺

### Step 2: 2,2-dimethyl-5-(4,4,5,5-tetramethyl-1,3,2-dioxaborolan-2-yl)-2,3-dihydro-1H-pyrrolizine-7-carbo nitrile 16b

Compound **IN-5** (112 mg, 0.47 mmol) was dissolved in 1,4-dioxane (10 mL), bis(pinacolato)diboron (238 mg, 0.94 mmol), Pd(dppf)Cl₂ dichloromethane complex (19 mg, 0.02 mmol) and potassium acetate (138 mg, 1.41 mmol) were added in sequence at room temperature, the temperature was lifted to 100°C for overnight reaction under nitrogen protection, and the reaction was complete shown by TLC. The reaction solution was cooled to room temperature and filtered, the filtrate was concentrated, and the crude product was purified by Prep-TLC to obtain the colorless liquid title compound **16b** (118 mg, yield 88%).

LC-MS: m/z=287.2[M+H]⁺

### Step 3: ((1R,3S)-3-((5--chloro-4-(7-cyano-2,2-dimethyl-2,3-dihydro-1H-pyrrolizin-5-yl)pyridin-2-yl)carba moyl)cyclohexyl)carbamic acid tert-butyl ester 16c

Compound **16a** (59 mg, 0.21 mmol) was dissolved in 1,4-dioxane (3 mL), Compound **16b** (89 mg, 0.20 mmol), Tetrakis(triphenylphosphine)palladium (12 mg, 0.01 mmol), potassium phosphate (87 mg, 0.41 mmol) and water (0.5 mL) were added in sequence at room temperature, under nitrogen protection, the temperature was lifted to 95°C for reaction overnight, and a small amount of the starting material was remained shown by LC-MS. The reaction solution was cooled to room temperature and filtered, the filtrate was concentrated, and the crude product was purified by Prep-TLC to obtain the white solid title compound **16c** (24 mg, yield 23.4%).

LC-MS: m/z=512.3[M+H]⁺

### Step 4: (1S,3R)-3-amino-N-(5-chloro-4-(7-cyano-2,2-dimethyl-2,3-dihydro-1H-pyrrolizin-5-yl)pyridin-2-yl)cyclohexane-1-carboxamide 16d

Compound **16c** (24 mg, 0.05 mmol) was dissolved in dichloromethane (6 mL), trifluoroacetic acid (2 mL) was added, the reaction was carried out for 1 hour at room temperature, and the reaction was complete shown by TLC. The reaction solution was concentrated to obtain the title compound **16d** (19 mg, crude product), which was directly used for the next step.

### Step 5: (1S,3R)-3-acetamido-N-(5-chloro-4-(7-cyano-2,2-dimethyl-2,3-dihydro-1H-pyrrolizin-5-yl)pyridin -2-yl)cyclohexane-1-carboxamide 16

Compound **16d** (19 mg, crude product) was dissolved in dichloromethane (5 mL), triethylamine (14 mg, 0.14 mmol) and acetic anhydride (7 mg, 0.07 mmol) were added, the reaction was carried out overnight at room temperature, and the reaction was complete shown by TLC. The reaction solution was concentrated, and the crude product was purified by Prep-TLC to obtain the light yellow solid title compound **16** (16 mg, two-step yield 75%).

LC-MS: m/z=454.3[M+H]⁺

¹H NMR (400 MHz, CD3OD) δ 8.25 (s, 1H), 8.13 (s, 1H), 6.80 (s, 1H), 3.83 (s, 2H), 3.63-3.62 (m, 1H), 2.78 (s, 2H), 2.50-2.47 (m, 1H), 1.96-1.80 (m, 7H), 1.37-1.30 (m, 4H), 1.07 (s, 6H). (99.15% purity by HPLC)

### Embodiment 17

### 5-(2-((1S,3R)-3-acetylaminocyclohexane-1-carboxamido)-5-chloropyridin-4-yl)-2,2-dimethyl-2,3-dihydro-1H-pyrrolizine-7-carboxamide 17

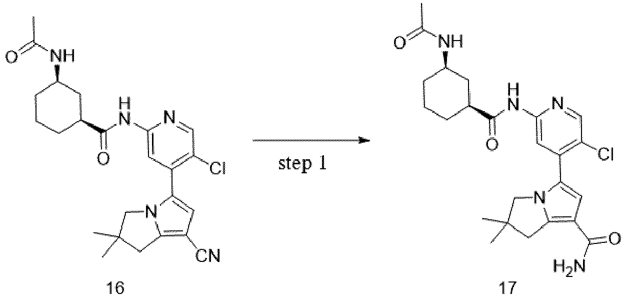

Compound **16** (70 mg, 0.15 mmol) was dispersed in dimethyl sulfoxide (1 mL), hydrogen peroxide (1 mL, 30%) and potassium carbonate (47 mg, 0.34 mmol) were added in sequence, it was stirred at room temperature for 1 hour, and the complete reaction of the starting material was shown by TLC. The reaction solution was quenched with water and extracted with ethyl acetate, the organic phases were combined, washed with saturated salt water, dried over anhydrous sodium sulfate and concentrated, and the crude product was purified by Prep-TLC (dichloromethane/methanol 10:1) and triturated with dichloromethane to obtain the white solid title compound **17** (55 mg, yield 78%).

LC-MS: m/z= 472.2[M+H]⁺

¹H NMR (400 MHz, DMSO-*d₆*) δ 10.63 (s, 1H), 8.39 (s, 1H), 8.21 (s, 1H), 7.77 (d, J = 8.0 Hz, 1H), 7.25 (br, 1H), 7.08 (s, 1H), 6.75 (br, 1H), 3.81 (s, 2H), 3.61-3.52 (m, 1H), 2.88 (s, 2H), 2.65-2.56 (m, 1H), 1.90-1.87 (m, 1H), 1.82-1.70 (m, 6H), 1.29-1.27 (m, 2H), 1.19 (s, 7H), 1.12-1.03 (m, 1H). (98.91% purity by HPLC)

### Embodiment 18

### (5S,7S)-2-acetyl-N-(5-chloro-4-(5,5-dimethyl-5,6-dihydro-4H-pyrrolo[1,2-b]pyrazol-3-yl)pyridin-2-yl)-2-azaspiro[4.4]nonane-7-carboxamide (assumed) 18-1

### (5R,7R)-2-acetyl-N-(5-chloro-4-(5,5-dimethyl-5,6-dihydro-4H-pyrrolo[1,2-b]pyrazol-3-yl)pyridin-2-yl)-2-azaspiro[4.4]nonane-7-carboxamide (assumed) 18-2

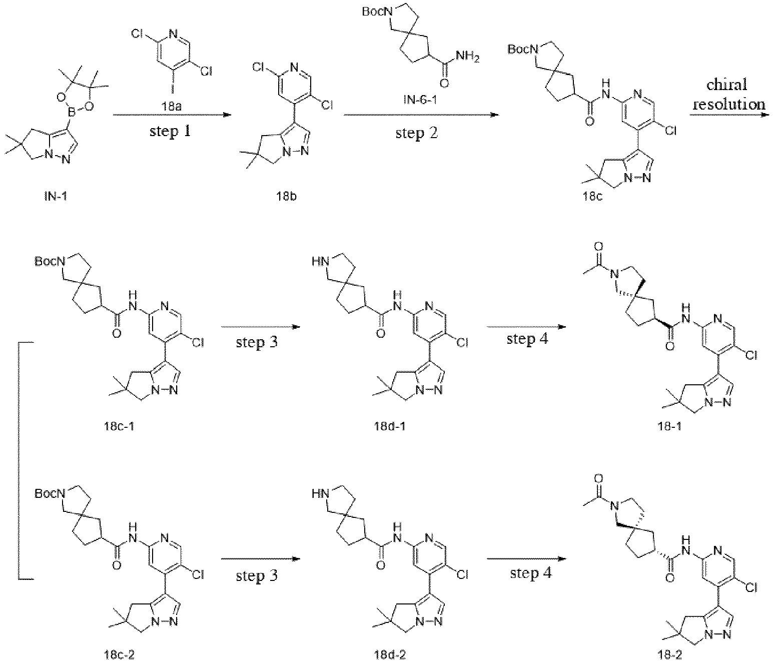

### Step1: 3-(2,5-dichloropyridin-4-yl)-5,5-dimethyl-5,6-dihydro-4H-pyrrolo[1,2-b]pyrazole 18b

Intermediate **IN-1** (230 mg, 0.88 mmol) and 2,5-dichloro-4-iodopyridine **18a** (200 mg, 0.73 mmol) were dissolved in 1,4-dioxane (6 mL) and water (2 mL), potassium carbonate (202 mg, 1.46 mmol) and Pd(dppf)Cl₂ dichloromethane complex (50 mg, 0.06 mmol) were added, it was replaced with nitrogen three times, the temperature was lifted to 85°C and it was stirred for 3 hours. The reaction was complete shown by TLC. The reaction solution was cooled to room temperature, quenched with water and extracted with ethyl acetate, the organic phases were combined, washed with saturated salt water, dried over anhydrous sodium sulfate and concentrated, and the crude product was purified by silica gel column chromatography to obtain the light yellow solid title compound **18b** (108 mg, yield 52%).

### Step 2: 7-((5-chloro-4-(5,5-dimethyl-5,6-dihydro-4H-pyrrolo[1,2-b]pyrazol-3-yl)pyridin-2-yl)carbamoyl)-2-azaspiro[4.4]nonane-2-carboxylic acid tert-butyl ester 18c-1,2

Intermediate **IN-6-1** (133 mg, 0.50 mmol) and Compound 18b (158 mg, 0.56 mmol) were dissolved in 1,4-dioxane (5 mL), cesium carbonate (498 mg, 1.53 mmol), tetrakis(triphenylphosphine)palladium (118 mg, 0.10 mmol) and Xantphos (31 mg, 0.05 mmol) were added, it was replaced with nitrogen three times, the temperature was lifted to 110°C, and it was stirred for 1 hour. The reaction was complete shown by TLC. The reaction solution was cooled to room temperature, quenched with water and extracted with ethyl acetate, the organic phases were combined, washed with saturated salt water, dried over anhydrous sodium sulfate and concentrated, and the crude product was purified by silica gel column chromatography to obtain the light yellow solid compound **18c** (176 mg, yield 68%). Chiral resolution (DAICEL AD-H, 30*250mm, 5um, 30mL/min, IPA:Hexane=30:70) to obtain the title compound **18c-1** (Peak 1, RT 16.0 min) (46 mg, yield 26%) and the title compound **18c-2** (Peak 2, RT 22.3 min) (54 mg, yield 31%).

### Step 3: N-(5-chloro-4-(5,5-dimethyl-5,6-dihydro-4H-pyrrolo[1,2-b]pyrazol-3-yl)pyridin-2-yl)-2-azaspiro[ 4.4]nonane-7-carboxamide 18d-1,2

Compound **18c-1** (46 mg, 0.09 mmol) was dissolved in dichloromethane (1 mL), trifluoroacetic acid (0.5 mL) was added, it was stirred at room temperature for 30 minutes, and the reaction was complete shown by TLC. The reaction solution was concentrated, adjusted to be pH alkaline with a saturated sodium bicarbonate aqueous solution and extracted with ethyl acetate, the organic phases were combined, washed with saturated salt water, dried over anhydrous sodium sulfate and concentrated to obtain the title compound **18d-1** (crude product), which was directly used for the next step.

Compound **18c-2** (54 mg, 0.10 mmol) was dissolved in dichloromethane (1 mL), trifluoroacetic acid (0.5 mL) was added, it was stirred at room temperature for 30 minutes, and the reaction was complete shown by TLC. The reaction solution was concentrated, adjusted to be pH alkaline with a saturated sodium bicarbonate aqueous solution and extracted with ethyl acetate, the organic phases were combined, washed with saturated salt water, dried over anhydrous sodium sulfate and concentrated to obtain the title compound **18d-2** (crude product), which was directly used for the next step.

### Step 4: (5S,7S)-2-acetyl-N-(5-chloro-4-(5,5-dimethyl-5,6-dihydro-4H-pyrrolo[1,2-b]pyrazol-3-yl)pyridin-2-yl)-2-azaspiro[4.4]nonane-7-carboxamide (assumed) 18-1 &

### (5R,7R)-2-acetyl-N-(5-chloro-4-(5,5-dimethyl-5,6-dihydro-4H-pyrrolo[1,2-b]pyrazol-3-yl)pyridin-2-yl)-2-azaspiro[4.4]nonane-7-carboxamide (assumed) 18-2

Compound **18d-1** (37 mg, crude product) was dissolved in dichloromethane (1 mL), triethylamine (22 mg, 0.22 mmol) and acetic anhydride (17 mg, 0.17 mmol) were added, it was stirred at room temperature for 30 minutes, and the reaction was complete shown by TLC. The reaction solution was quenched with water and extracted with ethyl acetate, the organic phases were combined, washed with saturated salt water, dried over anhydrous sodium sulfate and concentrated, and the crude product was purified by Prep-TLC to obtain the white solid title compound **18-1** (33 mg, two-step yield 80%)

LC-MS: m/z=456.3[M+H]⁺

¹H NMR (400 MHz, CDCl3) δ 9.29 (br, 0.5H), 8.68 (br, 0.5H), 8.36-8.31 (m, 1H), 8.21-8.20 (m, 1H), 8.16-8.11 (m, 1H), 3.96 (d, J = 3.2 Hz, 2H), 3.52 (q, J = 7.2 Hz, 2H), 3.35-3.30 (m, 2H), 3.00-2.97 (m, 3H), 2.11-1.70 (m, 11H), 1.35 (d, J = 4.0 Hz, 6H). (98.58% purity by HPLC) Compound **18d-2** (43 mg, crude product) was dissolved in dichloromethane (1 mL), triethylamine (30 mg, 0.30 mmol) and acetic anhydride (25 mg, 0.24 mmol) were added, it was stirred at room temperature for 30 minutes, and the reaction was complete shown by TLC. The reaction solution was quenched with water and extracted with ethyl acetate, the organic phases were combined, washed with saturated salt water, dried over anhydrous sodium sulfate and concentrated, and the crude product was purified by Prep-TLC to obtain the white solid title compound **18-2** (36 mg, two-step yield 72%).

LC-MS: m/z=456.3[M+H]⁺

¹H NMR (400 MHz, CDCl3) δ 8.86 (br, 0.5H), 8.55 (br, 0.5H), 8.32-8.28 (m, 1H), 8.21-8.20 (m, 1H), 8.13-8.09 (m, 1H), 3.96 (d, J = 2.0 Hz, 2H), 3.51 (q, J = 7.2 Hz, 2H), 3.34-3.29 (m, 2H), 3.00-2.96 (m, 3H), 2.10-1.71 (m, 11H), 1.34 (d, J = 3.2 Hz, 6H). (99.24% purity by HPLC)

### Embodiment 19

### (5S,7R)-2-acetyl-N-(5-chloro-4-(5,5-dimethyl-5,6-dihydro-4H-pyrrolo[1,2-b]pyrazol-3-yl)pyridin-2-yl)-2-azaspiro[4.4]nonane-7-carboxamide (assumed) 19-1

### (5R,7S)-2-acetyl-N-(5-chloro-4-(5,5-dimethyl-5,6-dihydro-4H-pyrrolo[1,2-b]pyrazol-3-yl)pyridin-2-yl)-2-azaspiro[4.4]nonane-7-carboxamide (assumed) 19-2

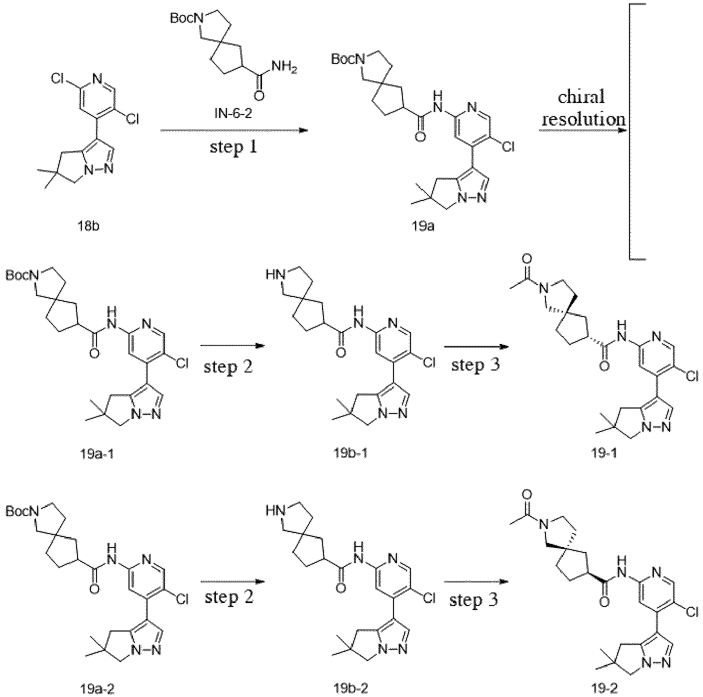

### Step 1: 7-((5-chloro-4-(5,5-dimethyl-5,6-dihydro-4H-pyrrolo[1,2-b]pyrazol-3-yl)pyridin-2-yl)carbamoyl)-2-azaspiro[4.4]nonane-2-carboxylic acid tert-butyl ester 19a-1,2

Compound **18b** (70 mg, 0.26 mmol) and Intermediate **IN-6-2** (83 mg, 0.29 mmol) were dissolved in 1,4-dioxane (2 mL), cesium carbonate (262 mg, 0.80 mmol), tetrakis(triphenylphosphine)palladium (62 mg, 0.05 mmol) and Xantphos (16 mg, 0.03 mmol) were added, it was replaced with nitrogen three times, the temperature was lifted to 110°C, and it was stirred for 1 hour. The reaction was complete shown by TLC. The reaction solution was cooled to room temperature, quenched with water and extracted with ethyl acetate, the organic phases were combined, washed with saturated salt water, dried over anhydrous sodium sulfate and concentrated, and the crude product was purified by silica gel column chromatography to obtain the light yellow solid compound **19a** (100 mg, yield 78%). Chiral resolution (DAICEL AD-H, 30*250mm, Sum, 30mL/min, IPA:Hexane=30:70) to obtain the title compound **19a-1** (peak 1, RT 26.0 min) (22 mg, yield 22%) and the title compound **19a-2** (peak 2, RT 32.0 min) (30 mg, yield 30%).

LC-MS: m/z=514.3[M+H]⁺

### Step 2: N-(5-chloro-4-(5,5-dimethyl-5,6-dihydro-4H-pyrrolo[1,2-b]pyrazol-3-yl)pyridin-2-yl)-2-azaspiro[ 4.4]nonane-7-carboxamide 19b-1,2

Compound **19a-1** (22 mg, 0.04 mmol) was dissolved in dichloromethane (1 mL), trifluoroacetic acid (0.5 mL) was added, it was stirred at room temperature for 30 minutes, and the reaction was complete shown by TLC. The reaction solution was concentrated, adjusted to be pH alkaline with a saturated sodium bicarbonate aqueous solution and extracted with ethyl acetate, and the organic phases were combined, washed with saturated salt water, dried over anhydrous sodium sulfate and concentrated to obtain the title compound **19b-1** (crude product), which was directly used for the next step.

Compound **19a-2** (32 mg, 0.06 mmol) was dissolved in dichloromethane (1 mL), trifluoroacetic acid (0.5 mL) was added, it was stirred at room temperature for 30 minutes, and the reaction was complete shown by TLC. The reaction solution was concentrated, adjusted to be pH alkaline with a saturated sodium bicarbonate aqueous solution and extracted with ethyl acetate, the organic phases were combined, washed with saturated salt water, dried over anhydrous sodium sulfate and concentrated to obtain the title compound **19b-2** (crude product), which was directly used for the next step.

LC-MS: m/z=414.2[M+H]⁺

### Step 3: (5S,7R)-2-acetyl-N-(5-chloro-4-(5,5-dimethyl-5,6-dihydro-4H-pyrrolo[1,2-b]pyrazol-3-yl)pyridin-2-yl)-2-azaspiro[4.4]nonane-7-carboxamide (assumed) 19-1 &

### (5R,7S)-2-acetyl-N-(5-chloro-4-(5,5-dimethyl-5,6-dihydro-4H-pyrrolo[1,2-b]pyrazol-3-yl)pyridin-2-yl)-2-azaspiro[4.4]nonane-7-carboxamide (assumed) 19-2

Compound **19b-1** (18 mg, crude product) was dissolved in dichloromethane (1 mL), triethylamine (11 mg, 0.11 mmol) and acetic anhydride (9 mg, 0.09 mmol) were added, it was stirred at room temperature for 30 minutes, and the reaction was complete shown by TLC. The reaction solution was quenched with water and extracted with ethyl acetate, the organic phases were combined, washed with saturated salt water, dried over anhydrous sodium sulfate and concentrated, and the crude product was purified by Prep-TLC to obtain the white solid title compound **19-1** (11 mg, two-step yield 60%).

LC-MS: m/z=456.3[M+H]⁺

¹H NMR (400 MHz, CDCl3) δ 9.05 (br, 0.5H), 8.61 (br, 0.5H), 8.34-8.31 (m, 1H), 8.21-8.20 (m, 1H), 8.18-8.12 (m, 1H), 3.96 (s, 2H), 3.54-3.32 (m, 4H), 3.01-2.97 (m, 3H), 2.16-1.63 (m, 11H), 1.35 (d, J = 6.8 Hz, 6H). (99.44% purity by HPLC)

Compound **19b-2** (26 mg, crude product) was dissolved in dichloromethane (1 mL), triethylamine (15 mg, 0.15 mmol) and acetic anhydride (12 mg, 0.12 mmol) were added, it was stirred at room temperature for 30 minutes, and the reaction was complete shown by TLC. The reaction solution was quenched with water and extracted with ethyl acetate, the organic phases were combined, washed with saturated salt water, dried over anhydrous sodium sulfate and concentrated, and the crude product was purified by Prep-TLC to obtain the white solid title compound 19-2 (16 mg, two-step yield 58%).

LC-MS: m/z=456.3[M+H]⁺

¹H NMR (400 MHz, CDCl3) δ 9.22 (br, 0.5H), 8.71 (br, 0.5H), 8.38-8.33 (m, 1H), 8.21-8.13 (m, 2H), 3.96 (s, 2H), 3.55-3.32 (m, 4H), 3.03-2.98 (m, 3H), 2.05-1.63 (m, 11H), 1.35 (d, J = 7.6 Hz, 6H). (98.19% purity by HPLC) (99.74% purity by HPLC)

### Embodiment 20

### (1S,3S)-N-(5-chloro-4-(5,5-dimethyl-5,6-dihydro-4H-pyrrolo[1,2-b]pyrazol-3-yl)pyridin-2-yl)-3-( pyridin-3-yl)cyclohexane-1-carboxamide (assumed) 20-1

### (1R,3S)-N-(5-chloro-4-(5,5-dimethyl-5,6-dihydro-4H-pyrrolo[1,2-b]pyrazol-3-yl)pyridin-2-yl)-3-( pyridin-3-yl)cyclohexane-1-carboxamide (assumed) 20-2

### (1S,3R)-N-(5-chloro-4-(5,5-dimethyl-5,6-dihydro-4H-pyrrolo[1,2-b]pyrazol-3-yl)pyridin-2-yl)-3-( pyridin-3-yl)cyclohexane-1-carboxamide (assumed) 20-3

### (1R,3R)-N-(5-chloro-4-(5,5-dimethyl-5,6-dihydro-4H-pyrrolo[1,2-b]pyrazol-3-yl)pyridin-2-yl)-3-( pyridin-3-yl)cyclohexane-1-carboxamide (assumed) 20-4

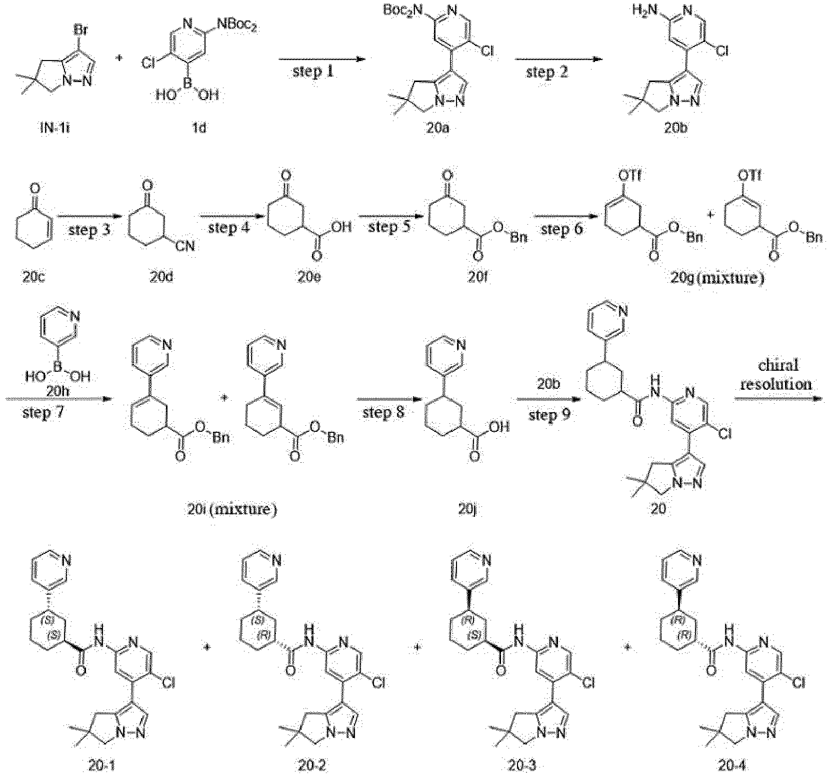

### Step 1: (5-chloro-4-(5,5-dimethyl-5,6-dihydro-4H-pyrrolo[1,2-b]pyrazol-3-yl)pyridin-2-yl)iminodicarbox ylic acid di-tert-butyl ester 20a

Compound **IN-1i** (215 mg, 1.0 mmol) and Compound **1d** (746 mg, 2.0 mmol) were dispersed in 1,4-dioxane (15 mL) and water (5 mL), sodium carbonate (212 mg, 2.0 mmol) and Pd(dppf)Cl₂ (catalytic amount) were added in sequence at room temperature, after the addition, the temperature was lifted to 100°C, it was stirred for 1 hour under nitrogen protection, and the complete reaction of the starting material was shown by TLC. The reaction solution was cooled to room temperature and filtered, the filter cake was washed multiple times with ethyl acetate, the filtrate was diluted with water and extracted with ethyl acetate, the organic phases were combined, washed with saturated salt water, dried over anhydrous sodium sulfate and concentrated, and the crude product was purified by silica gel column chromatography to obtain the white solid title compound **20a** (398 mg, yield 86%).

### Step 2: 5-chloro-4-(5,5-dimethyl-5,6-dihydro-4H-pyrrolo[1,2-b]pyrazol-3-yl)pyridin-2-amine 20b

Compound **20a** (398 mg, 0.86 mmol) was dissolved in dichloromethane (6 mL), trifluoroacetic acid (2mL) was added, it was stirred at room temperature for 1 hour, and the complete reaction of the starting material was shown by TLC. The reaction solution was concentrated, added with a saturated sodium bicarbonate aqueous solution, extracted with ethyl acetate, washed with saturated salt water, dried over anhydrous sodium sulfate, and concentrated to obtain the yellow solid title compound **20b** (215 mg, crude product), which was directly used for the next step.

### Step 3: 3-oxocyclohexane-1-carbonitrile 20d

2-cyclohexen-1-one **20c** (80.0 g, 365 mmol) was dissolved in methanol (800 mL), cyanotrimethylsilane (99.2 g, 1.0 mol) and tetrabutylammonium fluoride (261 g, 1.0 mol) were added at room temperature, and the temperature was lifted to 60°C for reaction for 16 hours. The complete reaction of the starting material was shown by TLC. The reaction solution was cooled to room temperature, quenched with water and extracted with ethyl acetate, the organic phases were combined, washed with saturated salt water, dried over anhydrous sodium sulfate and concentrated, and the crude product was separated and purified by silica gel column chromatography to obtain the colorless liquid title compound **20d** (19.0 g, yield 42%).

LC-MS: m/z=124.2[M+H]⁺

### Step 4: 3-oxocyclohexane-1-carboxylic acid 20e

Compound **20d** (14.0 g, 113.7 mmol) was dissolved in 1,-4-dioxane (140 mL), concentrated hydrochloric acid (70 mL) was added at room temperature,and the temperature was lifted to 80°C for reaction for16 hours. The complete reaction of the starting material was shown by TLC. The reaction solution was cooled to room temperature, quenched with water and extracted with ethyl acetate, and the organic phases were combined, washed with saturated salt water, dried over anhydrous sodium sulfate and concentrated to obtain the title compound **20e** (15.0 g, crude product), which was directly used for the next step.

LC-MS: m/z=141.2[M-H]⁻

### Step 5: 3-oxocyclohexane-1-carboxylic acid benzyl ester 20f

Compound **20e** (15.0 g, crude product) and benzyl bromide (19.8 g, 115.8 mmol) were dissolved in acetonitrile (300 mL), 1,8-diazabicyclo undec-7-ene (16.8 g, 110.4 mmol) was added, and the reaction was carried out at room temperature for 16 hours. the complete reaction of the starting material was shown by TLC. The reaction solution was quenched with water and extracted with ethyl acetate, the organic phases were combined, washed with saturated salt water, dried over anhydrous sodium sulfate and concentrated, and the crude product was separated and purified by Prep-HPLC to obtain the title compound **20f** (8.0 g, two-step yield 33%).

LC-MS: m/z=233.2[M+H]⁺

¹H NMR (400 MHz, CDCl₃) δ 7.39-7.31 (m, 5H), 5.14 (s, 2H), 2.89-2.81 (m, 1H), 2.57 (d, J = 8.0 Hz, 2H), 2.41-2.27 (m, 2H), 2.17-2.02 (m, 2H), 1.90-1.81 (m, 1H), 1.77-1.66 (m, 1H).

### Step 6: mixture of 3-(((trifluoromethyl)sulfonyl)oxy)benzylcyclohex-3-ene-1-carboxylic acid benzyl ester and 3-(((trifluoromethyl)sulfonyl)oxy)benzylcyclohex-2-ene-1-carboxylic acid benzyl ester 20g

Compound **20f** (2.66 g, 5.73 mmol) was dissolved in anhydrous tetrahydrofuran (80 mL), the temperature was lowered to -70° C, lithium hexamethyldisilazide (6.9 mL, 6.9 mmol, 1M tetrahydrofuran solution) was slowly added, after the addition the reaction was carried out at -70°C for one hour, N-phenylbis(trifluoromethanesulfonyl)imide (5.74 g, 16.06 mmol) in tetrahydrofuran (10 mL) solution was slowly added dropwise, and after the addition the temperature was slowly lifted to room temperature for reaction overnight. The reaction solution was quenched with a saturated ammonium chloride solution and extracted with ethyl acetate, the organic phases were combined, washed with saturated salt water, dried over anhydrous sodium sulfate and concentrated, and the crude product was purified by silica gel column chromatography to obtain the title compound **20g** (3.9 g, yield 94%).

### Step 7: mixture of 3-(pyridin-3-yl)cyclohex-3-ene-1-carboxylic acid benzyl ester and 3-(pyridin-3-yl)cyclohex-2-ene-1-carboxylic acid benzyl ester 20i

Compound **20g** (1.9 g, 5.21 mmol) and 3-pyridineboronic acid **20h** (645 mg, 5.25 mmol) were dissolved in 1,4-dioxane (30 mL) and water (10 mL), Pd(dppf)Cl₂ (383 mg, 0.52 mmol) and sodium carbonate (976 mg, 9.21 mmol) were added at room temperature, it was replaced with nitrogen three times, and the temperature was lifted to 100°C for 2 hours under nitrogen protection. The complete reaction of the starting material was shown by TLC. The reaction solution was cooled to room temperature, added with water and extracted with ethyl acetate, the organic phases were combined, washed with saturated salt water, dried over anhydrous sodium sulfate and concentrated, and the crude product was purified by silica gel column chromatography to obtain the title compound **20i** (560 mg, yield 37%).

LC-MS: m/z=294.2[M+H]⁺

### Step 8: 3-(pyridin-3-yl)cyclohexane-1-carboxylic acid 20j

Compound **20i** (560 mg, 1.91 mmol) was dissolved in ethyl acetate (30 mL), palladium/carbon (150 mg, 10%) was added, and the reaction was carried out for 4 hours at room temperature in hydrogen atmosphere. The complete reaction of the starting material was shown by TLC. The reaction solution was filtered with diatomite, and the filtrate was concentrated to obtain the white solid title compound **20j** (320 mg, crude product), which was directly used for the next step.

### Step 9: (1S,3S)-N-(5-chloro-4-(5,5-dimethyl-5,6-dihydro-4H-pyrrolo[1,2-b]pyrazol-3-yl)pyridin-2-yl)-3-( pyridin-3-yl)cyclohexane-1-carboxamide (assumed) 20-1 &

### (1R,3S)-N-(5-chloro-4-(5,5-dimethyl-5,6-dihydro-4H-pyrrolo[1,2-b]pyrazol-3-yl)pyridin-2-yl)-3-( pyridin-3-yl)cyclohexane-1-carboxamide (assumed) 20-2 &

### (1S,3R)-N-(5-chloro-4-(5,5-dimethyl-5,6-dihydro-4H-pyrrolo[1,2-b]pyrazol-3-yl)pyridin-2-yl)-3-( pyridin-3-yl)cyclohexane-1-carboxamide (assumed) 20-3 &

### (1R,3R)-N-(5-chloro-4-(5,5-dimethyl-5,6-dihydro-4H-pyrrolo[1,2-b]pyrazol-3-yl)pyridin-2-yl)-3-( pyridin-3-yl)cyclohexane-1-carboxamide (assumed) 20-4

Compound **20j** (320 mg, crude product) and compound **20b** (412 mg, crude product) were dissolved in ethyl acetate (20 mL), 1-propylphosphonic anhydride (2.3 g, 3.61 mmol, 50% ethyl acetate solution) and pyridine (457 mg, 5.78 mmol) were added at room temperature, and the temperature was lifted to 40°C for reaction for 2 hours. The reaction solution was quenched with water and extracted with ethyl acetate, the organic phases were combined, washed with saturated salt water, dried over anhydrous sodium sulfate and concentrated to obtain the white solid compound **20** (400 mg, two-step yield 74%). Chiral resolution (DAICEL AD-H, 30*250mm, 5um, 30mL/min, IPA:Hexane=40:60) to obtain the title compound **20-1** (peak 1, RT 34 min) (12.9 mg, yield 3%), the title compound **20-2** (peak 2, RT 48 min) (7.9 mg, yield 2%), the title compound **20-3** (peak 3, RT 94 min) (63.2 mg, yield 16%), and the title compound **20-4** (peak 4, RT 130 min)

(58.1 mg, yield 14%).

### Compound 20-1

LC-MS: m/z=450.2 [M+H]⁺

¹H NMR (400 MHz, DMSO-*d₆*) δ 10.55 (s, 1H), 8.51-8.26 (m, 4H), 7.91 (s, 1H), 7.70 (s, 1H), 7.34 (s, 1H), 3.96 (s, 2H), 3.00 (s, 1H), 2.95-2.90 (m, 3H), 2.08-1.59 (m, 8H), 1.28 (s, 6H). (98.87% purity by HPLC)

### Compound 20-2

LC-MS: m/z=450.2 [M+H]⁺

¹H NMR (400 MHz, DMSO-*d₆*) δ 10.55 (s, 1H), 8.51-8.27 (m, 4H), 7.97 (s, 1H), 7.71 (d, J = 7.2 Hz, 1H), 7.35-7.32 (m, 1H), 3.96 (s, 2H), 3.10 (s, 1H), 2.96-2.90 (m, 3H), 2.06-1.59 (m, 8H), 1.28 (s, 6H). (99.34% purity by HPLC)

### Compound 20-3

LC-MS: m/z=450.2 [M+H]⁺

¹H NMR (400 MHz, DMSO-*d₆*) δ 10.60 (s, 1H), 8.49 (d, J = 1.6 Hz, 1H), 8.41 (dd, J = 1.6, 4.8 Hz, 1H), 8.35 (s, 1H), 8.27 (s, 1H), 8.01 (s, 1H), 7.71-7.68 (m, 1H), 7.32 (dd, J = 4.8, 7.6 Hz, 1H), 3.94 (s, 2H), 2.89 (s, 2H), 2.76-2.64 (m, 2H), 1.95-1.90 (m, 3H), 1.80-1.77 (m, 1H), 1.71-1.62 (m, 1H), 1.47-1.45 (m, 3H), 1.27 (s, 6H). (99.96% purity by HPLC)

### Compound 20-4

LC-MS: m/z=450.2 [M+H]⁺

¹H NMR (400 MHz, DMSO-*d₆*) δ 10.60 (s, 1H), 8.49 (s, 1H), 8.41 (d, J = 3.6 Hz, 1H), 8.35 (s, 1H), 8.27 (s, 1H), 8.01 (s, 1H), 7.70 (d, J = 6.8 Hz, 1H), 7.33-7.31 (m, 1H), 3.94 (s, 2H), 2.89 (s, 2H), 2.73-2.63 (m, 2H), 1.95-1.90 (m, 3H), 1.82-1.77 (m, 1H), 1.71-1.62 (m, 1H), 1.52-1.42 (m, 3H), 1.27 (s, 6H). (97.79% purity by HPLC)

### Embodiment 21

### (1S,3S)-N-(5-chloro-4-(5,5-dimethyl-5,6-dihydro-4H-pyrrolo[1,2-b]pyrazol-3-yl)pyridin-2-yl)-3-( pyridin-4-yl)cyclohexane-1-carboxamide (assumed) 21-1

### (1S,3R)-N-(5-chloro-4-(5,5-dimethyl-5,6-dihydro-4H-pyrrolo[1,2-b]pyrazol-3-yl)pyridin-2-yl)-3-( pyridin-4-yl)cyclohexane-1-carboxamide (assumed) 21-2

### (1R,3S)-N-(5-chloro-4-(5,5-dimethyl-5,6-dihydro-4H-pyrrolo[1,2-b]pyrazol-3-yl)pyridin-2-yl)-3-( pyridin-4-yl)cyclohexane-1-carboxamide (assumed) 21-3

### (1R,3R)-N-(5-chloro-4-(5,5-dimethyl-5,6-dihydro-4H-pyrrolo[1,2-b]pyrazol-3-yl)pyridin-2-yl)-3-( pyridin-4-yl)cyclohexane-1-carboxamide (assumed) 21-4

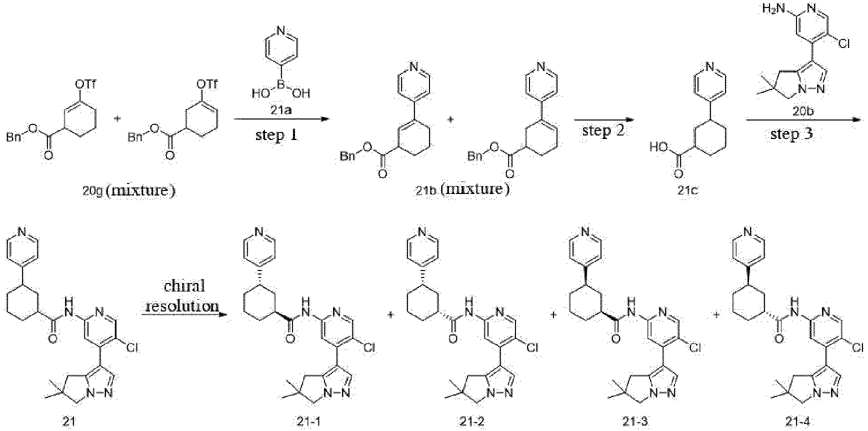

### Step 1: mixture of 3-(pyridin-4-yl)cyclohex-2-ene-1-carboxylic acid benzyl ester and 3-(pyridin-4-yl)cyclohex-3-ene-1-carboxylic acid benzyl ester 21b

Mixed Compound **20g** (2.1 g, 5.76 mmol) and 3-pyridineboronic acid **21a** (710 mg, 5.78 mmol) were dissolved in 1,4-dioxane (30 mL) and water (10 mL), Pd(dppf)Cl₂ (400 mg, 0.54 mmol) and sodium carbonate (1.22 g, 11.51 mmol) were added at room temperature, and the temperature was lifted to 100°C for reaction for 2 hours under nitrogen protection. The complete reaction of the starting material was shown by TLC. The reaction solution was cooled to room temperature, added with water and extracted with ethyl acetate, the organic phases were combined, washed with saturated salt water, dried over anhydrous sodium sulfate and concentrated, and the crude product was purified by silica gel column chromatography to obtain the title compound **21b** (690 mg, yield 41%).

### Step 2: 3-(pyridin-4-yl)cyclohexane-1-carboxylic acid 21c

Mixed Compound **21b** (690 mg, 2.35 mmol) was dissolved in ethyl acetate (20 mL), palladium/carbon (200 mg, 10%) was added, and the reaction was carried out overnight at room temperature in hydrogen atmosphere. The reaction solution was filtered with diatomite, and the filtrate was concentrated to obtain the title compound **21c** (360 mg, crude product), which was directly used for the next step.

### Step 3: (1S,3S)-N-(5-chloro-4-(5,5-dimethyl-5,6-dihydro-4H-pyrrolo[1,2-b]pyrazol-3-yl)pyridin-2-yl)-3-( pyridin-4-yl)cyclohexane-1-carboxamide (assumed) 21-1 &

### (1S,3R)-N-(5-chloro-4-(5,5-dimethyl-5,6-dihydro-4H-pyrrolo[1,2-b]pyrazol-3-yl)pyridin-2-yl)-3-( pyridin-4-yl)cyclohexane-1-carboxamide (assumed) 21-2 &

### (1R,3S)-N-(5-chloro-4-(5,5-dimethyl-5,6-dihydro-4H-pyrrolo[1,2-b]pyrazol-3-yl)pyridin-2-yl)-3-( pyridin-4-yl)cyclohexane-1-carboxamide (assumed) 21-3 &

### (1R,3R)-N-(5-chloro-4-(5,5-dimethyl-5,6-dihydro-4H-pyrrolo[1,2-b]pyrazol-3-yl)pyridin-2-yl)-3-( pyridin-4-yl)cyclohexane-1-carboxamide (assumed) 21-4

Compound **21c** (360 mg, crude product) and Compound **20b** (466 mg, 1.77 mmol) were dissolved in ethyl acetate (20 mL), 1-propylphosphonic anhydride (4.45 g, 6.99 mmol, 50% ethyl acetate solution) and pyridine (829 mg, 10.48 mmol) were added at room temperature, and the temperature was lifted to 40°C for reaction for 2 hours. The reaction solution was added with water and extracted with ethyl acetate, the organic phases were combined, washed with saturated salt water, dried over anhydrous sodium sulfate, concentrated and purified by silica gel column chromatography to obtain the white solid compound **21** (360 mg, yield 45%). Chiral resolution (DAICEL AD-H, 30*250mm, 5um, 30mL/min, IPA:Hexane=40:60) to obtain the title compound **21-1** (peak 1, RT 19.5 min) (10.1 mg, yield 3%), the title compound **21-2** (peak 2, RT 35.0 min) (5.8 mg, yield 2%), the title compound **21-3** (peak 3, RT 44.0 min) (51.1 mg, yield 14%), and the title compound **21-4** (peak 4, RT 56.5 min) (62.1 mg, yield 17%).

### Compound 21-1

LC-MS: m/z=450.2 [M+H]⁺

¹H NMR (400 MHz, DMSO-*d₆*) δ 10.55 (s, 1H), 8.47 (d, J = 5.6 Hz, 2H), 8.36 (s, 1H), 8.26 (s, 1H), 7.97 (s, 1H), 7.31 (d, J = 5.6 Hz, 2H), 3.96 (s, 2H), 3.10-3.04 (m, 1H), 2.92-2.90 (m, 3H), 2.07-2.04 (m, 1H), 1.90-1.77 (m, 3H), 1.66-1.53 (m, 4H), 1.27 (s, 6H). (97.91% purity by HPLC)

### Compound 21-2

LC-MS: m/z=450.2 [M+H]⁺

¹H NMR (400 MHz, DMSO-*d₆*) δ 10.56 (s, 1H), 8.47 (d, J = 5.6 Hz, 2H), 8.36 (s, 1H), 8.26 (s, 1H), 7.97 (s, 1H), 7.31 (d, J = 5.6 Hz, 2H), 3.95 (s, 2H), 3.11-3.02 (m, 1H), 2.95-2.90 (m, 3H), 2.08-2.04 (m, 1H), 1.91-1.87 (m, 2H), 1.79-1.53 (m, 5H), 1.27 (s, 6H). (98.25% purity by HPLC)

### Compound 21-3

LC-MS: m/z=450.2 [M+H]⁺

¹H NMR (400 MHz, DMSO-*d₆*) δ 10.60 (s, 1H), 8.47 (d, J = 5.6 Hz, 2H), 8.35 (s, 1H), 8.27 (s, 1H), 8.01 (s, 1H), 7.29 (d, J = 5.6 Hz, 2H), 3.94 (s, 2H), 2.89 (s, 2H), 2.74-2.58 (m, 2H), 1.95-1.91 (m, 3H), 1.80-1.78 (m, 1H), 1.70-1.60 (m, 1H), 1.48-1.2 (m, 3H), 1.27 (s, 6H). (99.12% purity by HPLC)

### Compound 21-4

LC-MS: m/z=450.2 [M+H]⁺

¹H NMR (400 MHz, DMSO-*d₆*) δ 10.61 (s, 1H), 8.47 (d, J = 5.6 Hz, 2H), 8.35 (s, 1H), 8.27 (s, 1H), 8.01 (s, 1H), 7.29 (d, J = 5.6 Hz, 2H), 3.94 (s, 2H), 2.89 (s, 2H), 2.72-2.61 (m, 2H), 1.95-1.89 (m, 3H), 1.81-1.79 (m, 1H), 1.70-1.60 (m, 1H), 1.48-1.42 (m, 3H), 1.27 (s, 6H). (98.81% purity by HPLC)

### Embodiment 22

### (1S,3R)-N-(5-chloro-4-(5,5-dimethyl-5,6-dihydro-4H-pyrrolo[1,2-b]pyrazol-3-yl)pyridin-2-yl)-3-( 1-methyl-1H-pyrazol-3-yl)cyclohexane-1-carboxamide (assumed) 22-1-1

### (1R,3S)-N-(5-chloro-4-(5,5-dimethyl-5,6-dihydro-4H-pyrrolo[1,2-b]pyrazol-3-yl)pyridin-2-yl)-3-( 1-methyl-1H-pyrazol-3-yl)cyclohexane-1-carboxamide (assumed) 22-1-2

### (1S,3R)-N-(5-chloro-4-(5,5-dimethyl-5,6-dihydro-4H-pyrrolo[1,2-b]pyrazol-3-yl)pyridin-2-yl)-3-( 1-methyl-1H-pyrazol-5-yl)cyclohexane-1-carboxamide (assumed) 22-2-1

### (1R,3S)-N-(5-chloro-4-(5,5-dimethyl-5,6-dihydro-4H-pyrrolo[1,2-b]pyrazol-3-yl)pyridin-2-yl)-3-( 1-methyl-1H-pyrazol-5-yl)cyclohexane-1-carboxamide (assumed) 22-2-2

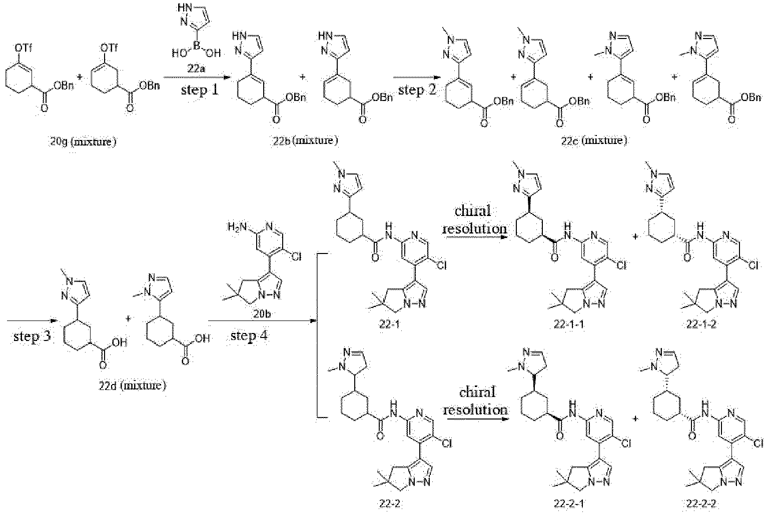

### Step 1: mixture of 3-(1H-pyrazol-3-yl)cyclohex-2-ene-1-carboxylic acid benzyl ester and 3-(1H-pyrazol-3-yl)cyclohex-3-ene-1-carboxylic acid benzyl ester 22b

Mixed Compound **20g** (2.6 g, 7.14 mmol) and 1*H*-pyrazole-3-boronic acid **22a** (800 mg, 7.15 mmol) were dissolved in 1,4-dioxane (15 mL) and water (5 mL), Pd(dppf)Cl₂ (150 mg, 0.20 mmol) and sodium carbonate (1.5 g, 14.15 mmol) were added at room temperature, the temperature was lifted to 100°C for reaction for 2 hours under nitrogen protection, and the reaction was complete shown by TLC. The reaction solution was cooled to room temperature, quenched with water and extracted with ethyl acetate, the organic phases were combined, washed with saturated salt water, dried over anhydrous sodium sulfate and concentrated, and the crude product was purified by silica gel column chromatography to obtain the white solid title compound **22b** (1.6 g, yield 80%).

Step 2: mixture of 3-(1-methyl-1*H*-pyrazol-3-yl)cyclohex-2-ene-1-carboxylic acid benzyl ester, 3-(1-methyl-1*H*-pyrazol-3-yl)cyclohex-3-ene-1-carboxylic acid benzyl ester, 3-(1-methyl-1*H*-pyrazol-5-yl)cyclohex-2-ene-1-carboxylic acid benzyl ester and 3-(1-methyl-1*H*-pyrazol-5-yl)cyclohex-3-ene-1-carboxylic acid benzyl ester **22c** Mixed Compound **22b** (1.6 g, 5.67 mmol) was dissolved in *N,N*-dimethylformamide (20 mL), cesium carbonate (2.76 g, 8.50 mmol) was added, methyl iodide (0.81 g, 5.71 mmol) was added dropwise at room temperature, after the addition it was stirred for three hours at room temperature, and the reaction was complete shown by TLC. The reaction solution was quenched with water and extracted with ethyl acetate, the organic phases were combined, washed with saturated salt water, dried over anhydrous sodium sulfate and concentrated, and the crude product was separated and purified by silica gel column chromatography to obtain the white solid title compound **22c** (900 mg, yield 54%).

### Step 3: mixture of 3-(1-methyl-1H-pyrazol-3-yl)cyclohexane-1-carboxylic acid and 3-(1-methyl-1H-pyrazol-5-yl)cyclohexane-1-carboxylic acid 22d

The compound **22c** (900 mg, 3.04 mmol) was dissolved in methanol (20 mL), palladium/carbon (200 mg, 10%) was added at room temperature, the reaction was carried out overnight at 40°C under hydrogen atmosphere, and the reaction was complete shown by TLC. The reaction solution was filtered with diatomite, and the filtrate was concentrated to obtain the white solid mixture title compound **22d** (510 mg, crude product), which was directly used for the next step.

### Step 4: (1S,3R)-N-(5-chloro-4-(5,5-dimethyl-5,6-dihydro-4H-pyrrolo[1,2-b]pyrazol-3-yl)pyridin-2-yl)-3-( 1-methyl-1H-pyrazol-3-yl)cyclohexane-1-carboxamide (assumed) 22-1-1 &

### (1R,3S)-N-(5-chloro-4-(5,5-dimethyl-5,6-dihydro-4H-pyrrolo[1,2-b]pyrazol-3-yl)pyridin-2-yl)-3-( 1-methyl-1H-pyrazol-3-yl)cyclohexane-1-carboxamide (assumed) 22-1-2 &

### (1S,3R)-N-(5-chloro-4-(5,5-dimethyl-5,6-dihydro-4H-pyrrolo[1,2-b]pyrazol-3-yl)pyridin-2-yl)-3-( 1-methyl-1H-pyrazol-5-yl)cyclohexane-1-carboxamide (assumed) 22-2-1 &

### (1R,3S)-N-(5-chloro-4-(5,5-dimethyl-5,6-dihydro-4H-pyrrolo[1,2-b]pyrazol-3-yl)pyridin-2-yl)-3-( 1-methyl-1H-pyrazol-5-yl)cyclohexane-1-carboxamide (assumed) 22-2-2

Mixed Compound **22d** (510 mg, 2.45 mmol) and Compound **20b** (642 mg, 2.44 mmol) were dissolved in ethyl acetate (10 mL), 1-propylphosphonic anhydride (6.2 g, 9.74 mmol, 50% ethyl acetate solution) and pyridine (1.16 g, 14.66 mmol) were added, the reaction was carried out overnight at room temperature, and the reaction was complete shown by TLC. The reaction solution was quenched with water and extracted with ethyl acetate, the organic phases were combined, washed with saturated salt water, dried over anhydrous sodium sulfate and concentrated, and the crude product was purified by Prep-TLC to obtain the low-polarity compound **22-1** (140 mg, yield 13%) and the high-polarity compound **22-2** (58 mg, yield 4.2%). Compound **22-1** was chiral resolved (NANOMICRO OD-5H, 30*250mm, 5um, 30mL/min, IPA:Hexane=40:60) to obtain the title compound **22-1-1** (peak 1, RT 30.6 min) (32 mg, yield 23%) and the title compound **22-1-2** (peak 2, RT 92.1 min) (30 mg, yield 21%). Compound **22-2** was chiral resolved (NANOMICRO 30*250mm, 5um, 30mL/min, IPA:Hexane=40:60) to obtain the title compound **22-2-1** (Peak 1, RT 30.0 min) (7 mg, yield 12%) and the title compound **22-2-2** (peak 2, RT 38.0 min) (9 mg, yield 16%).

### Compound 22-1-1

LC-MS: m/z=453.2[M+H]⁺

¹H NMR (400 MHz, DMSO-*d₆*) δ 10.57 (s, 1H), 8.35 (s, 1H), 8.27 (s, 1H), 8.01 (s, 1H), 7.52 (s, 1H), 6.04 (s, 1H), 3.94 (s, 2H), 3.75 (s, 3H), 2.90 (s, 2H), 2.67-2.51 (m, 2H), 2.05-2.02 (m, 1H), 1.92-1.84 (m, 3H), 1.58-1.48 (m, 1H), 1.43-1.39 (m, 2H), 1.27 (s, 7H). (97.25% purity by HPLC)

### Compound 22-1-2

LC-MS: m/z=453.2[M+H]⁺

¹H NMR (400 MHz, DMSO-*d₆*) δ 10.57 (s, 1H), 8.35 (s, 1H), 8.27 (s, 1H), 8.01 (s, 1H), 7.52 (s, 1H), 6.04 (s, 1H), 3.94 (s, 2H), 3.75 (s, 3H), 2.90 (s, 2H), 2.67-2.51 (m, 2H), 2.06-1.85 (m, 4H), 1.58-1.40 (m, 3H), 1.27 (s, 7H). (99.78% purity by HPLC)

### Compound 22-2-1

LC-MS: m/z=453.2[M+H]⁺

¹H NMR (400 MHz, DMSO-*d₆*) δ 10.62 (s, 1H), 8.35 (s, 1H), 8.27 (s, 1H), 8.01 (s, 1H), 7.27 (s, 1H), 6.05 (s, 1H), 3.94 (s, 2H), 3.78 (m, 3H), 2.89 (s, 2H), 2.77-2.68 (m, 2H), 2.03-1.86 (m, 4H), 1.55-1.44 (m, 3H), 1.27(s, 7H). (96.27% purity by HPLC)

### Compound 22-1-2

LC-MS: m/z=453.2[M+H]⁺

¹H NMR (400 MHz, DMSO-*d₆*) δ 10.62 (s, 1H), 8.35 (s, 1H), 8.27 (s, 1H), 8.01 (s, 1H), 7.2 (s, 1H), 6.05 (s, 1H), 3.94 (s, 2H), 3.78 (m, 3H), 2.89 (s, 2H), 2.77-2.68 (m, 2H), 2.03-1.86 (m, 4H), 1.53-1.42 (m, 3H), 1.27(s, 7H). (99.53% purity by HPLC)

### Embodiment 23

### (1S,3R)-N-(5-chloro-4-(5,5-dimethyl-5,6-dihydro-4H-pyrrolo[1,2-b]pyrazol-3-yl)pyridin-2-yl)-3-( 1-methyl-1H-pyrazol-4-yl)cyclohexane-1-carboxamide (assumed) 23-1

### (1R,3S)-N-(5-chloro-4-(5,5-dimethyl-5,6-dihydro-4H-pyrrolo[1,2-b]pyrazol-3-yl)pyridin-2-yl)-3-( 1-methyl-1H-pyrazol-4-yl)cyclohexane-1-carboxamide (assumed) 23-2

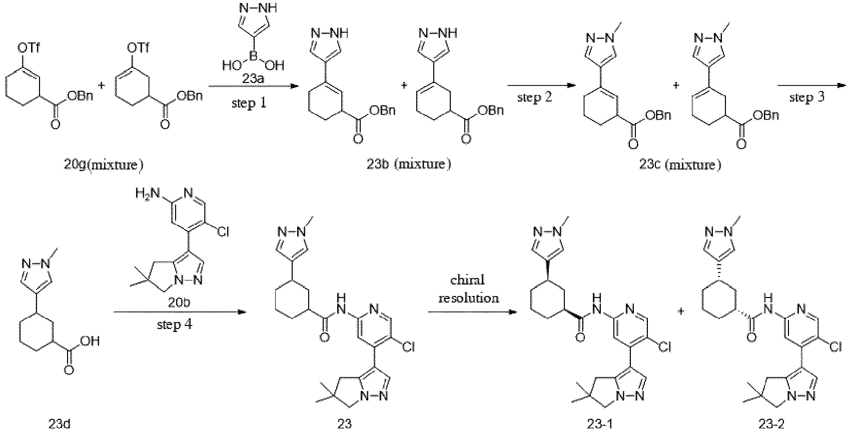

### Step 1: mixture of 3-(1H-pyrazol-4-yl)cyclohex-2-ene-1-carboxylic acid benzyl ester and 3-(1H-pyrazol-4-yl) cyclohex-3-ene-1-carboxylic acid benzyl ester 23b

Mixed Compound **20g** (2.6 g, 7.14 mmol) and 1*H*-pyrazole-4-boronic acid **23a** (800 mg, 7.15 mmol) were dissolved in 1,4-dioxane (15 mL) and water (5 mL), Pd(dppf)Cl₂ (150 mg, 0.20 mmol) and sodium carbonate (1.5 g, 14.15 mmol) were added, the temperature was lifted to 100°C for reaction for two hours under nitrogen protection, and the reaction was complete shown by TLC. The reaction solution was cooled to room temperature, quenched with water and extracted with ethyl acetate, the organic phases were combined, washed with saturated salt water, dried over anhydrous sodium sulfate and concentrated, and the crude product was purified by silica gel column chromatography to obtain the white solid title compound **23b** (590 mg, yield 29.5%).

### Step 2: mixture of 3-(1-methyl-1H-pyrazol-4-yl)cyclohex-2-ene-1-carboxylic acid benzyl ester and 3-(1-methyl-1H-pyrazol-4-yl)cyclohex-3-ene-1-carboxylic acid benzyl ester 23c

Mixed Compound **23b** (590 mg, 2.09 mmol) was dissolved in *N,N*-dimethylformamide (10 mL), cesium carbonate (1.02 g, 3.13 mmol) was added, methyl iodide (297 mg, 2.09 mmol) was added dropwise at room temperature, after the addition it was stirred at room temperature for 3 hours, and the reaction was complete shown by TLC. The reaction solution was quenched with water and extracted with ethyl acetate, the organic phases were combined, washed with saturated salt water, dried over anhydrous sodium sulfate and concentrated, and the crude product was purified by silica gel column chromatography to obtain the white solid title compound **23c** (380 mg, yield 61.3%).

### Step 3: 3-(1-methyl-1H-pyrazol-4-yl)cyclohexane-1-carboxylic acid 23d

Compound **23c** (320 mg, 1.08 mmol) was dissolved in ethanol (15 mL), palladium/carbon (100 mg, 10%) was added, and the reaction was carried out overnight at room temperature in hydrogen atmosphere. The reaction was complete shown by LC-MS. The reaction solution was filtered with diatomite, and the filtrate was concentrated to obtain the white solid title compound **23d** (290 mg, crude product), which was directly used for the next step.

LC-MS: m/z=209.2[M+H]⁺

### Step 4: (1S,3R)-N-(5-chloro-4-(5,5-dimethyl-5,6-dihydro-4H-pyrrolo[1,2-b]pyrazol-3-yl)pyridin-2-yl)-3-( 1-methyl-1H-pyrazol-4-yl)cyclohexane-1-carboxamide (assumed) 23-1 &

### (1R,3S)-N-(5-chloro-4-(5,5-dimethyl-5,6-dihydro-4H-pyrrolo[1,2-b]pyrazol-3-yl)pyridin-2-yl)-3-( 1-methyl-1H-pyrazol-4-yl)cyclohexane-1-carboxamide (assumed) 23-2

Compound **23d** (200 mg, crude product) and Compound **20b** (252 mg, 0.96 mmol) were dissolved in ethyl acetate (10 mL), 1-propylphosphonic anhydride (2.4 g, 3.77 mmol, 50% ethyl acetate solution) and pyridine (455 mg, 5.75 mmol) were added, and the reaction was carried out overnight at room temperature. The reaction solution was added with water and extracted with ethyl acetate, the organic phases were combined, washed with saturated salt water, dried over anhydrous sodium sulfate and concentrated, and the crude product was purified by silica gel column chromatography to obtain the compound **23** (170 mg, two-step yield 35%) and chiral resolved (NANOMICRO OD-5H, 30^{∗}250mm, 5um, 30mL/min, IPA:Hexane=40:60) to obtain the title compound **23-1** (peak 1, RT 42.5 min) (32 mg, yield 19%) and the title compound **23-2** (peak 2, RT 50.0 min) (30 mg, yield 18%).

### Compound 23-1

LC-MS: m/z=453.2[M+H]⁺

¹H NMR (400 MHz, DMSO-*d₆*) δ 10.56 (s, 1H), 8.35 (s, 1H), 8.28 (s, 1H), 8.01 (s, 1H), 7.48 (s, 1H), 7.28 (s, 1H), 3.94 (s, 2H), 3.76 (s, 3H), 2.89 (s, 2H), 2.71-2.64 (m, 1H), 2.49-2.46 (m, 1H), 2.04-1.83 (m, 4H), 1.49-1.37 (m, 3H), 1.27 (s, 7H). (99.51% purity by HPLC)

### Compound 23-2

LC-MS: m/z=453.2[M+H]⁺

¹H NMR (400 MHz, DMSO-*d₆*) δ 10.56 (s, 1H), 8.35 (s, 1H), 8.27 (s, 1H), 8.01 (s, 1H), 7.48 (s, 1H), 7.28 (s, 1H), 3.94 (s, 2H), 3.76 (s, 3H), 2.89 (s, 2H), 2.69-2.64 (m, 1H), 2.49-2.46 (m, 1H), 2.04-1.85 (m, 4H), 1.49-1.37 (m, 3H), 1.27 (s, 7H). (98.22% purity by HPLC)

### Embodiment 24

### 7-(2-((2r,3aR,5s,6aS)-5-acetylaminooctahydropentalene-2-carboxamido)-5-chloropyridin-4-yl)-2, 2-dimethyl-2,3-dihydro-1H-pyrrolizine-5-carboxamide (assumed) 24-1

### 7-(2-((2s,3aR,5r,6aS)-5-acetylaminooctahydropentalene-2-carboxamido)-5-chloropyridin-4-yl)-2, 2-dimethyl-2,3-dihydro-1H-pyrrolizine-5-carboxamide (assumed) 24-2

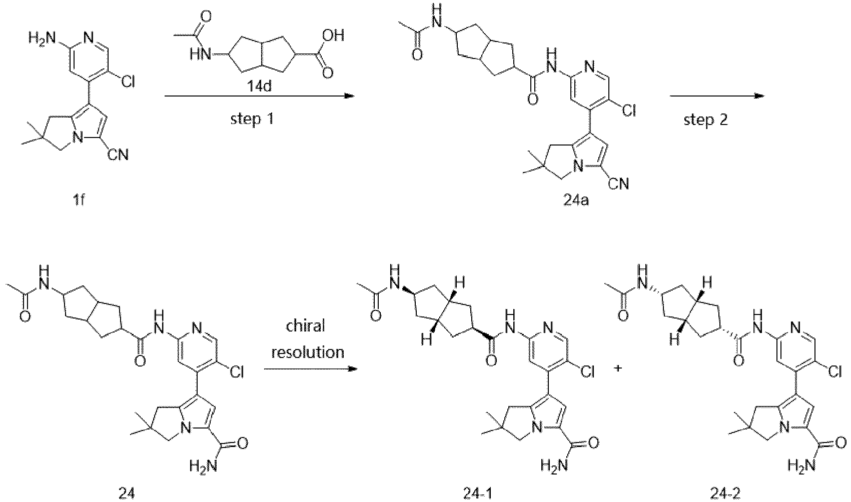

### Step 1: 5-acetylamino-N-(5-chloro-4-(5-cyano-2,2-dimethyl-2,3-dihydro-1H-pyrrolizin-7-yl)pyridin-2-yl) octahydropentalene-2-carboxamide 24a

Compound **14d** (272 mg, 0.95 mmol) and Compound **1f** (241 mg, 1.15 mmol) were dissolved in N,N-dimethylformamide (6 mL), 1-propylphosphonic anhydride (1.2 g, 1.88 mmol, 50% N,N-dimethylformamide solution) and pyridine (300 mg, 3.79 mmol) were added at room temperature, the temperature was lifted to 40°C for reaction for 3 hours, and the reaction was complete by TLC. The reaction solution was quenched with water and extracted with ethyl acetate, the organic phases were combined, washed with water, washed with saturated salt water, dried over anhydrous sodium sulfate and concentrated, and the crude product was separated and purified by silica gel column chromatography to obtain the white solid title compound **24a** (220 mg, yield 48%).

### Step 2: 7-(2-((2r,3aR,5s,6aS)-5-acetylaminooctahydropentalene-2-carboxamido)-5-chloropyridin-4-yl)-2, 2-dimethyl-2,3-dihydro-1H-pyrrolizine-5-carboxamide (assumed) 24-1 &

### 7-(2-((2s,3aR,5r,6aS)-5-acetylaminooctahydropentalene-2-carboxamido)-5-chloropyridin-4-yl)-2, 2-dimethyl-2,3-dihydro-1H-pyrrolizine-5-carboxamide (assumed) 24-2

Compound **24a** (220 mg, 0.46 mmol) was dissolved in dimethyl sulfoxide (6 mL), potassium carbonate (127 mg, 0.92 mmol) and hydrogen peroxide (2 mL, 30%) were added, the reaction was carried out for 1 hour at room temperature, and the reaction was complete shown by TLC. The reaction solution was quenched with water, a white solid was precipitated and filtered, and the filter cake was washed and dried to obtain the white solid compound **24** crude product, which was chiral resolved (NANOMICRO OD-5H, 30^{∗}250mm, 5um, 30mL/min, IPA:Hexane=30:70) to obtain the white solid title compound **24-1** (peak 3, RT 53.0 min) (55 mg, yield 31%) and the title compound **24-2** (peak 4, RT 60.0 min) (33 mg, yield 18.7%).

### Compound 24-1

LC-MS: m/z=498.2[M+H]⁺

¹H NMR (400 MHz, DMSO-*d₆*) δ 10.52 (s, 1H), 8.32 (s, 1H), 8.25 (s, 1H), 7.87 (d, J = 7.6 Hz, 1H), 7.62 (br, 1H), 7.35 (s, 1H), 6.95 (br 1H), 4.04 (s, 3H), 3.06-3.02 (m, 1H), 2.79 (s, 2H), 2.43-2.31 (m, 2H), 2.15-2.03 (m, 4H), 1.76 (s, 3H), 1.55-1.48 (m, 2H), 1.24-1.17 (m, 8H). (98.86% purity by HPLC)

### Compound 24-2

LC-MS: m/z=498.2[M+H]⁺

¹H NMR (400 MHz, DMSO-*d₆*) δ 10.63 (s, 1H), 8.32 (s, 1H), 8.24 (s, 1H), 7.86 (d, J = 7.6 Hz, 1H), 7.63 (br, 1H), 7.34 (s, 1H), 6.94 (br, 1H), 4.04 (s, 2H), 3.77-3.70 (m, 1H), 3.08-3.04 (m, 1H), 2.78 (s, 2H), 2.49-2.45 (m, 2H), 2.10-2.04 (m, 2H), 1.81-1.78 (m, 5H), 1.73-1.63 (m, 2H), 1.24-1.20 (m, 8H). (90.76% purity by HPLC)

### Embodiment 25

### (2s,3aR,5r,6aS)-N²-(5-chloro-4-(5-cyano-2,2-dimethyl-2,3-dihydro-1H-pyrrolizin-7-yl)pyridin-2-y l)-N⁵-methyloctahydropentalene-2,5-dicarboxamide (assumed) 25-1

### (2r,3aR,5s,6aS)-N²-(5-chloro-4-(5-cyano-2,2-dimethyl-2,3-dihydro-1H-pyrrolizin-7-yl)pyridin-2-y l)-N⁵-methyloctahydropentalene-2,5-dicarboxamide (assumed) 25-2

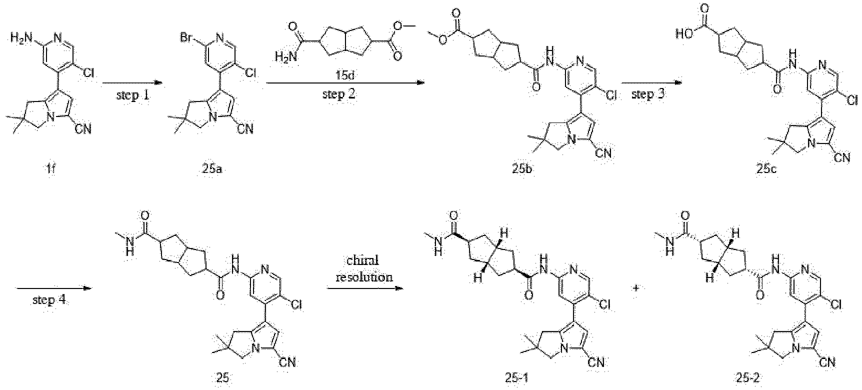

### Step 1: 7-(2-bromo-5-chloropyridin-4-yl)-2,2-dimethyl-2,3-dihydro-1H-pyrrolizine-5-carbonitrile 25a

Compound **1f** (1.0 g, 3.49 mmol) was dissolved in dibromomethane (20 mL), copper bromide (800 mg, 3.58 mmol) and isoamyl nitrite (820 mg, 7.00 mmol) were added at room temperature, under nitrogen protection, the temperature was lifted to 60°C, it was stirred for 3 hours, and the reaction was complete shown by TLC. The reaction solution was cooled to room temperature, diluted with water and extracted with ethyl acetate, the organic phases were combined, washed with saturated salt water, dried over anhydrous sodium sulfate and concentrated, and the crude product was separated and purified by silica gel column chromatography to obtain the yellow solid title compound **25a** (700 mg, yield 59%).

### Step 2: 5-((5-chloro-4-(5-cyano-2,2-dimethyl-2,3-dihydro-1H-pyrrolizin-7-yl)pyridin-2-yl)carbamoyl)oct ahydropentalene-2-carboxylic acid methyl ester 25b

Compound **25a** (85 mg, 0.24 mmol) and Compound **15d** (61 mg, 0.29 mmol) were dissolved in 1,4-dioxane (1 mL), cesium carbonate (240 mg, 0.74 mmol), Xantphos (28 mg, 0.05 mmol) and tetrakis(triphenylphosphine)palladium (28 mg, 0.02 mmol) were added at room temperature, the temperature was lifted to 120°C for reaction for 2 hours under nitrogen protection, and the reaction was complete shown by TLC. The reaction solution was cooled to room temperature, diluted with water and extracted with ethyl acetate, the organic phases were combined, washed with saturated salt water, dried over anhydrous sodium sulfate and concentrated, and the crude product was purified by Prep-TLC to obtain the yellow solid title compound **25b** (30 mg, yield 26%).

### Step 3: 5-((5-chloro-4-(5-cyano-2,2-dimethyl-2,3-dihydro-1H-pyrrolizin-7-yl)pyridin-2-yl)carbamoyl)oct ahydropentalene-2-carboxylic acid 25c

Compound **25b** (400 mg, 0.83 mmol) was dissolved in tetrahydrofuran (8 mL) and water (3 mL), sodium hydroxide (140 mg, 3.50 mmol) was added, the reaction was carried out for 72 hours at room temperature, and the reaction was complete shown by TLC. The reaction solution was concentrated to remove tetrahydrofuran, adjusted to be acidic by diluted hydrochloric acid (1N) and extracted with dichloromethane, the organic phases were combined, washed with water, washed with saturated salt water, dried over anhydrous sodium sulfate and concentrated to obtain the white solid title compound **25c** (350 mg, crude product), which was directly used for the next step.

### Step 4: (2s,3aR,5r,6aS)-N²-(5-chloro-4-(5-cyano-2,2-dimethyl-2,3-dihydro-1H-pyrrolizin-7-yl)pyridin-2-y l)-N⁵-methyloctahydropentalene-2,5-dicarboxamide (assumed) 25-1 &

### (2r,3aR,5s,6aS)-N²-(5-chloro-4-(5-cyano-2,2-dimethyl-2,3-dihydro-1H-pyrrolizin-7-yl)pyridin-2-y l)-N⁵-methyloctahydropentalene-2,5-dicarboxamide (assumed) 25-2

Compound **25c** (400 mg, crude product) and methylamine hydrochloride (147 mg, 2.18 mmol) were dissolved in dichloromethane (10 mL), HATU (830 mg, 2.18 mmol) and *N,N*-diisopropylethylamine (188 mg, 1.45 mmol) were added, the reaction was carried out overnight at room temperature, and the reaction was complete shown by TLC. The reaction solution was quenched with water, extracted with dichloromethane, washed with water, washed with saturated salt water, dried over anhydrous sodium sulfate and concentrated, and the crude product was separated and purified by silica gel column chromatography to obtain the white solid compound **25** (400 mg, two-step yield 88%), which was chiral resolved (NANOMICRO OD-5H, 30*250mm, 5um, 30mL/min, EtOH:Hexane=20:80) to otain the title compound **25-1** (Peak 1, RT 36.0 min) (76 mg, yield 19%) and the title compound **25-2** (Peak 2, RT 43.0 min) (200 mg, yield 50%).

### Compound 25-1

LC-MS: m/z=480.2[M+H]⁺

¹H NMR (400 MHz, DMSO-*d₆*) δ 10.57 (s, 1H), 8.36 (s, 1H), 8.24 (s, 1H), 7.68-7.67 (m, 1H), 7.42 (s, 1H), 3.95 (s, 2H), 3.07-3.02 (m, 1H), 2.87 (s, 2H), 2.67-2.61 (m, 1H), 2.55 (d, J = 4.4 Hz, 3H), 2.43-2.40 (m, 2H), 2.09-2.02 (m, 2H), 1.97-1.90 (m, 2H), 1.57-1.45 (m, 4H) 1.24 (s, 6H). (95.38% purity by HPLC)

### Compound 25-2

LC-MS: m/z=480.2[M+H]⁺

¹H NMR (400 MHz, DMSO-*d₆*) δ 10.57 (s, 1H), 8.36 (s, 1H), 8.24 (s, 1H), 7.68-7.67 (m, 1H), 7.42 (s, 1H), 3.95 (s, 2H), 3.09-3.00 (m, 1H), 2.87 (s, 2H), 2.67-2.61 (m, 1H), 2.55 (d, J = 4.4 Hz, 3H), 2.45-2.40 (m, 2H), 2.09-2.02 (m, 2H), 1.97-1.91 (m, 2H), 1.56-1.45 (m, 4H) 1.24 (s, 6H). (94.41% purity by HPLC)

### Embodiment 26

### (2s,3aR,5r,6aS)-N²-(4-(5-carbamoyl-2,2-dimethyl-2,3-dihydro-1H-pyrrolizin-7-yl)-5-chloropyridi n-2-yl)-N⁵-methyloctahydropentalene-2,5-dicarboxamide (assumed) 26-1

### (2r,3aR,5s,6aS)-N²-(4-(5-carbamoyl-2,2-dimethyl-2,3-dihydro-1H-pyrrolyl-7-yl)-5-chloropyridin-2-yl)-N⁵-methyloctahydropentalene-2,5-dicarboxamide (assumed) 26-2

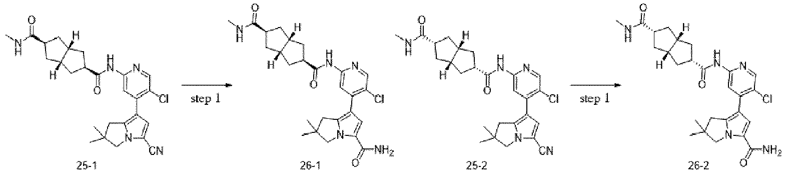

Compound **25-1** (50 mg, 0.10 mmol) was dissolved in dimethyl sulfoxide (6 mL), hydrogen peroxide (1.1 g, 30%) and potassium carbonate (27 mg, 0.20 mmol) were added, the reaction was carried out for 1 hour at room temperature, and the reaction was complete shown by TLC. The reaction solution was added with water, the solid was precipitated and filtered, and the filter cake was washed and dried to obtain the white solid title compound **26-1** (35 mg, yield 67%). Compound **25-2** (100 mg, 0.21 mmol) was dissolved in dimethyl sulfoxide (6 mL), hydrogen peroxide (2.2 g, 30%) and potassium carbonate (58 mg, 0.42 mmol) were added, the reaction was carried out for 1 hour at room temperature, and the reaction was complete shown by TLC. The reaction solution was added with water, the solid was precipitated and filtered, and the filter cake was washed and dried to obtain the white solid title compound **26-2** (35 mg, yield 34%).

### Compound 26-1

LC-MS: m/z=498.3 [M+H]⁺

¹H NMR (400 MHz, DMSO-*d₆*) δ 10.53 (s, 1H), 8.32 (s, 1H), 8.24 (s, 1H), 7.81-7.80 (m, 1H), 7.62 (br, 1H), 7.37 (s, 1H), 6.96 (br, 1H), 4.04 (s, 2H), 2.79-2.63 (m, 4H), 2.57-2.51 (m, 5H), 2.13-2.06 (m, 2H), 1.72-1.64 (m, 2H), 1.56-1.52 (m, 2H), 1.37-1.29 (m, 2H), 1.21 (s, 6H). (98.24% purity by HPLC)

### Compound 26-2

LC-MS: m/z=498.3 [M+H]⁺

¹H NMR (400 MHz, DMSO-*d₆*) δ 10.49 (s, 1H), 8.32 (s, 1H), 8.25 (s, 1H), 7.67-7.61 (m, 2H), 7.35 (s, 1H), 6.96 (br, 1H), 4.04 (s, 2H), 3.11-3.00 (m, 1H), 2.79 (s, 2H), 2.70-2.61 (m, 1H), 2.56-2.55 (m, 3H), 2.45-2.43 (m, 2H), 2.09-2.03 (m, 2H), 1.97-1.91 (m, 2H), 158-1.44 (m, 4H) 1.21 (s, 6H). (98.31% purity by HPLC)

### Embodiment 27

### (1S,3R)-3-acetamido-N-(4-(7-cyano-2,2-dimethyl-2,3-dihydro-1H-pyrrolizin-5-yl)-5-fluoropyridin -2-yl)cyclohexane-1-carboxamide 27

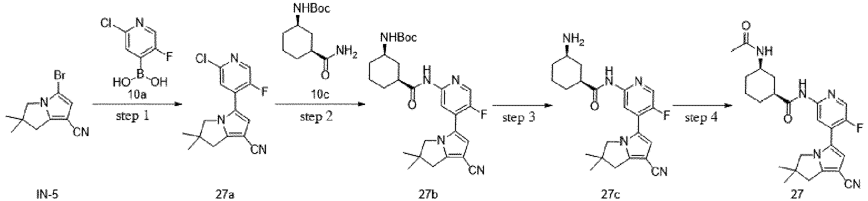

### Step1: 5-(2-chloro-5-fluoropyridin-4-yl)-2,2-dimethyl-2,3-dihydro-1H-pyrrolizine-7-carbonitrile 27a

Intermediate **IN-5** (500 mg, 2.09 mmol) and 2-chloro-5-fluoropyridine-4-boronic acid **10a** (550 mg, 3.14 mmol) were dissolved in a mixed solvent of 1,4-dioxane (10 mL) and water (2 mL), sodium carbonate (332 mg, 3.13 mmol) and Pd(dppf)Cl₂ (145 mg, 0.20 mmol) were added at room temperature, the temperature was lifted to 90°C for reaction for 3 hours under nitrogen protection, and the reaction was complete shown by TLC. The reaction solution was cooled to room temperature, added with water and extracted with ethyl acetate, the organic phases were combined, washed with saturated salt water, dried over anhydrous sodium sulfate and concentrated, and the crude product was separated and purified by silica gel column chromatography to obtain the white solid title compound **27a** (250 mg, yield 41%).

LC-MS: m/z=290.1 [M+H]⁺

### Step 2: ((1R,3S)-3-((4-(7-cyano-2,2-dimethyl-2,3-dihydro-1H-pyrrolizin-5-yl)-5-fluoropyridin-2-yl)carba moyl)cyclohexyl)carbamic acid tert-butyl ester 27b

Compound **27a** (250 mg, 0.86 mmol) was dissolved in 1,4-dioxane (3 mL), Compound **10c** (313 mg, 1.29 mmol), Pd₂(dba)₃ (79 mg, 0.09 mmol), Xphos (82 mg, 0.17 mmol) and potassium tert-butoxide (193 mg, 1.72 mmol) were added at room temperature, the temperature was lifted to 100°C for reaction overnight under nitrogen protection, and the reaction was complete shown by TLC. The reaction solution was cooled to room temperature, added with water and extracted with ethyl acetate, the organic phases were combined, washed with saturated salt water, dried over anhydrous sodium sulfate and concentrated, and the crude product was purified by silica gel column chromatography to obtain the white solid title compound **27b** (87 mg, yield 20%). LC-MS: m/z=496.3 [M+H]⁺

### Step 3: (1S,3R)-3-amino-N-(4-(7-cyano-2,2-dimethyl-2,3-dihydro-1H-pyrrolizin-5-yl)-5-fluoropyridin-2-y l)cyclohexane-1-carboxamide 27c

Compound **27b** (87 mg, 0.18 mmol) was dissolved in dichloromethane (6 mL), trifluoroacetic acid (3 mL) was added at room temperature, the reaction was carried out for 1 hour at room temperature, and the reaction was complete shown by TLC. The reaction solution was added with water and extracted with ethyl acetate, the organic phase was discarded, the aqueous phase was adjusted to pH=9 with a saturated sodium carbonate aqueous solution and extracted with ethyl acetate, the organic phases were combined, washed with saturated salt water, dried over anhydrous sodium sulfate and concentrated to obtain the white solid title compound **27c** (43 mg, crude product), which was directly used for the next step.

### Step 4: (1S,3R)-3-acetamido-N-(4-(7-cyano-2,2-dimethyl-2,3-dihydro-1H-pyrrolizin-5-yl)-5-fluoropyridin -2-yl)cyclohexane-1-carboxamide 27

Compound **27c** (43 mg, crude product) was dissolved in tetrahydrofuran (2 mL), triethylamine (22 mg, 0.22 mmol) and anhydrous acetic anhydride (11 mg, 0.11 mmol) were added at room temperature, the temperature was lifted to 50°C for reaction for 1 hour, and the reaction was complete shown by TLC. The reaction solution was cooled to room temperature, quenched with water and extracted with ethyl acetate, the organic phases were combined, washed with saturated salt, dried over anhydrous sodium sulfate and concentrated, and the crude product was separated and purified by Prep-TLC to obtain the white solid title compound **27** (25 mg, two-step yield 32%).

LC-MS: m/z=438.2 [M+H]⁺

¹H NMR (400 MHz, DMSO-*d₆*) δ 10.62 (s, 1H), 8.37 (d, J = 2.4 Hz, 1H), 8.31 (d, J = 6.0 Hz, 1H), 7.77 (d, J = 7.6 Hz, 1H), 6.96 (d, J = 2.0 Hz, 1H), 3.99 (s, 2H), 3.61-3.54 (m, 1H), 2.86 (s, 2H), 2.64-2.58 (m, 1H), 1.90-1.77 (m, 7H), 1.36-1.27(m, 3H), 1.22 (s, 7H). (99.53% purity by HPLC)

### Embodiment 28

### 5-(2-((1S,3R)-3-acetylaminocyclohexane-1-carboxamido)pyridin-4-yl)-2,2-dimethyl-2,3-dihydro-1H-pyrrolizine-7-carboxamide 28

Compound **17** (62 mg, 0.13 mmol) was dissolved in anhydrous methanol (10 mL), palladium/carbon (5 mg, 10%) was added at room temperature, the temperature was lifted to 50°C for reaction for 1 hour at room temperature, and the complete reaction of the starting material was shown by LC-MS. The reaction solution was naturally cooled to room temperature and filtered with diatomite, the filter cake was washed with absolute ethanol, the filtrate was concentrated, and the crude product was separated and purified by Prep-TLC to obtain the white solid title compound 28 (35 mg, yield 62%).

LC-MS: m/z=438.2 [M+H]⁺

¹H NMR (400 MHz, DMSO-*d₆*) δ 10.44 (s, 1H), 8.28 (s, 1H), 8.23 (d, J = 5.6 Hz, 1H), 7.81 (d, J = 8.0 Hz, 1H), 7.29 (br, 1H), 7.15 (dd, J = 1.6 Hz, 5.2 Hz, 1H), 7.12 (s, 1H), 6.77 (br, 1H), 3.99 (s, 2H), 3.62-3.53 (m, 1H), 2.84 (s, 2H), 2.67-2.59 (m, 1H), 1.91-1.87 (m, 1H), 1.78 (s, 6H), 1.35-1.29 (m, 3H), 1.21 (s, 6H), 1.11-1.05 (m, 1H). (98.90% purity by HPLC)

### Embodiment 29

### (1S,3R)-3-acetamido-N-(4-(5,5-dimethyl-5,6-dihydro-4H-pyrrolo[1,2-b]pyrazol-3-yl)-5-methoxyp yridin-2-yl)cyclohexane-1-carboxamide 29

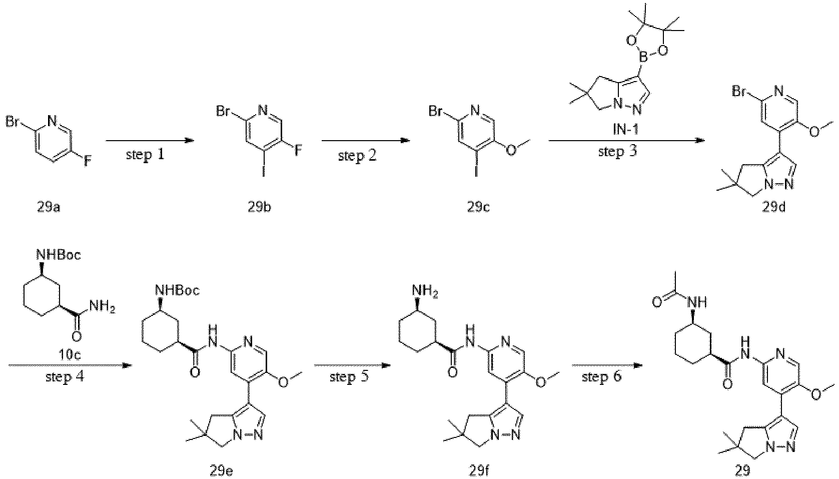

### Step 1: 2-bromo-5-fluoro-4-iodopyridine 29b

2-bromo-5-fluoropyridine **29a** (5.0 g, 28.4 mmol) was dissolved in dried tetrahydrofuran (50 mL), under nitrogen protection, the temperature was lowered to -65°C, it was stirred for 5 minutes, after that lithium diisopropylamide (18.5 mL, 36.9 mmol, 2M tetrahydrofuran solution) was added, it was stirred at -65°C for 1 hour, iodine (7.9 g, 31.2 mmol) in tetrahydrofuran (10 mL) solution was added, it was stirred at -65°C for 15 minutes, the temperature was slowly lifted to room temperature, it was stirred for 1 hour, and the reaction was complete shown by TLC. The reaction solution was poured into water and extracted with ethyl acetate, the organic phases were combined, washed with saturated salt water, dried over anhydrous sodium sulfate and concentrated, and the crude product was separated and purified by silica gel column chromatography to obtain the yellow solid title compound **29b** (6.5 g, yield 76%).

¹H NMR (400 MHz, DMSO-*d₆*) δ 8.37 (s, 1H), 8.25 (d, *J=* 4.5 Hz, 1H).

### Step 2: 2-bromo-4-iodo-5-methoxypyridine 29c

Compound **29b** (1.0 g, 3.3 mmol) was dissolved in methanol (10 mL), potassium tert-butoxide (740 mg, 6.6 mmol) was added at room temperature, the reaction was carried out for 2 hours by heating and refluxing, and the reaction was complete shown by TLC. The reaction solution was poured into water and extracted with ethyl acetate, the organic phases were combined, washed with saturated salt water, dried over anhydrous sodium sulfate and concentrated, and the crude product was separated and purified by silica gel column chromatography to obtain the yellow solid title compound **29c** (1.1 g, yield 106%).

### Step 3: 3-(2-bromo-5-methoxypyridin-4-yl)-5,5-dime thyl-5,6-dihydro-4H-pyrrolo[1,2-b]pyrazole 29d

Compound **29c** (200 mg, 0.64 mol) was dissolved in a mixed solvent of 1,4-dioxane (6 mL) and water (2 mL), sodium carbonate (201 mg, 1.90 mmol) was added at room temperature, Intermediate **IN-1** (200 mg, 0.77 mmol) and tetrakis(triphenylphosphine)palladium (70 mg, 0.06 mmol) were added at room temperature, the temperature was lifted to 90°C for reaction for 2 hours under nitrogen protection, and the reaction was complete shown by TLC. The reaction solution was cooled to room temperature, poured into water and extracted with ethyl acetate, the organic phases were combined, washed with saturated salt water, dried over anhydrous sodium sulfate and concentrated, and the crude product was separated and purified by silica gel column chromatography to obtain the light yellow solid title compound **29d** (110 mg, yield 54%). LC-MS: m/z=324.1[M+H]⁺

### Step 4: ((1R,3S)-3-((4-(5,5-dimethyl-5,6-dihydro-4H-pyrrolo[1,2-b]pyrazol-3-yl)-5-methoxypyridin-2-yl) carbamoyl)cyclohexyl)carbamic acid tert-butyl ester 29e

Compound **29d** (110 mg, 0.34 mol) was dissolved in 1,4-dioxane (5 mL), cesium carbonate (332 mg, 1.02 mmol), Compound **10c** (99 mg, 0.41 mmol), tetrakis(triphenylphosphine)palladium (35 mg, 0.03 mmol) and Xphos (17 mg, 0.03 mmol) were added at room temperature, the temperature was lifted to 90°C for reaction for 2 hours under nitrogen protection, and the reaction was complete shown by TLC. The reaction solution was cooled to room temperature, poured into water and extracted with ethyl acetate, the organic phases were combined, washed with saturated salt water, dried over anhydrous sodium sulfate and concentrated, and the crude product was separated and purified by silica gel column chromatography to obtain the light yellow solid title compound **29e** (105 mg, yield 64%).

LC-MS: m/z=484.4[M+H]⁺

### Step 5: (1S,3R)-3-amino-N-(4-(5,5-dimethyl-5,6-dihydro-4H-pyrrolo[1,2-b]pyrazol-3-yl)-5-methoxypyrid in-2-yl)cyclohexane-1-carboxamide 29f

Compound **29e** (65 mg, 0.13 mmol) was dissolved in dichloromethane (5 mL), trifluoroacetic acid (1 mL) was added, and the reaction was carried out for 1 hour at room temperature. The reaction solution was concentrated, adjusted to be pH weakly alkaline with a saturated sodium bicarbonate solution and extracted with ethyl acetate, and the organic phases were combined, washed with saturated salt water, dried over anhydrous sodium sulfate and concentrated to obtain the yellow solid title compound **29f** (55 mg, crude product), which was directly used for the next step.

### Step 6: (1S,3R)-3-acetamido-N-(4-(5,5-dimethyl-5,6-dihydro-4H-pyrrolo[1,2-b]pyrazol-3-yl)-5-methoxyp yridin-2-yl)cyclohexane-1-carboxamide 29

Compound **29f** (55 mg, crude product) was dissolved in dichloromethane (5 mL), triethylamine (24 mg, 0.24 mmol) and acetic anhydride (18 mg, 0.18 mmol) were added, and the reaction was carried out at room temperature for 1 hour. The reaction solution was poured into water and extracted with ethyl acetate, the organic phases were combined, washed with saturated salt water, dried over anhydrous sodium sulfate and concentrated, and the crude product was separated and purified by silica gel column chromatography to obtain the yellow solid title compound **29** (21 mg, two-step yield 37%)

LC-MS: m/z=426.3[M+H]⁺

¹H NMR (400 MHz, DMSO-*d₆*) δ10.25 (s, 1H), 8.18 (s, 1H), 8.05 (s, 1H), 7.93 (s, 1H), 7.77 (d, *J* = 8.0 Hz, 1H), 3.91 (s, 3H), 3.90 (s, 2H), 3.64-3.45 (m, 1H), 2.89 (s, 2H), 2.62-2.54 (m, 1H), 1.89-1.87 (m, 1H), 1.78-1.72 (m, 6H), 1.32-1.23 (m, 9H), 1.09-1.06 (m, 1H). (97.28% purity by HPLC)

### Embodiment 30

### (1S,3R)-3-acetamido-N-(4-(5,5-dimethyl-5,6-dihydro-4H-pyrrolo[1,2-b]pyrazol-3-yl)-5-(trifluoro methyl)pyridin-2-yl)cyclohexane-1-carboxamide 30

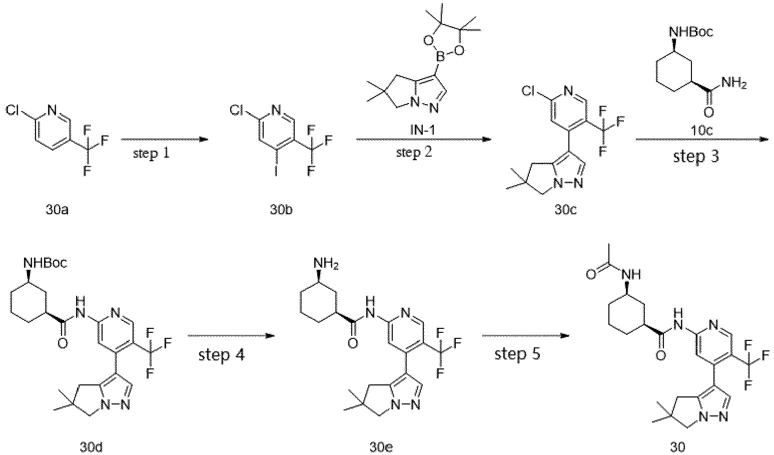

### Step 1: 2-chloro-4-iodo-5-(trifluoromethyl)pyridine 30b

2-chloro-5-(trifluoromethyl)pyridine **30a** (10.0 g, 55.1 mmol) was dissolved in dry tetrahydrofuran (100 mL), under nitrogen protection, the temperature was lowered to -65°C, it was stirred for 5 minutes, lithium diisopropylamide (36 mL, 72 mmol, 2M tetrahydrofuran solution), it was stirred at -65°C for 2 hours, then iodine (15.4 g, 60.6 mmol) in tetrahydrofuran (10 mL) solution were added, it was stirred at -65°C for 15 minutes, the temperature was slowly lifted to room temperature, it was stirred for 1 hour, and the reaction was complete shown by TLC. The reaction solution was poured into water and extracted with ethyl acetate, the organic phases were combined, washed with saturated salt water, dried over anhydrous sodium sulfate and concentrated, and the crude product was separated and purified by silica gel column chromatography to obtain the yellow solid title compound **30b** (4.0 g, yield 24%).

¹H NMR (400 MHz, CDCl₃) δ 8.55 (m, 1H), 8.03 (s, 1H).

### Step 2: 3-(2-chloro-5-(trifluoromethyl)pyridin-4-yl)-5,5-dimethyl-5,6-dihydro-4H-pyrrolo[1,2-b]pyrazole 30c

Compound **30b** (300 mg, 0.98 mmol) was dissolved in 1,4-dioxane (5 mL) and water (2.5 mL), sodium carbonate (318 mg, 3.00 mmol), Intermediate **IN-1** (288 mg, 1.1 mmol) and tetrakis(triphenylphosphine)palladium (116 mg, 0.1 mmol) were added at room temperature, the temperature waslifted to 100°C for reaction for 1 hour under nitrogen protection, and the reaction was complete shown by TLC. The reaction solution was cooled to room temperature, poured into water and extracted with ethyl acetate, the organic phases were combined, washed with saturated salt water, dried over anhydrous sodium sulfate and concentrated, and the crude product was separated and purified by silica gel column chromatography to obtain the white solid title compound **30c** (171 mg, yield 56%).

LC-MS: m/z=316.1[M+H]⁺

¹H NMR (400 MHz, DMSO-*d₆*) δ 8.68 (s, 1H), 7.70 (s, 1H), 7.27 (s, 1H), 3.97 (s, 2H), 2.80 (s, 2H), 1.34 (s, 6H).

### Step 3: ((1R,3S)-3-((4-(5,5-dimethyl-5,6-dihydro-4H-pyrrolo[1,2-b]pyrazol-3-yl)-5-(trifluoromethyl)pyrid in-2-yl)carbamoyl)cyclohexyl)carbamic acid tert-butyl ester 30d

Compound **30c** (130 mg, 0.41 mmol) was dissolved in 1,4-dioxane (5 mL), cesium carbonate (406 mg, 1.25 mmol), Compound **10c** (83 mg, 0.34 mmol), tetrakis(triphenylphosphine)palladium (24 mg, 0.02 mmol) and Xphos (12 mg, 0.02 mmol) were added at room temperature, the temperature was lifted to 90°C for reaction for 2 hours under nitrogen protection, and the reaction was complete shown by TLC. The reaction solution was cooled to room temperature, poured into water and extracted with ethyl acetate, the organic phases were combined, washed with saturated salt water, dried over anhydrous sodium sulfate and concentrated, and the crude product was separated and purified by silica gel column chromatography to obtain the light yellow solid title compound **30d** (110 mg, yield 62%).

### Step 4: (1S,3R)-3-amino-N-(4-(5,5-dimethyl-5,6-dihydro-4H-pyrrolo[1,2-b]pyrazol-3-yl)-5-(trifluorometh yl)pyridin-2-yl)cyclohexane-1-carboxamide 30e

Compound **30d** (110 mg, 0.21 mmol) was dissolved in dichloromethane (5 mL), trifluoroacetic acid (1 mL) was added, the reaction was carried out for 1 hour at room temperature, and the reaction was complete shown by TLC. The reaction solution was concentrated, adjusted to pH=8-9 with a saturated sodium bicarbonate aqueous solution and extracted with ethyl acetate, and the organic phases were combined, washed with saturated salt water, dried over anhydrous sodium sulfate and concentrated to obtain the title compound **30e** (105 mg, crude product), which was directly used for the next step.

### Step 5: (1S,3R)-3-acetamido-N-(4-(5,5-dimethyl-5,6-dihydro-4H-pyrrolo[1,2-b]pyrazol-3-yl)-5-(trifluoro methyl)pyridin-2-yl)cyclohexane-1-carboxamide 30

Compound **30e** (50 mg, crude product) was dissolved in dichloromethane (5 mL), triethylamine (24 mg, 0.24 mmol) and acetic anhydride (18 mg, 0.18 mmol) were added and the reaction was carried out at room temperature for 1 hour. The reaction solution was poured into water and extracted with ethyl acetate, the organic phases were combined, washed with saturated salt water, dried over anhydrous sodium sulfate and concentrated, and the crude product was purified by Prep-TLC to obtain the yellow solid title compound **30** (21.5 mg, two-step yield 46%).

LC-MS: m/z=464.3[M+H]⁺

¹H NMR (400 MHz, DMSO-*d₆*) δ10.87 (s, 1H), 8.65 (s, 1H), 8.22 (s, 1H), 7.76 (d, *J* = 7.6 Hz, 1H), 7.61 (s, 1H), 3.94 (s, 2H), 3.61-3.51 (m, 1H), 2.82 (s, 2H), 2.67-2.63 (m, 1H), 1.92-1.89 (m, 1H), 1.83-1.71 (m, 6H), 1.31-1.23 (m, 9H), 1.12-1.07(m, 1H). (98.59% purity by HPLC)

### Embodiment 31

### 5-(2-((1S,3R)-3-acetylaminocyclohexane-1-carboxamido)-5-fluoropyridin-4-yl)-2,2-dimethyl-2,3-dihydro-1H-pyrrolizine-7-carboxamide 31

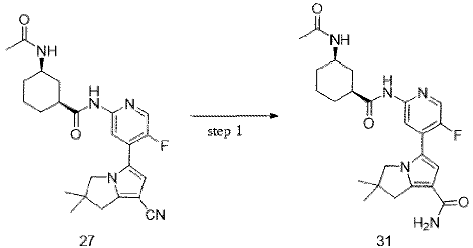

Compound **27** (50 mg, 0.11 mmol) was dissolved in dimethyl sulfoxide (5 mL), anhydrous potassium carbonate (31 mg, 0.22 mmol) and hydrogen peroxide aqueous solution (2 mL, 30%) were added at room temperature, the reaction was carried out for 30 minutes at room temperature, and the reaction was complete shown by TLC. The reaction solution was cooled to room temperature, added with water and extracted with ethyl acetate, the organic phases were combined, washed with saturated salt water, dried over anhydrous sodium sulfate and concentrated, and the crude product was separated and purified by Prep-TLC to obtain the white solid title compound **31** (26 mg, yield 50%).

LC-MS: m/z=456.3 [M+H]⁺

¹H NMR (400 MHz, DMSO-*d₆*) δ 10.55 (s, 1H), 8.34-8.31 (m, 2H), 7.80 (d, *J* = 7.6 Hz, 1H), 7.33 (s, 1H), 7.12 (d, J=2.4 Hz, 1H), 6.79 (s, 1H), 3.93 (s, 2H), 3.61-3.53 (m, 1H), 2.88 (s, 2H), 2.65-2.59 (m, 1H), 1.90-1.78 (m, 7H), 1.37-1.28 (m, 3H), 1.20 (s, 6H), 1.13-1.05 (m, 1H). (94.83% purity by HPLC)

### Embodiment 32

### (3aR,5s,6aS)-2-acetyl-N-(4-(5-carbamoyl-2,2-dimethyl-2,3-dihydro-1H-pyrrolizin-7-yl)pyridin-2-yl)octahydrocyclopenta[c]pyrrole-5-carboxamide (assumed) 32-1

### (3aR,5r,6aS)-2-acetyl-N-(4-(5-carbamoyl-2,2-dimethyl-2,3-dihydro-1H-pyrrolizin-7-yl)pyridin-2-yl)octahydrocyclopenta[c]pyrrole-5-carboxamide (assumed) 32-2

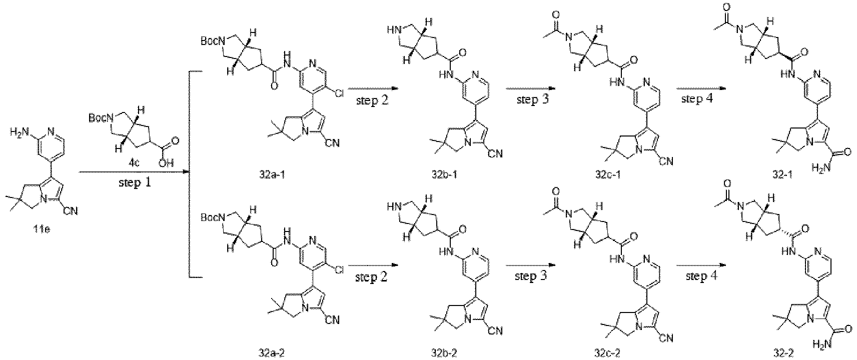

### Step 1: (3aR,6aS)-5-((5-chloro-4-(5-cyano-2,2-dimethyl-2,3-dihydro-1H-pyrrolizin-7-yl)pyridin-2-yl)carb amoyl)hexahydrocyclopenta[c]pyrrole-2(1H)-carboxylic acid tert-butyl ester 32a-1,2

Compound **11e** (900 mg, 3.52 mmol) and Compound **4c** (1.01 g, 3.52 mmol) were dissolved in ethyl acetate (20 mL), 1-propylphosphonic anhydride (9.0 g, 14.14 mmol, 50% ethyl acetate solution) and pyridine (1.67 g, 21.11 mmol) were added at room temperature, the temperature was lifted to 80°C for reaction overnight, and a small amount of the starting material was remained.monitored by TLC. The reaction solution was added with water and extracted with ethyl acetate, the organic phases were combined, washed with water, washed with saturated salt water, dried over anhydrous sodium sulfate and concentrated, and the crude product was chromatographed on silica gel column to obtain the low-polarity title compound **32a-1** (470 mg, yield 27%), and the high-polarity title compound **32a-2** (500 mg, yield 29%).

### Step 2: (3aR,6aS)-N-(4-(5-cyano-2,2-dimethyl-2,3-dihydro-1H-pyrrolizin-7-yl)pyridin-2-yl)octahydrocycl openta[c]pyrrole-5-carboxamide 32b-1,2

Compound **32a-1** (120 mg, 0.25 mmol) was dissolved in dichloromethane (6 mL), trifluoroacetic acid (2 mL) was added, the reaction was carried out for 1 hour at room temperature, and the reaction was complete monitored by TLC. The reaction solution was added with a saturated sodium bicarbonate solution to adjust the alkalinity and extracted with dichloromethane, and the organic phases were combined, washed with water, washed with saturated salt water, dried over anhydrous sodium sulfate and concentrated to obtain the title compound **32b-1** (90 mg, crude product), which was directly used for the next step.

Compound **32a-2** (150 mg, 0.31 mmol) was dissolved in dichloromethane (6 mL), trifluoroacetic acid (2 mL) was added, the reaction was carried out for 1 hour at room temperature, and the reaction was complete monitored by TLC. The reaction solution was added with a saturated sodium bicarbonate solution to adjust the alkalinity and extracted with dichloromethane, and the organic phases were combined, washed with water, washed with saturated salt water, dried over anhydrous sodium sulfate and concentrated to obtain the title compound **32b-2** (120 mg, crude product), which was directly used for the next step.

### Step 3: (3aR,6aS)-2-acetyl-N-(4-(5-cyano-2,2-dimethyl-2,3-dihydro-1H-pyrrolizin-7-yl)pyridin-2-yl)octah ydrocyclopenta[c]pyrrole-5-carboxamide 32c-1,2

Compound **32b-1** (90 mg, crude product) was dissolved in tetrahydrofuran (8 mL), acetic anhydride (31 mg, 0.30 mmol) and triethylamine (35 mg, 0.35 mmol) were added, the reaction was carried out for 2 hours at room temperature, and the reaction was complete monitored by TLC. The reaction solution was added with water and extracted with ethyl acetate, the organic phases were combined, washed with water, washed with saturated salt water, dried over anhydrous sodium sulfate and concentrated, and the crude product was purified by crude Prep-TLC to obtain the title compound **32c-1** (50 mg, two-step yield 50%).

Compound **32b-2** (120 mg, crude product) was dissolved in tetrahydrofuran (8 mL), acetic anhydride (41 mg, 0.40 mmol) and triethylamine (47 mg, 0.46 mmol) were added, the reaction was carried out for 2 hours at room temperature, and the reaction was complete monitored by TLC. The reaction solution was added with water and extracted with ethyl acetate, and the organic phases were combined, washed with water, washed with saturated salt water, dried over anhydrous sodium sulfate and concentrated to obtain the title compound **32c-2** (100 mg, crude product), which was directly used for the next step.

### Step 4: (3aR,5s,6aS)-2-acetyl-N-(4-(5-carbamoyl-2,2-dimethyl-2,3-dihydro-1H-pyrrolizin-7-yl)pyridin-2-yl)octahydrocyclopenta[c]pyrrole-5-carboxamide (assumed) 32-1 &

### (3aR,5r,6aS)-2-acetyl-N-(4-(5-carbamoyl-2,2-dimethyl-2,3-dihydro-1H-pyrrolizin-7-yl)pyridin-2-yl)octahydrocyclopenta[c]pyrrole-5-carboxamide (assumed) 32-2

Compound **32c-1** (50 mg, 0.12 mmol) was dissolved in dimethyl sulfoxide (6 mL), hydrogen peroxide (1 mL, 30%) and potassium carbonate (33 mg, 0.24 mmol) were added, the reaction was carried out at room temperature for 1 hour, and the reaction was complete monitored by TLC. The reaction solution was added with water and extracted with ethyl acetate, the organic phases were combined, washed with saturated salt water, dried over anhydrous sodium sulfate and concentrated, and the crude product was purified by Prep-TLC to obtain the title compound **32-1** (25 mg, yield 48%).

LC-MS: m/z=450.1 [M+H]⁺

¹H NMR(400 MHz, DMSO-*d₆*) δ 10.34 (s, 1H), 8.24 (s, 1H), 8.19 (d, *J* = 4.0 Hz, 1H), 7.6 (s, 1H), 7.33 (s, 1H), 7.06 (d, *J* = 4.0 Hz, 1H), 6.95 (s, 1H), 4.00 (s, 2H), 3.67-3.63 (m, 1H), 3.53-3.48 (m, 1H), 3.25-3.14 (m, 3H), 2.88 (s, 2H), 2.85-2.72 (m, 2H), 2.03-1.94 (m, 5H), 1.82-1.72 (m, 2H), 1.23 (s, 6H). (92.06% purity by HPLC)

Compound **32c-2** (100 mg, crude product) was dissolved in dimethyl sulfoxide (8 mL), hydrogen peroxide (1 mL, 30%) and potassium carbonate (64 mg, 0.46 mmol) were added, the reaction was carried out for 1 hour at room temperature, and the reaction was complete monitored by TLC. The reaction solution was added with water, extracted with ethyl acetate, the organic phases were combined, washed with water, washed with saturated salt water, dried over anhydrous sodium sulfate and concentrated, and the crude product was purified by Prep-TLC to obtain the title compound **32-2** (55 mg, three-step yield 40%).

LC-MS: m/z=450.1 [M+H]⁺

¹H NMR(400 MHz, DMSO-*d₆*) δ 10.36 (s, 1H), 8.26 (s, 1H), 8.19 (d, *J* = 4.0 Hz, 1H), 7.6 (s, 1H), 7.33 (s, 1H), 7.06 (dd, *J* = 2.0, 5.2Hz, 1H), 6.92 (s, 1H), 3.99 (s, 2H), 3.59-3.55 (m, 1H), 3.43-3.34 (m, 2H), 3.30-3.26 (m, 1 H), 3.13-3.09 (m, 1H), 2.88 (s, 2H), 2.73-2.59 (m, 2H), 2.18-2.10 (m, 2H), 1.94 (s, 3H), 1.79-1.68 (m, 2H), 1.23 (s, 6H). (91.29% purity by HPLC)

### Embodiment 33

### 7-(2-((1S,3R)-3-acetylaminocyclohexane-1-carboxamido)-5-fluoropyridin-4-yl) -2,2-dimethyl -2,3-dihydro-1H-pyrrolizine-5-carboxamide 33

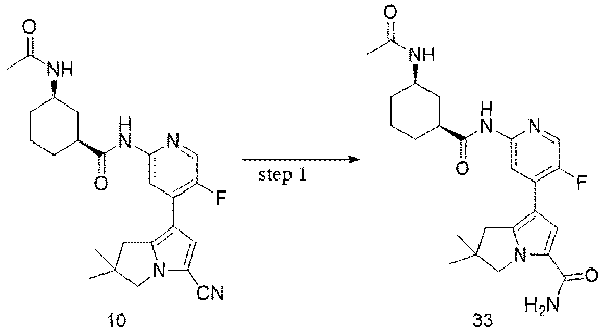

Compound **10** (58 mg, 0.13 mmol) was dissolved in dimethyl sulfoxide (1 mL), hydrogen peroxide (0.5 mL, 30%) and potassium carbonate (36 mg, 0.26 mmol) were added, and the reaction was carried out at room temperature for 1 hour. The reaction was complete shown by TLC. The reaction solution was added with water, extracted with ethyl acetate, the organic phases were combined, washed with saturated salt water, dried over anhydrous sodium sulfate and concentrated, and the crude product was purified by Prep-TLC to obtain the title compound 33 (26 mg, yield 44%).

LC-MS: m/z=456.3[M+H]⁺

¹H NMR (400 MHz, DMSO-*d₆*) δ 10.46 (s, 1H), 8.30 (d, J = 6.4 Hz, 1H), 8.24 (d, J = 2.8 Hz, 1H), 7.80 (d, J = 7.6 Hz, 1H), 7.69 (br, 1H), 7.33 (d, J = 2.0 Hz, 1H), 6.99 (br, 1H), 4.03 (s, 2H), 3.60-3.56 (m, 1H), 2.84 (s, 2H), 2.64-2.57 (m, 1H), 1.90-1.87 (m, 1H), 1.78 (s, 6H), 1.30-1.22 (m, 9H), 1.10-1.07 (m, 1H). (95.94% purity by HPLC)

### Embodiment 34

### 7-(2-((2r,3aR,5s,6aS)-5-acetylaminooctahydropentalene-2-carboxamido)-5-fluoropyridin-4-yl)-2,2 -dimethyl-2,3-dihydro-1H-pyrrolizine-5-carboxamide (assumed) 34-1

### 7-(2-((2s,3aR,5r,6aS)-5-acetylaminooctahydropentalene-2-carboxamido)-5-fluoropyridin-4-yl)-2,2 -dimethyl-2,3-dihydro-1H-pyrrolizine-5-carboxamide (assumed) 34-2

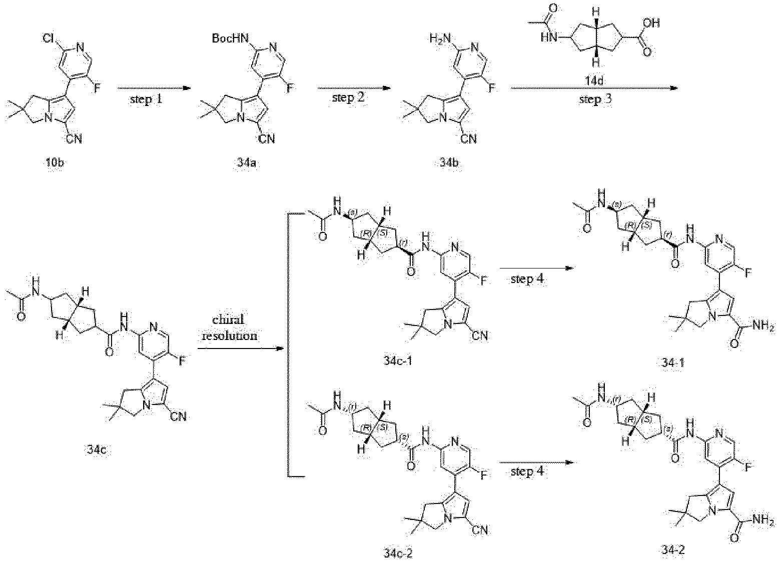

### Step 1: 4-(5-cyano-2,2-dimethyl-2,3-dihydro-1H-pyrrolizin-7-yl)-5-fluoropyridin-2-yl)carbamic acid tert-butyl ester 34a

Under nitrogen protection, Compound **10b** (1.12 g, 3.87 mmol) was dissolved in 1,4-dioxane (20 mL), tert-butyl carbamate (1.13 g, 9.68 mmol), cesium carbonate (2.51 g, 7.74 mmol), Pd₂(dba)₃ (284 mg, 0.31 mmol) and XantPhos (365 mg, 0.63 mmol) were added at room temperature, the temperature was lifted to 100°C for reaction for 4 hours, and the reaction was complete shown by TLC. The reaction solution was cooled to room temperature, diluted with water and extracted with ethyl acetate, the organic phases were combined, washed with saturated salt water, dried over anhydrous sodium sulfate and concentrated, and the crude product was purified by silica gel column chromatography to obtain the title compound **34a** (1.02 g, yield 71%).

### Step 2: 7-(2-amino-5-fluoropyridin-4-yl)-2,2-dimethyl-2,3-dihydro-1H-pyrrolizine-5-carbonitrile 34b

Compound **34a** (1.02 g, 2.74 mmol) was dissolved in dichloromethane (5 mL), trifluoroacetic acid (10 mL) was added, the reaction was carried out for 1 hour at room temperature, and the reaction was complete shown by TLC. The reaction solution was concentrated to remove trifluoroacetic acid, added with a saturated sodium bicarbonate solution to adjust the pH to be alkaline and extracted with dichloromethane, and the organic phases were combined, washed with saturated salt water, dried over anhydrous sodium sulfate and concentrated to obtain the title compound **34b** (756 mg, crude product), which was directly used for the next step.

### Step 3: (2r,3aR,5s,6aS)-5-acetamido-N-(4-(5-cyano-2,2-dimethyl-2,3-dihydro-1H-pyrrolizin-7-yl)-5-fluor opyridin-2-yl)octahydropentalene-2-carboxamide 34c-1 &

### (2s,3aR,5r,6aS)-5-acetamido-N-(4-(5-cyano-2,2-dimethyl-2,3-dihydro-1H-pyrrolizin-7-yl)-5-fluor opyridin-2-yl)octahydropentalene-2-carboxamide 34c-2

Compound **14d** (160 mg, 0.75 mmol) and Compound **34b** (200 mg, crude product) were dissolved in ethyl acetate (10 mL), triethylamine (300 mg, 2.96 mmol) and 1-propylphosphonic anhydride (2.8 g, 4.10 mmol, 50% ethyl acetate solution) were added in sequence at room temperature, the temperature was lifted to 60°C, it was stirred for 2 hours, and the complete reaction of the starting material was shown by TLC. The reaction solution was cooled to room temperature, quenched with water and extracted with ethyl acetate, the organic phases were combined, washed with saturated salt water, dried over anhydrous sodium sulfate, and concentrated to obtain the title compound **34c** (180 mg, crude product). Chiral resolution (DAICEL AD-H, 20*250mm, 5um, 30mL/min, IPA:Hexane=20:80) to obtain Compound **34c-1** (RT 26.83 min) (75 mg, yield 42%) and Compound **34c-2** (RT 32.47 min) (30 mg, yield 17%).

### Step 4: 7-(2-((2r,3aR,5s,6aS)-5-acetylaminooctahydropentalene-2-carboxamido)-5-fluoropyridin-4-yl)-2,2 -dimethyl-2,3-dihydro-1H-pyrrolizine-5-carboxamide (assumed) 34-1 &

### 7-(2-((2s,3aR,5r,6aS)-5-acetylaminooctahydropentalene-2-carboxamido)-5-fluoropyridin-4-yl)-2,2 -dimethyl-2,3-dihydro-1H-pyrrolizine-5-carboxamide (assumed) 34-2

Compound **34c -1** (75 mg, 0.16 mmol) was dissolved in dimethyl sulfoxide (6 mL), hydrogen peroxide (1 mL, 30%) and potassium carbonate (45 mg, 0.32 mmol) were added, the reaction was carried out for 1 hour at room temperature, and the reaction was complete shown by TLC. The reaction solution was added with water and extracted with ethyl acetate, the organic phases were combined, washed with water, washed with saturated salt water, dried over anhydrous sodium sulfate and concentrated, and the crude product was purified by Prep-TLC to obtain the title compound **34-1** (8.0 mg, yield 10.3%).

LC-MS: m/z=482.3 [M+H]⁺

¹H NMR (400 MHz, DMSO-*d₆*) δ 10.44 (s, 1H), 8.33 (d, *J* = 6.0 Hz, 1H), 8.24 (d, *J* = 2.8 Hz, 1H), 7.90 (d, *J* = 7.6 Hz, 1H), 7.68 (s, 1H), 7.33 (d, *J* = 2.4 Hz, 1H), 6.99 (s, 1H), 4.03 (s, 3H), 3.06-2.94 (m, 1H), 2.85 (s, 2H), 2.43-2.90 (m, 2H), 2.22-1.99 (m, 4H), 1.91 (s, 2H), 1.76 (s, 3H), 1.58-1.45 (m, 2H), 1.34-1.08 (m, 6H). (98.98% purity by HPLC)

Compound **34c-2** (30 mg, 0.074 mmol) was dissolved in dimethyl sulfoxide (6 mL), hydrogen peroxide (1 mL, 30%) and potassium carbonate (21 mg, 0.15 mmol) were added, the reaction was carried out for 1 hour at room temperature, and the reaction was complete shown by TLC. The reaction solution was added with water and extracted with ethyl acetate, the organic phases were combined, washed with water, washed with saturated salt water, dried over anhydrous sodium sulfate and concentrated, and the crude product was purified by Prep-TLC to obtain the title compound **34-2** (4.0 mg, yield 12.8%).

LC-MS: m/z=482.3 [M+H]⁺

¹H NMR (400 MHz, DMSO-*d₆*) δ 10.55 (s, 1H), 8.32 (d, *J* = 6.0 Hz, 1H), 8.24 (d, *J* = 2.8 Hz, 1H), 7.88 (d, *J* = 7.2 Hz, 1H), 7.69 (s, 1H), 7.32 (d, *J* = 2.0 Hz, 1H), 6.98 (s, 1H), 4.03 (s, 2H), 3.77-3.68 (m, 1H), 3.10-2.99 (m, 1H), 2.84 (s, 2H), 2.62-2.38 (m, 2H), 2.12-2.02 (m, 2H), 1.85-1.72 (m, 5H), 1.70-1.61 (m, 2H), 1.21 (s, 6H), 1.07-0.94 (m, 2H). (98.87% purity by HPLC)

### Test Example 1: Test of Inhibitory Effect of the Compounds on the Kinase Activity of CDK9 in Vitro

The detection of the inhibitory effect of the compounds of the present disclosure on the kinase activity of CDK9 in vitro was determined by the following method:
Compound preparation: The compound was accurately weighed and dissolved in DMSO (Sigma, D2650) to a concentration of 100 um for later use. The compound was diluted to 5-fold of a desired highest concentration, and diluted 4-fold into 6 concentration gradients, allowing the final concentration of the compound in the reaction system to be 100, 25, 6.25, 1.56, 0.39, 0.098 nM. 5 ul was taken to a 384-well plate (Corning, 4512).

Kinase reaction: 10uL CDK9 kinase (Millipore, 14-685M) solution was added to the 384-well plate (Corning, 4512) containing the compound, and allowed to stand at room temperature for 10 min; a mixed solution of 10 uL ATP(Sigma, A7699) and 0.2ug/uL CTD3 substrate polypeptide (GL Biochem, SY356885) was added, and allowed to stand at 28°C for a short time; and then a stop solution was added to terminate the reaction at 25 uL per well.

Detection: Data was collected by Caliper.

Calculation: The IC50 value was calculated by Graphpad prism 5.0 software according to the concentration of the compound and the corresponding signal value. Test Results: See Table 1. Conclusion: The compounds of the present disclosure have a significant inhibitory effect on CDK9 kinase activity.

### Test Example 2: Inhibition Effect of the Compounds on the Proliferation of Mv4-11 Cells

The inhibition effect of the compounds of the present disclosure on the proliferation of Mv4-11 cells (ATCC:CRL-9591TM) in vitro was determined by the following method:
Cell inoculation: Mv4-11 cells with good growing state in logarithmic growth phase were inoculated into a 96-well plate at 20000 cells per well and 50 uL, and cultured at 37°C and under 5% CO₂ condition for 2-4 h.

Administration: The compound was diluted with 1640 medium containing 10% FBS and 1% PS: 0.4 uL of the compound with an initial concentration of 1 mM was placed in a dilution plate, each added with 199.6 uL of the above culture medium, and gradiently diluted 4-fold, so that the final concentration in the added cell was 1000, 250, 62.5, 15.625, 3.91, 0.98, 0.24, 0.06 nM. 50 µL of the medium containing the compound was sequentially added, and it was placed in a 37°C, 5% CO₂ cell incubator for 48 hours.

Detection: 10uL CCK8 (Dojindo, CK 04) solution was added to each well, incubated in 37°C. 5% CO₂ cell incubator for 2 hours, and then the OD450 value was read by Synegry H1(BioTek) multimode microplate reader.

Calculation: The IC50 value was calculated using Graphpad prism 5.0 software according to the concentration of the compound and the corresponding signal value. Test Results: See Table 1. Meanwhile, in order to better reflect the inhibitory activity of the compounds provided by the embodiments of the present disclosure on CDK9 enzyme, the method of Test Examples 1 and 2 was used in the present disclosure to test the IC50 of CDK9 inhibitor AZD4573, and the results are shown in Table 1.

**TABLE 1 Inhibition of the Compounds of the Present Disclosure on Kinase Activity of CDK9 IC50(nM)**

| Compound No. | CDK9 | Mv4-11 | Compound No. | CDK9 | Mv4-11 |
|---|---|---|---|---|---|
| AZD4573 | 2.8 | 4.2 | 17 | 4.9 | 36.1 |
| 1 | 2.1 | 1.6 | 18-1 | 3.2 | 1.4 |
| 2 | 2.8 | 27.6 | 20-2 | 7.0 | 15.0 |
| 4-1 | - | 14.8 | 20-3 | >100 | >1000 |
| 4-2 | 2.9 | 6.8 | 20-4 | 2.8 | 0.5 |
| 5 | 2.8 | 7.6 | 21-1 | - | 68.5 |
| 6 | 3.7 | 5.4 | 21-2 | - | 16.2 |
| 7-1 | 3.2 | 17.5 | 21-3 | 2.8 | 6.3 |
| 7-2 | - | 8.6 | 21-4 | - | 71.9 |
| 8-1 | 3.5 | 7.7 | 22-1-1 | 3.2 | 2.8 |
| 8-2 | 3.1 | 0.4 | 23-1 | 3.8 | 7.9 |
| 10 | - | 26.9 | 24-1 | 3.8 | 5.9 |
| 11 | - | 2.2 | 26-1 | 2.9 | 4.1 |
| 12 | 2.9 | 26.0 | 26-2 | 3.5 | 5.1 |
| 14-1-1 | - | 23.9 | 27 | 2.2 | 3.5 |
| 14-1-2 | - | 11.1 | 28 | - | 36.5 |
| 14-2-1 | 4.3 | 10.6 | 30 | 6.3 | 15.3 |
| 14-2-2 | 10.4 | 44.6 | 31 | 4.8 | 9.2 |
| 15-1 | 10.1 | 7.9 | 32-1 | 4.8 | 3.5 |
| 15-2 | 3.7 | 14.3 | 32-2 | 2.8 | 0.9 |
| 16 | 2.9 | | 33 | ∼ | 1.3 |
| 19-1 | 5.5 | 12.2 | 34-1 | 4.9 | 2.3 |
| 19-2 | - | 84.2 | 34-2 | ∼ | 9.0 |
| 20-1 | 28.6 | 352 | | | |

Conclusion: The compounds of the present disclosure have a significant inhibitory effect on CDK9, and the inhibitory activity of some of the compounds are significantly better than that of AZD4573.

Test Example 3: Determination of Inhibitory Activity of the Compounds on the phosphorylation of RNA pol II Ser2 in Mv4-11 Cells

The inhibition effect of the compounds of the present disclosure on the in vitro Mv4-11 cell RNA pol II Ser 2 was determined by the following method:
Cell inoculation: Mv4-11 cells with good growing state in logarithmic growth phase were inoculated into a six-well plate with 2*105 cells/well, and cultured overnight at 37°C and under 5% CO₂ condition.

Administration: The compound was diluted with 2mL of 1640 medium containing 10% FBS and 1% PS. 2 ul of the medium containing the compound was added to the overnight cultured cells, and it was placed in a 37° C, 5% CO₂ cell incubator for 6 hours.

Protein extraction and quantification: Cell suspension was collected and centrifuged at 1000 g for 5 min, the cell culturing medium was discarded, and PBS was added for resuspension and centrifugation; it was repeated three times, the residual liquid was sucked dry, 80 uL cell lysate was added per well, it was placed to ice, shaked for 10 min on a shaker, centrifuged at 12000 g for 5 min, and the supernatant, ie, the total protein solution, was collected; the protein concentration was tested by BCA.

12% SDS-PAGE electrophoresis was used to detect, after that trarsmembran was carried out at 100V voltagefor 3h, blocking solution (Beyotime: P0235) was used to block for 15 min, TBST(Sangon Biotech: C520002) was used to wash the membrane three times, and the primary antibody (target protein CST:13499, internal reference Beyotime: AF1186) was incubated at 4°C overnight; the membrane was washed with TBST three times; the secondary antibody (Beyotime: A0208) was incubated at room temperature for 1 hour; the membrane was washed with TBST three times; ECL (Tanon: 180-501) was used for exposure and coloration, and the phosphorylation inhibition effect of compounds 7-1, 12, 22-1-1 and AZD4573 with different concentrations on RNA pol II Ser2 in MV 4-11 cells was shown in FIG. 1.

It can be seen from FIG. 1 that the compounds of the present disclosure, such as 7-1, 12 and 22-1-1, have a significant inhibitory effect on the phosphorylation of RNA pol II Ser 2 in Mv4-11 cells, and the inhibitory activity of some of the compounds are significantly better than that of AZD4573.

The above are merely embodiments of the present disclosure, and the description thereof is relatively specific and detailed, but cannot be understood as a limitation to the patent scope of the present disclosure. It should be noted that a person of ordinary skill in the art may make several variations and improvements without departing from the concept of the present disclosure, and these are all within the protection scope of the present disclosure.

## Claims

1. A polycyclic amide derivative, a pharmaceutically acceptable salt thereof, a tautomer thereof or a stereoisomer thereof, wherein the structure of the polycyclic amide derivative is represented by formula (I): 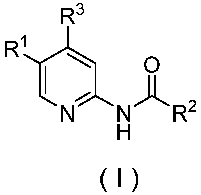
wherein: R¹ is selected from hydrogen, halogen, cyano, substituted or unsubstituted C₁-C₃ alkyl, or substituted or unsubstituted C₁-C₃ alkoxy, wherein "substituted" refers to optionally substituted with 1-3 halogens;
R² is selected from 5-7 membered cycloalkyl, 5-7 membered cycloalkenyl, 7-10 membered fused cycloalkyl, 7-10 membered bridged cycloalkyl, 7-10 membered spirocycloalkyl, 6-7 membered heterocyclyl, 6-7-membered heterocyclenyl, 7-10 membered fused heterocyclyl, 7-10 membered bridged heterocyclyl, and 7-10 membered spiroheterocyclyl, wherein 6-7 membered cycloalkyl, 6-7 membered cycloalkenyl, 7-10 membered fused cycloalkyl, 7-10 membered bridged cycloalkyl, 7-10 membered spirocycloalkyl, 6-7 membered heterocyclyl, 6-7 membered heterocycloalkenyl, 7-10 membered fused heterocyclyl, 7-10 membered bridged heterocyclyl, and 7-10 membered spiroheterocyclyl are optionally substituted with 1-3 R^{a};
R^{a} is selected from C₁-C₃ alkyl, hydroxyl, halogen, cyano, C₁-C₃ alkoxy, 3-7 membered cycloalkyl, 3-7 membered heterocyclyl, phenyl, 5-6 membered heteroaryl, 8-10 membered fused aryl, 8-10 membered fused heteroaryl, =O, NH₂, NHR_{b}, NR^{b}₂, S(O)R^{b}, S(O)₂R^{b}, S(O)NH₂, S(O)NHR^{b}, S(O)N(R^{b})₂, S(O)₂NH₂, S(O)₂NHR^{b}, S(O)₂N(R^{b})₂, NHS(O)R^{b}, NR^{b}S(O)R^{b}, NHS(O)₂R^{b}, NR^{b}S(O)₂R^{b}, C(O)R^{b}, C(O)OR^{b}, OC(O)R^{b}, NHC(O)R^{b}, NR^{b}C(O)R^{b}, NHC(O)OR^{b}, NR^{b}C(O)OR^{b}, C(O)NH₂, C(O)NHR^{b} or C(O)N(R^{b})₂, wherein alkyl, alkoxy, cycloalkyl, heterocyclyl, phenyl, 5-6 membered heteroaryl, 8-10 membered fused aryl, 8-10 membered fused heteroary and amino are optionally further substituted with one or more R^{a1};
R^{b} is independently selected from substituted or unsubstituted C₁-C₃ alkyl, substituted or unsubstituted 3-6 membered cycloalkyl, or substituted or unsubstituted heterocyclyl; wherein "substituted" refers to optionally substituted with 1-3 substituents selected from C₁-C₃ alkyl, hydroxyl, halogen, cyano, amino or alkoxy;
R^{a1} is selected from C₁-C₃ alkyl, hydroxyl, halogen, cyano, amino, C₁-C₃ alkoxy, S(O)R^{b}, S(O)₂R^{b}, S(O)NH₂, S(O)NHR^{b}, S(O)N(R^{b})₂, S(O)₂NH₂, S(O)₂NHR^{b}, S(O)₂N(R^{b})₂, NHS(O)R^{b}, NR^{b}S(O)R^{b}, NHS(O)₂R^{b}, NR^{b}S(O)₂R^{b}, C(O)R^{b}, C(O)OR^{b}, OC(O)R^{b}, NHC(O)R^{b}, NR^{b}C(O)R^{b}, NHC(O)OR^{b}, NR^{b}C(O)OR^{b}, C(O)NH₂, C(O)NHR^{b}, C(O)N(R^{b})₂, wherein alkyl and alkoxy are optionally further substituted with 1-3 halogens, hydroxyl groups, cyano groups, amino groups or alkoxy groups;
R³ is 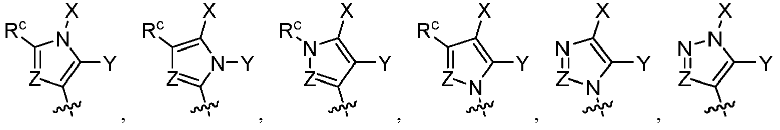 or 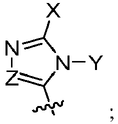
Z is N or CR^{c};
R^{c} is independently selected from H, halogen, CN, C(O)NH₂, C(O)NHR^{b}, C(O)N(R^{b})₂, C(O)R^{b}, substituted or unsubstituted C₁-C₃ alkyl, substituted or unsubstituted 3-6 membered cycloalkyl, or substituted or unsubstituted 4-7 membered heterocyclyl, wherein "substituted" refers to optionally substituted with 1-3 substituents selected from alkyl, hydroxyl, halogen, cyano, amino, or alkoxy; X and Y together with the atoms to which they are bonded form 5-7 membered heterocyclyl or cycloalkyl, wherein heterocyclyl comprises 1-2 heteroatoms selected from N, O, S; 5-7 membered heterocyclyl or cycloalkyl is saturated or partially saturated and the ring carbons therein may be optionally further substituted with 1-3 R^{d};
R^{d} is independently selected from halogen, OH, CN, =O, C₁-C₃ alkyl, 3-6 membered cycloalkyl or heterocyclyl, wherein alkyl, cycloalkyl and heterocyclyl are optionally further substituted with 1-3 substituents selected from alkyl, hydroxyl, halogen, cyano, amino or alkoxy;
when R³ is 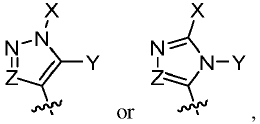 at least one of the following conditions must be satisfied:
(1) R¹ can only be C₁-C₃ alkoxy or C₁-C₃ alkyl substituted with 1-3 halogens;
(2) R² is selected from 6-7 membered heterocyclenyl, 7-10 membered fused cycloalkyl, 7-10 membered bridged cycloalkyl, 7-10 membered spirocycloalkyl, 7-10 membered fused heterocyclyl, 7-10 membered bridged heterocyclyl, and 7-10 membered spiroheterocyclyl, wherein 6-7 membered heterocyclenyl, 7-10 membered fused cycloalkyl, 7-10 membered bridged cycloalkyl, 7-10 membered spirocycloalkyl, 7-10 membered fused heterocyclyl, 7-10 membered bridged heterocyclyl, and 7-10 membered spiroheterocyclyl must be further substituted with 1-3 R^{e} at the same time;
(3) R² is substituted with phenyl, 5-6 membered heteroaryl, 8-10 membered fused aryl or 8-10 membered fused heteroaryl, wherein phenyl, 5-6 membered heteroaryl, 8-10 membered fused aryl or 8-10 membered fused heteroaryl is optionally further substituted with 1-3 R^{a1};
R^{e} is selected from S(O)R^{b}, S(O)₂R^{b}, S(O)NH₂, S(O)NHR^{b}, S(O)N(R^{b})₂, S(O)₂NH₂, S(O)₂NHR^{b}, S(O)₂N(R^{b})₂, NHS(O)₂R^{b}, NR^{b}S(O)₂R^{b}, C(O)R^{b}, C(O)OR^{b}, OC(O)R^{b}, C(O)NH₂, C(O)NHR^{b} or C(O)N(R^{b})₂.

2. The polycyclic amide derivative, the pharmaceutically acceptable salt thereof, the tautomer thereof or the stereoisomer thereof according to claim 1, wherein the structure of the polycyclic amide derivative is represented by formula (II) or formula (III): 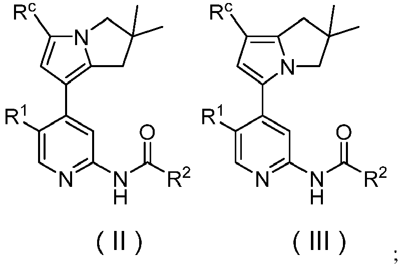 wherein R¹, R², and R^{C} have the same defined ranges as in claim 1.

3. The polycyclic amide derivative, the pharmaceutically acceptable salt thereof, the tautomer thereof or the stereoisomer thereof according to claim 1, wherein the structure of the polycyclic amide derivative is represented by formula (IV): 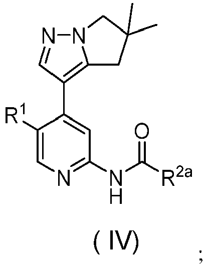 wherein:
R^{2a} is selected from 6-7 membered heterocyclenyl, 7-10 membered fused cycloalkyl, 7-10 membered bridged cycloalkyl, 7-10 membered spirocycloalkyl, 7-10 membered fused heterocyclyl, 7-10 membered bridged heterocyclyl, and 7-10 membered spiroheterocyclyl, wherein 6-7 membered heterocyclenyl, 7-10 membered fused cycloalkyl, 7-10 membered bridged cycloalkyl, 7-10 membered spirocycloalkyl, 7-10 membered fused heterocyclyl, 7-10 membered bridged heterocyclyl, and 7-10 membered spiroheterocyclyl are optionally further substituted with 1-3 R^{e}; R¹ and R^{e} have the same defined ranges as in claim 1.

4. The polycyclic amide derivative, the pharmaceutically acceptable salt thereof, the tautomer thereof or the stereoisomer thereof according to claim 1, wherein the structure of the polycyclic amide derivative is represented by formula (V): 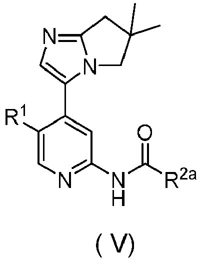 wherein:
R^{2a} is selected from 6-7 membered heterocyclenyl, 7-10 membered fused cycloalkyl, 7-10 membered bridged cycloalkyl, 7-10 membered spirocycloalkyl, 7-10 membered fused heterocyclyl, 7-10 membered bridged heterocyclyl, and 7-10 membered spiroheterocyclyl; wherein 6-7 membered heterocyclenyl, 7-10 membered fused cycloalkyl, 7-10 membered bridged cycloalkyl, 7-10 membered spirocycloalkyl, 7-10 membered fused heterocyclyl, 7-10 membered bridged heterocyclyl, and 7-10 membered spiroheterocyclyl are optionally further substituted with 1-3 R^{e}; R¹ and R^{e} have the same defined ranges as in claim 1.

5. The polycyclic amide derivative, the pharmaceutically acceptable salt thereof, the tautomer thereof or the stereoisomer thereof according to claim 1, wherein the structure of the polycyclic amide derivative is represented by formula (VI): 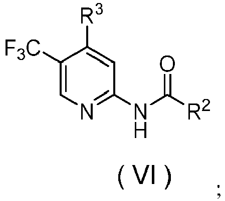 wherein R² and R³ have the same defined range as in claim 1.

6. The polycyclic amide derivative, the pharmaceutically acceptable salt thereof, the tautomer thereof or the stereoisomer thereof according to claim 1, wherein the structure of the polycyclic amide derivative is represented by formula (VII): 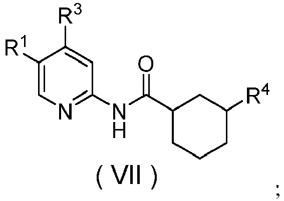 wherein R⁴ is phenyl, 5-6 membered heteroaryl, 8-10 membered fused aryl, or 8-10 membered fused heteroaryl, and R⁴ is optionally further substituted with 1-3 R^{a1}; R¹, R³, and R^{a1} have the same defined range as in claim 1.

7. The polycyclic amide derivative, the pharmaceutically acceptable salt thereof, the tautomer thereof or the stereoisomer thereof according to claim 1, wherein the polycyclic amide derivative is selected from any one of the following structures:

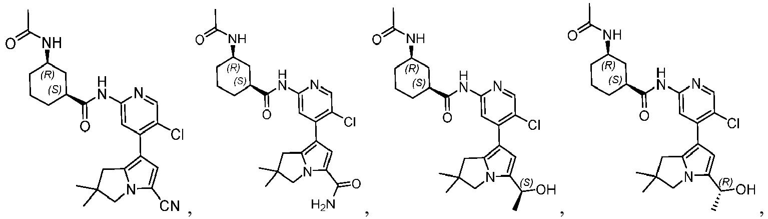

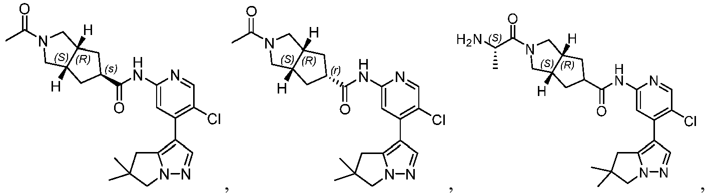

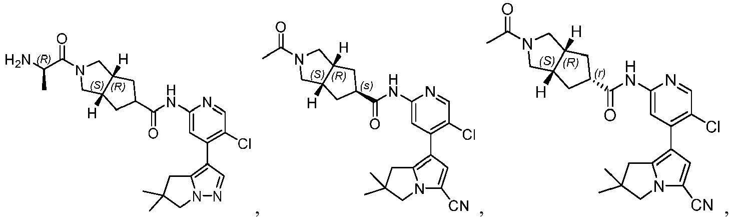

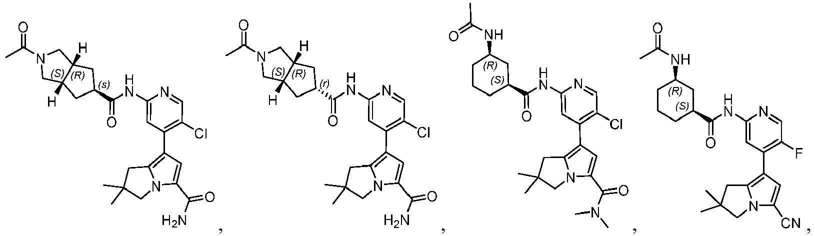

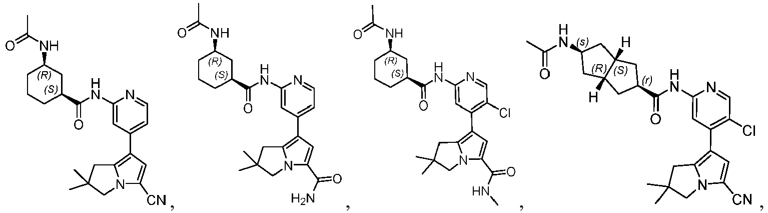

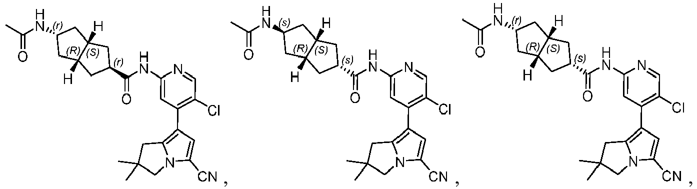

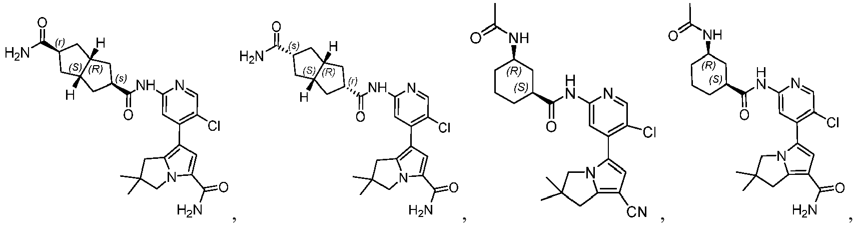

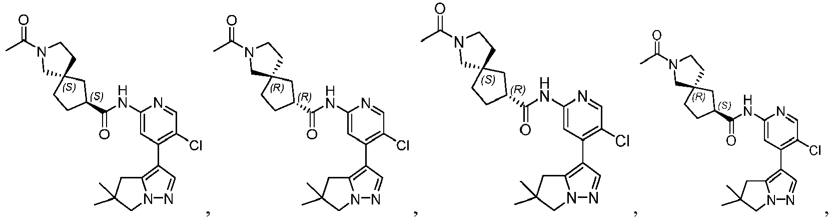

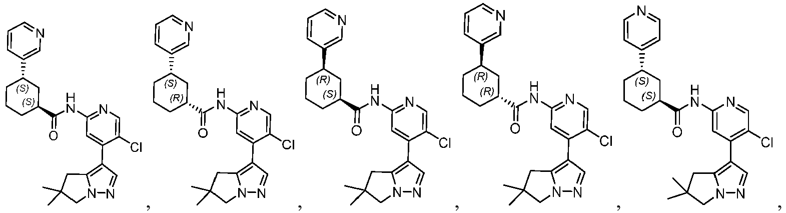

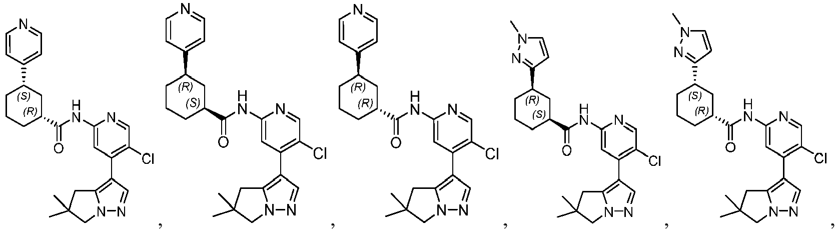

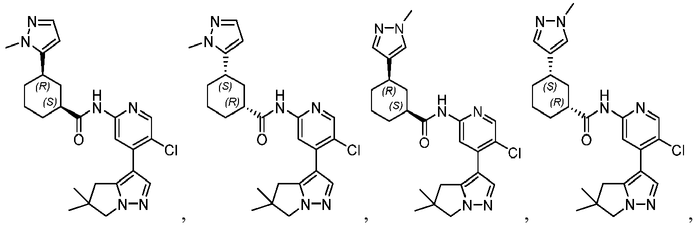

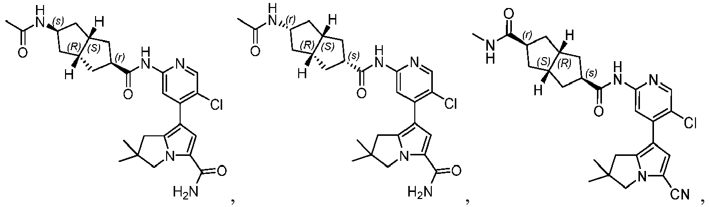

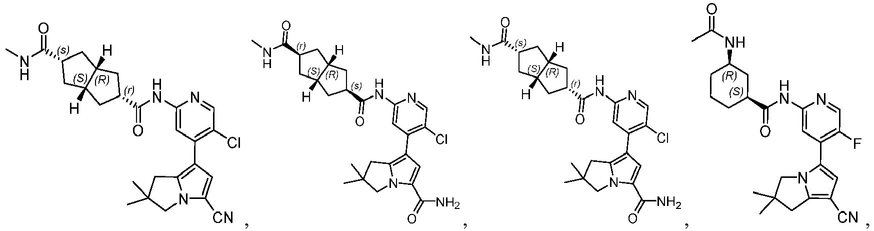

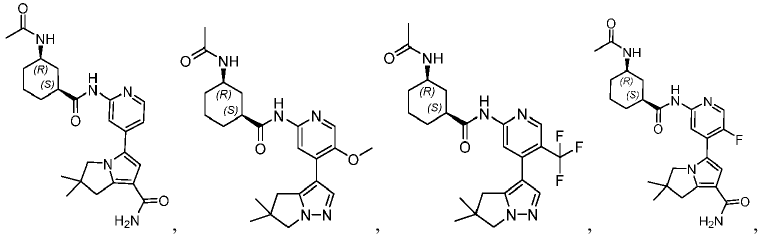

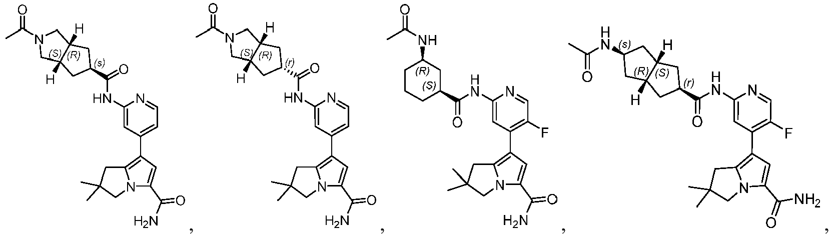

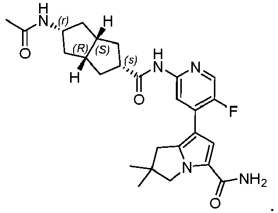

8. A preparation method of the polycyclic amide derivative, the pharmaceutically acceptable salt thereof, the tautomer thereof or the stereoisomer thereof according to any one of claims 1-7, selected from one of the following three methods:
Method One: 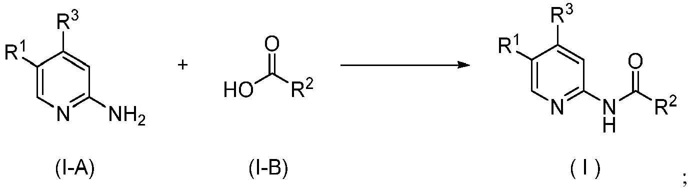
Method Two: 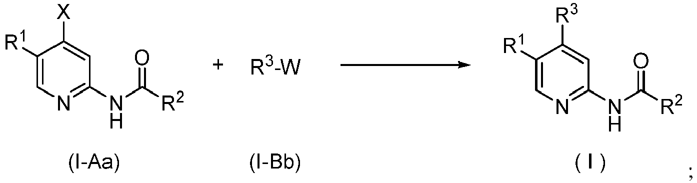
Method Three: 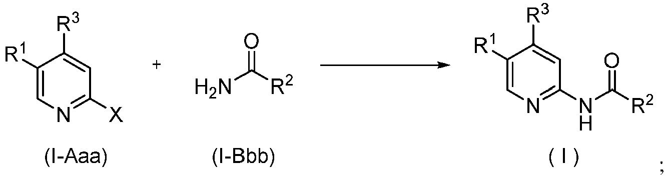 where W is 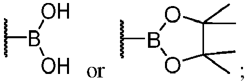 X is halogen; R¹, R², and R³ have the same defined ranges as in claim 1.

9. A pharmaceutical composition, wherein the pharmaceutical composition comprises the polycyclic amide derivative, the pharmaceutically acceptable salt thereof, the tautomer thereof or the stereoisomer thereof according to any one of claims 1-7; preferably, the pharmaceutical composition further comprises a pharmaceutically acceptable carrier and/or an excipient.

10. An application of the polycyclic amide derivative, the pharmaceutically acceptable salt thereof, the tautomer thereof or the stereoisomer thereof according to any one of claims 1-7, or the pharmaceutical composition according to claim 9 in the preparation of medicaments for treating cancers;
preferably, the cancer is blood cancer, further preferably acute myeloid leukemia, multiple myeloma, chronic lymphocytic leukemia, follicular lymphoma, or solid tumor; still further preferably, the solid tumor is breast cancer, prostate cancer, ovarian cancer, hepatocellular carcinoma, pancreatic cancer, kidney cancer, gastric cancer, colorectal cancer or lung cancer.
